(19)

Europäisches
Patentamt
European
Patent Office
Office européen
des brevets

(11) **EP 2 524 695 A1**

(12) **EUROPEAN PATENT APPLICATION**

(43) Date of publication:
**21.11.2012 Bulletin 2012/47**

(51) Int Cl.:
*A61K 36/185* (2006.01)   *A61K 36/58* (2006.01)
*A61K 36/19* (2006.01)   *A61K 36/67* (2006.01)
*A61K 36/8962* (2006.01)   *A61K 36/9068* (2006.01)
*A61K 36/59* (2006.01)   *A61K 36/53* (2006.01)

(21) Application number: **11166389.4**

(22) Date of filing: **17.05.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Himalaya Global Holdings Limited
Dubai (AE)**

(72) Inventors:
• **Mitra, Shankar Kumar
562 123, Bangalore (IN)**

• **Babu, Uddagiri Venkanna
562 123, Bangalore (IN)**
• **Saxena, Ekta
562 123, Bangalore (IN)**
• **Sharma, Anu Vrat
562 123, Bangalore (IN)**

(74) Representative: **Kuhnen & Wacker
Patent- und Rechtsanwaltsbüro
Prinz-Ludwig-Straße 40A
85354 Freising (DE)**

(54) **Herbal solid formulation and process for preparing the same**

(57)     Disclosed is a herbal solid formulation comprising *Andrographis paniculata, Terminalia arjuna, Azadirachta indica, Trikatu (Zingiber officinalis, Piper longum, Piper nigrum), Tinospora cordifolia, Ocimum sanctum, Withania somnifera, Zingiber officinale, Commiphora mukul* or *Allium sativum* extracts essentially free of excipients and preservatives and process for preparing the same.

Figure 1: LC chromatogram and total ion chromatogram of Arjuna CO$_2$ Extract

EP 2 524 695 A1

**Description**

Field of the Invention

This invention, in general relates to a herbal solid formulation. In particular, the present invention provides a herbal solid formulation comprising *Andrographis paniculata, Terminalia arjuna, Azadirachta indica, Trikatu (Zingiber officinalis, Piper longum, Piper nigrum), Tinospora cordifolia, Ocimum sanctum, Withania somnifera, Zingiber officinale, Commiphora mukul or Allium sativum* extracts without using any excipients and preservatives and process for preparing the same.

Background of the Invention

Herbal supplements have been witnessed tremendous growth and acceptance among the consumers during the last decade due to their safety and efficacy. Unlike allopathic medications, herbal extracts are safe and devoid of any side effects. There is a growing concern among the consumers worldwide using naturally derived products and avoiding synthetic chemicals in their food, personal care products and daily health supplements. Many herbal products those are available in the market as tablets and capsule using synthetic excipients such as binders, lubricants and diluents and preservatives such as Parabens and salts of benzoic acids etc. These excipients and preservatives are reported to have toxic and side effects.

Pharmaceutical dosage forms such as tablets and capsules should have certain properties such as hardness, friability, disintegration time (DT), stability and delivery of the drug to give required therapeutic benefits to the patient. These properties are achieved using the excipients such as binders, lubricants and diluents.

It is therefore very important and challenging task to develop a process of manufacturing herbal tablets using herbal extract and plant powder without using any synthetic excipient and preservative.

Related Art

US Patent 6,207,189 by Mercati et al disclose a process for the production of tablets and capsules of natural substances of vegetable origin wherein dry extracts and micronized powders of one or more medicinal herbs in appropriate proportions are blended and subjected to steam pressure followed by drying, preparation of granules and compression to tablets.

US Patent 6,468,563 by Schmidt et al. discloses a process for producing rapidly disintegrating pharmaceutical formulation containing an extract and lubricant and compressing the blend to form the pharmaceutical formulation.

Summary of the Invention

It is a principal object of the invention to provide a herbal solid formulation comprising *Andrographis paniculata, Terminalia arjuna, Azadirachta indica, Trikatu (Zingiber officinalis, Piper longum, Piper nigrum) Tinospora cordifolia, Ocimum sanctum, Withania somnifera, Zingiber officinale, Commiphora mukul or Allium sativum* extracts essentially free of additives/excipients and preservatives and providing required quantity of active constituents per dose.

It is another object of the invention to provide a herbal solid formulation of herb *Andrographis paniculata, Terminalia arjuna Azadirachta indica, Trikatu (Zingiber officinalis, Piper longum, Piper nigrum), Tinospora cordifolia, Ocimum sanctum, Withania somnifera, Zingiber officinale, Commiphora mukul or Allium sativum* extracts having reduced side effects and toxicity.

It is yet another object of the invention to provide a herbal solid formulation of herb *Andrographis paniculata, Terminalia arjuna, Azadirachta indica, Trikatu (Zingiber officinalis, Piper longum, Piper nigrum) Tinospora cordifolia Ocimum sanctum, Withania somnifera, Zingiber officinale, Commiphora mukul or Allium sativum* extracts essentially free of excipients/additives and preservatives and having desired friability, disintegration time and hardness.

It is still another object of the invention to provide a method for preparing extract of herb *Andrographis paniculata, Terminalia arjuna, Azadirachta indica, Trikatu (Zingiber officinalis, Piper longum, Piper nigrum) Tinospora cordifolia Ocimum sanctum, Withania somnifera, Zingiber officinale, Commiphora mukul or Allium sativum* used to prepare the solid formulation.

The above and other objects of the present invention are attained according to following preferred embodiments of the present invention. However the scope of the invention is not restricted to the particular embodiments discussed herein after.

In accordance with one preferred embodiment of the present invention, there is provided a herbal solid formulation comprising a blend of Super Critical Fluid ($CO_2$) extract, water extract and powder of herb *Terminalia arjuna, Azadirachta indica, Trikatu (Zingiber officinalis, Piper longum, Piper nigrum) Tinospora cordifolia or Ocimum sanctum,* wherein said blend of extract and said powder of herb is mixed in a ratio of about 1:0.5 to about 1:90.

In accordance with one preferred embodiment of the present invention, there is provided a herbal solid formulation comprises a blend of Super Critical Fluid ($CO_2$) extract, water extract and powder of *Withania somnifera, Zingiber officinale or Commiphora mukul,* wherein said blend of extract and said powder of herb is mixed in a ratio of about 1: 0.5 to about 1:10.

In accordance with one preferred embodiment of the present invention, there is provided a herbal solid formulation comprises a blend of freeze dried powder of herb *Allium sativum* and water extract of herb *Allium sativum* along with powder of *Allium sativum,* wherein said blend of extract and said powder of herb is mixed in a ratio of about 1:0.5 to about 1:10.

In accordance with one preferred embodiment of the present invention, there is provided a herbal solid formulation comprises a blend of Super Critical Fluid ($CO_2$) extract, water extract and powder of herb *Terminalia arjuna, Azadirachta indica, Trikatu (Zingiber officinalis, Piper longum, Piper nigrum) Tinospora cordifolia Ocimum sanctum, Withania somnifera, Zingiber officinale, Commiphora mukul or Allium sativum,* wherein said herbal solid formulation is essentially free of additives/excipients.

In accordance with another preferred embodiment of the invention there is provided a process for preparing a herbal solid formulation according to above essentially free of additives/excipients comprises of autoclaving powder of the herb, granulating the autoclaved powder with a water extract of the herb, lubricating the granulated mixture by adding the autoclaved powder of the herb and preparing the solid formulation.

In accordance with another preferred embodiment of the invention there is provided a process for preparing a herbal solid formulation essentially free of additives/excipients comprising granulating a blend of Super Critical Fluid ($CO_2$) extract and water extract of *Withania somnifera, Zingiber officinale or Commiphora mukul* autoclaving the resultant granular blend and further lubricating the granulated blend by adding the powder extract of *Withania somnifera, Zingiber officinale or Commiphora mukul* and preparing the solid formulation.

In accordance with one preferred embodiment of the present invention, there is provided a herbal solid formulation comprises a blend of freeze dried powder of herb *Allium sativum* and water extract of herb *Allium sativum* along with powder of *Allium sativum,* wherein said herbal solid formulation is essentially free of additives/excipients.

In accordance with another preferred embodiment of the invention there is provided a process for preparing a herbal solid formulation essentially free of additives/excipients comprising granulating a blend of freeze dried powder and water extract of *Allium sativum,* and lubricating the granulated blend by adding the powder of *Allium sativum* and preparing the solid formulation.

In accordance with yet another preferred embodiment of the invention, the powder of herb is obtained by pulverizing the herb to a powder having mesh size preferably between about 20 to about 100.

In accordance with yet another embodiment of the present invention, the extract of the herb is passed through a mesh having size between about 20 to 80.

In accordance with still another embodiment of the present invention, wherein the granulation of the blend of extracts and powder of the herb is carried out in presence of a solvent, preferably water and grain alcohol or combination thereof.

In accordance with yet another embodiment of the present invention there is provided a process for preparing the extract of the herb by Super Critical Fluid ($CO_2$) extraction, percolation, hot soxhalation or refluxing followed by filtration and concentration to dryness at optimum temperature.

In accordance with one other embodiment of the present invention, there is provided a process for sterilization of herbal powders by autoclaving the granular mixture, wherein autoclaving prevents microbial growth.

Brief Description of Drawings

Further objects of the present invention together with additional features contributing thereto and advantages accruing there from will be apparent from the following description of preferred embodiments of the invention which are shown in the accompanying drawing figures, wherein:

Figure 1 illustrates the LCMS chromatogram of *Terminalia Arjuna* supercritical fluid $CO_2$ extract.
Figure 2 illustrates the LCMS chromatogram of *Terminalia Arjuna* water extract.
Figure 3 illustrates LCMS chromatogram of Supercritical fluid $CO_2$ extract of *Azadirachta indica*
Figure 4 illustrates LCMS chromatogram of water extract of *Azadirachta indica*
Figure 5 illustrates LCMS chromatogram of water extract of Trikatu
Figure 6 illustrates LCMS chromatogram of Supercritical fluid $CO_2$ extract of Trikatu
Figure 7 illustrates LC and total ion chromatogram of Guduchi *(Tinospora)* $CO_2$ extract.
Figure 8 illustrates LC and total ion chromatogram of Guduchi *(Tinospora)* water extract.
Figure 9 illustrates LC and total ion chromatogram of Tulsi *(Ocimum)* $CO_2$ extract.

Figure 10 illustrates LC and total ion chromatogram of Tulsi *(Ocimum)* water extract.

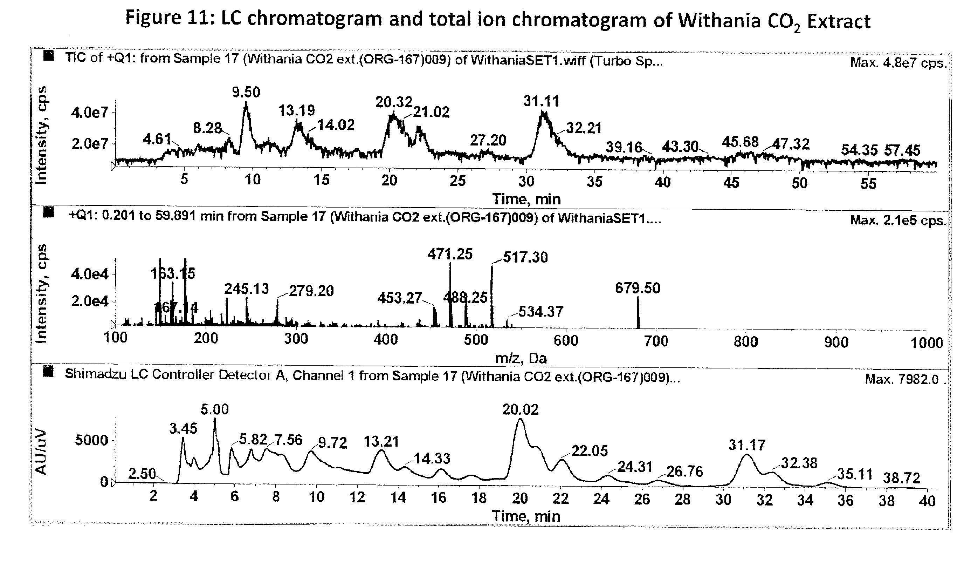

Figure 11 illustrates the LCMS chromatogram of *Withania somnifera* CO2 extract.

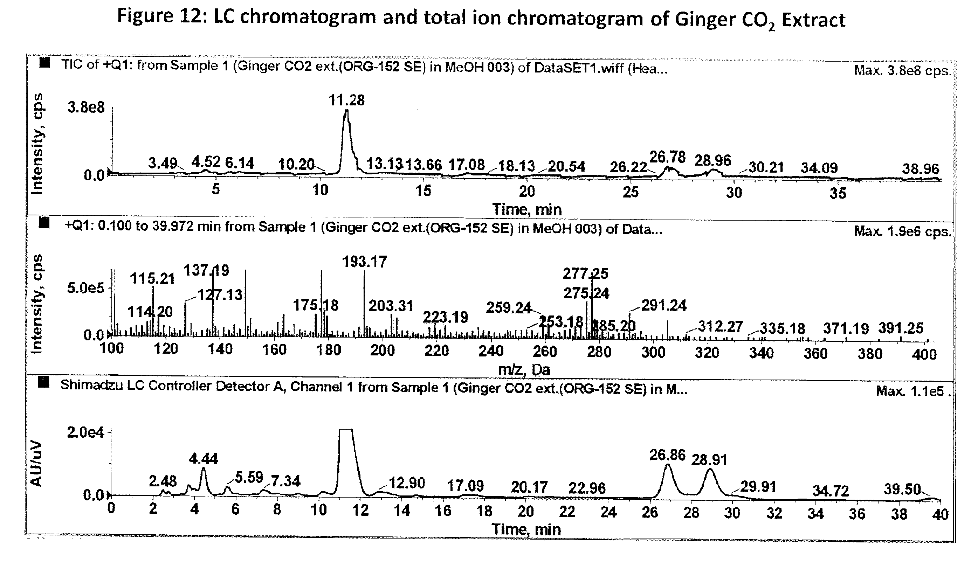

Figure 12 illustrates the LCMS chromatogram of Ginger *(Zingiber officinale)* CO2 extract.

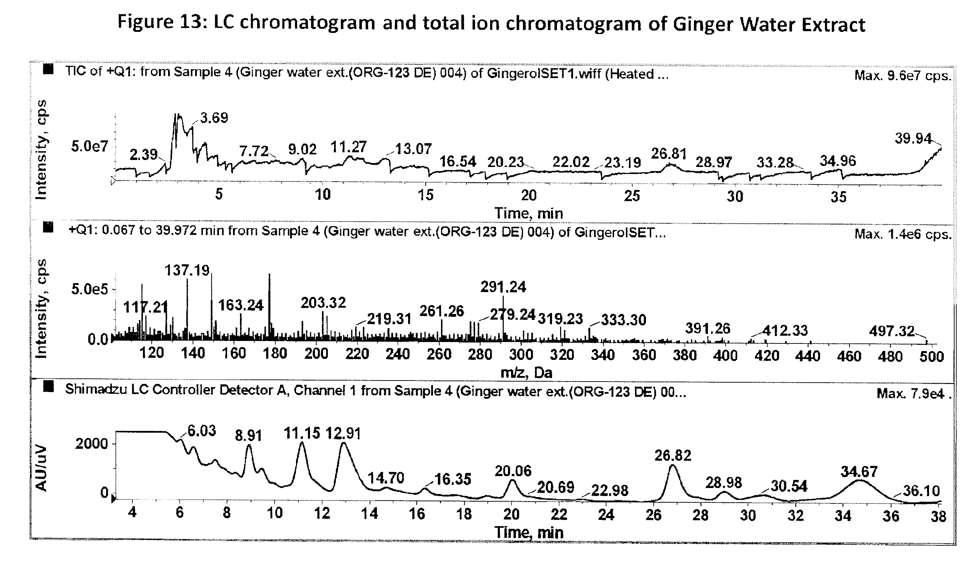

Figure 13 illustrates the LCMS chromatogram of Ginger *(Zingiber officinale)* $CO_2$ extract.

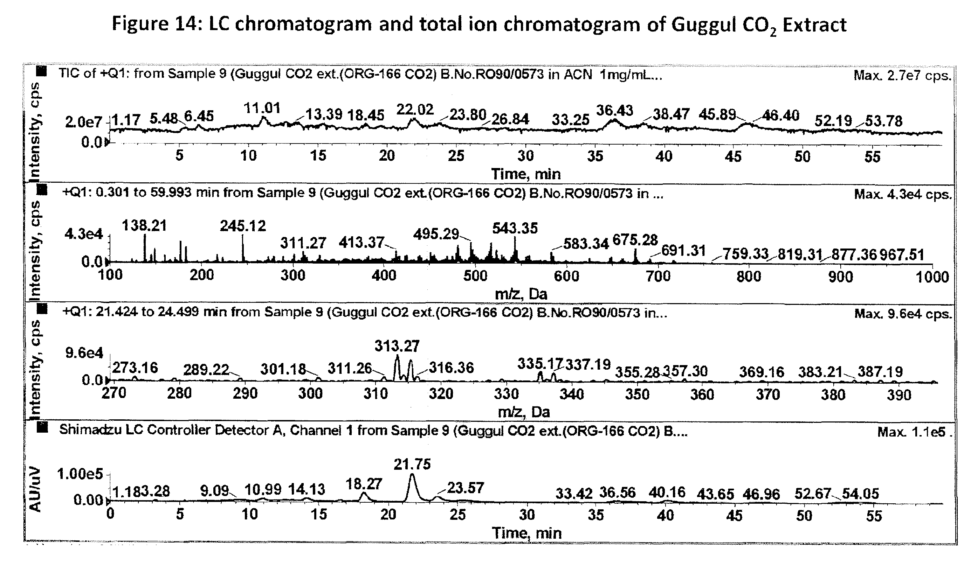

Figure 14 illustrates the LCMS chromatogram of Guggul CO2 extract.

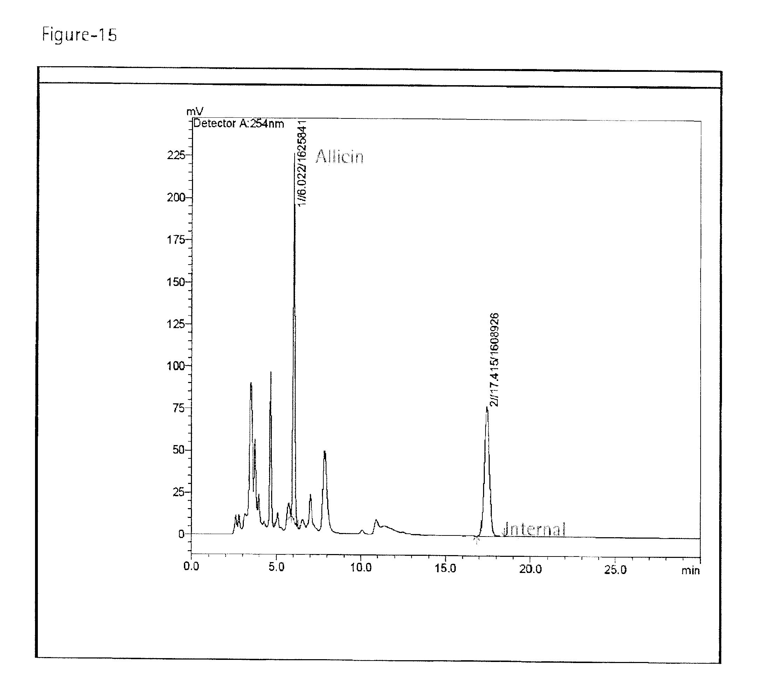

Figure 15 illustrates HPLC chromatogram of garlic.

Detailed Description of the Invention

While this specification concludes with claims particularly pointing out and distinctly claiming that, which is regarded as the invention, it is anticipated that the invention can be more readily understood through reading the following detailed description of the invention and study of the included examples.

The present invention provides a herbal solid formulation essentially free of excipients/additives or preservatives, wherein said formulation comprises a blend of Super Critical Fluid ($CO_2$) extract and water extract of *Andrographis paniculata, Terminalia arjuna, Azadirachta indica, Trikatu (Zingiber officinalis, Piper longum, Piper nigrum) Tinospora cordifolia, Ocimum sanctum Withania somnifera, Zingiber officinale, or Commiphora mukul* along with powder of *Andrographis paniculata, Terminalia arjuna, Azadirachta indica, Trikatu (Zingiber officinalis, Piper longum, Piper nigrum) Tinospora cordifolia, Ocimum sanctum Withania somnifera, Zingiber officinale, or Commiphora mukul* and a process for preparing the same.

The present invention provides an herbal solid formulation essentially free of excipients/additives or preservatives, wherein said formulation comprises a blend of freeze dried garlic powder, water extract and aerial parts powder of *Allium Sativum*

The present invention provides an herbal solid formulation essentially free of excipients/additives or preservatives, wherein said formulation comprises a blend of freeze dried powder and water extract of *Allium sativum* along with powder *Allium sativum* and a process for preparing the same.

The bulbs of *Allium sativum* were depoded, cleaned with water and chopped to reduce the size. The chopped material is freeze dried, hand crushed and passed though the mesh having size preferably between #10 to 100.

The extracts of the herb is prepared by Super Critical Fluid ($CO_2$) method. Alternately the same is prepared by employing percolation, hot soxhalation or refluxing method using a solvent, followed by filtration and concentration on a rotatory evaporator on steam bath at optimum temperature and under reduced pressure. The solvent employed includes organic grain alcohol, ethanol or water or combinations thereof, preferably grain alcohol. The extract is dried and passes through a mesh having size preferably between #20 to 80.

The powder of the herb is prepared by pulverizing the root of herb to a powder of different mesh sizes based on the requirement, preferably between about 20 to about 100, more preferably between 20 to 80. The extract and the powder of the herb is mixed in a predetermined ratio preferably between about 1: 0.5 to 1: 90 for optimum granulation.

The powder of the herb is prepared by pulverizing the aerial parts of herb to a powder of different mesh sizes based on the requirement, preferably between about 20 to about 100.

The freeze dried powder, extract and the powder of the herb is mixed in a predetermined ratio preferably between about 1:0.5 to about 1:10 for optimum granulation to prepare the herbal solid formulation. The granulation is performed using a solvent system followed by passing the granules through a mesh having size preferably between 12 to 24#. The solvent system employed for granulating the mixture includes grain alcohol, water or combination thereof.

The powder of the herb is prepared by pulverizing the root of herb to a powder of different mesh sizes based on the requirement, preferably between about 10 to about 100, more preferably between 20 to 80. The extract and the powder of the herb is mixed in a predetermined ratio preferably between about 1:0.5 to about 1:10 for optimum granulation.

The process of preparing the herbal solid formulation involves granulation of the blend of extracts and powder of the herb using a solvent system, followed by passing the granules through a mesh having size preferably between 12 to 24 # and autoclaving the granules. The solvent system employed for granulating the mixture includes grain alcohol, water or combination thereof. Autoclaving helps in microbial control of the solid formulation as it does not contain any preservatives.

The autoclaved granules are further lubricated using the powder of the *Andrographis paniculata, Terminalia arjuna, Azadirachta indica, Trikatu (Zingiber officinalis, Piper longum, Piper nigrum) Tinospora cordifolia, Ocimum sanctum, Withania somnifera, Zingiber officinale or Commiphora mukul* and compressed or encapsulated into tablets or capsules.

All extracts, granules and tablets are subjected to standardization by High Performance Thin Layer Chromatography (HPTLC) and High Performance Liquid Chromatography (HPLC) for identification and quantitative estimation of active marker compounds. The extracts were evaluated for toxicity studies in rats and tablets for safety studies in healthy human volunteers.

The solid formulation according to the present invention has desired hardness preferably between 2 to 8 kg/cm$^2$,

more preferably about 3 to about 4 kg/cm$^2$, friability less than about 1% w/w and disintegration time less than about 60 min, preferably between 5 to 60 min, more preferably less than about 30 min. The solid formulation complies with USFDA guidelines.

The solid formulation according to the present invention has desired hardness preferably between about 2 to 8 kg/cm$^2$, preferably 3 to about 4 kg/cm$^2$, friability less than about 1% w/w and disintegration time less than about 60 min, preferably between 5-30 min. The solid formulation complies with USFDA guidelines.

The solid formulation according to the present invention has desired hardness preferably between about 3 to about 4 kg/cm$^2$, friability less than about 1% and disintegration time less than about 30 min. The solid formulation complies with USFDA guidelines.

According to the present invention, the disclosed solid formulation is preferably granules, tablet or capsule.

The following non-limiting examples illustrate specific embodiments of the present invention. They are, not intended to be limiting the scope of present invention in any way.

Example-1

Preparation of *Andrographis paniculata* (Kalmegh) water extract

Approximately 100 Kg of shade dried plant material was subjected to extraction with 400 Liters of purified water by percolation method at Room Temperature. The water extractions after 24 hours were filtered through muslin cloth and concentrated to thick paste. After achieving the desired total solid content, the soft extract was spray dried to a free flowing dry extract powder.

Example-2

Preparation of *Andrographis paniculata* (Kalmegh) SCFE (CO2) Extract

Approximately 25 Kg of The whole plant of *Andrographis paniculata* was pulverized to fine powder and loaded in to extractor. Super Critical Carbon dioxide liquid was pmped in to the extractor at a pressure of 300 bar and 39°C temperature for 2-3 hours. Extract was separated into the container at pressure of 40 bar and 20°C temperature. The CO2 super critical liquid was recycled from the extraction vessel. The resultant extract was analyzed for active markers of Andrographolides.

Example-3

Formula of Granulation

Table 1

| S. No. | Name of the material | Weight per Tablet (mg) | Weight per Batch (Kg) |
|---|---|---|---|
| 1 | *Andrographis paniculata* (Water extract) (# 40 mesh) | 200.00 | 20.00 |
| 2 | *Andrographis paniculata* (Super critical fluid (CO2) extract) | 50.00 | 5.00 |
| 3 | *Andrographis paniculata* (#40) powder | 375.00 | 37.50 |

Example-4

Formula of Lubrication of granules

Table 2

| S. No. | Name of the material | Weight per Tablet (mg) | Weight per Batch (Kg) |
|---|---|---|---|
| 1 | *Andrographis paniculata* Extract Granules (# 16 mesh) | 625.00 | 62.50 |

(continued)

| S. No. | Name of the material | Weight per Tablet (mg) | Weight per Batch (Kg) |
|---|---|---|---|
| 2 | *Andrographis paniculata (#40) powder* | **25.00** | **2.50** |
| | *Total* | **650.00** | **65.00** |

Example-5

Manufacturing procedure of granulation and compression of Tablet

1. Dispense Extract, Plant powder as per Batch record.
2. Transfer *Andrographis paniculata* Leaf powder (#40 mesh) to stainless steel trays and sterilize at 160°C for 120 minutes.
3. Check the sterilized material for LOD (Loss on Drying), Bulk Density and Microbial growth.
4. Sift 25 Kg of *Andrographis paniculata* Water extract through #40 mesh and 37.50 Kg and 2.50 Kg of Leaf powder through # 40 mesh. Keep the materials packed in double poly bags.
5. Charge 25 Kg of Water extract and 37.50 Kg of Leaf powder in to RMG and mix slowly for 5 minutes. Add 5 Kg of *Andrographis paniculata* $CO_2$ extract and 2.5 Kg of Purified water to the mixture over a period of 3 minutes with medium speed.
6. Stop the mixer and scrap off the material from the sides and bottom and continue mixing by operating impeller mixing at high speed with chopper ON for 3 minute.
7. Discharge the material from RMG
8. Mill the wet mass obtained from the above procedure in Multi mill fitted with 8 mm screen.
9. Dry the wet mass obtained through Multi mill at 70°C to 80°C for 60 minutes and checks the moisture every 30 minutes to achieve LOD at 2.5 to 3.5%.
10. Sift the dried granules through #16 SS mesh and store in double polylines HDPE containers.
11. Analyze granules for LOD, BD, micro analysis, granules-fine ratio etc.
12. Maintain the temperature at 25°C and relative humidity NMT 40% of blending and compression area.
13. Transfer the sifted Leaf powder to blending area and blend the leaf powder and granules in to the double cone blender for 3 minutes at 20-25 RPM.
14. Compress the approved granules into tablets with punch size 17X8 8 mm size with upper and bottom as plain surface.
15. Check appearance, thickness and hardness of tablets every 30 minutes.
16. Check friability and DT for every 1 hour and average weight every 30 minutes.
17. Collect the tablets in double poly lined air tight container.
18. Final tablets have the following specifications:

**STANDARD PARAMETERS***

| | | |
|---|---|---|
| 1. Theoretical average weight | : | 650 mg |
| 2. Weight uniformity | : | 650 mg $\pm$ 5% (617.5mg to 682.5mg) |
| 3. Weight of 20 tablets | : | 13.00 g $\pm$ 3% (12.61gm to 13.39gm) |
| 4. Tablet thickness | : | 4.8 to 5.8 mm |
| 5. Tablet hardness | : | 4 to 6 Kg/cm$^2$ |
| 6. Friability | : | NMT 1.0% W/W |
| 7. Disintegration time | : | NMT 30 min. |
| 8. Andrographolide | : | 9 mg per caplet |

Example-6

Preparation of *Terminalia arjuna* (Arjuna) water extract

Approximately 100 Kg of shade dried plant material bark of *Terminalia arjuna* was subjected to extraction with 400 Liters of purified water by percolation method at Room Temperature. The water extractions after 24 hours were filtered through muslin cloth and concentrated to thick paste. After achieving the desired total solid content, the soft extract was spray dried to a free flowing dry extract powder. The residual plant materials were named as spent powder.

Example-7

Preparation of *Terminalia arjuna* (Arjuna) SCFE ($CO_2$) Extract

Approximately 25 Kg of bark of *Terminalia arjuna* was pulverized to fine powder and loaded in to extractor. Super Critical Carbon dioxide liquid was pumped in to the extractor at a pressure of 300 bar and 39 °C temperature for 2-3 hours. Extract was separated into the container at pressure of 40 bar and 20 °C temperature. The $CO_2$ super critical liquid was recycled from the extraction vessel.

Example-8

Formula of Granulation ( Table-3)

| S. No. | Name of the material | Weight per Tablet in mg | Weight per Batch in Kg |
|---|---|---|---|
| 1 | *Terminalia arjuna* (Water extract) (# 40 mesh) | 240.00 | 24.00 |
| 2 | *Terminalia arjuna* (Super critical fluid ($CO_2$) extract) | 10.00 | 1.00 |
| 3 | *Terminalia arjuna* ( spent powder ) | 200.00 | 20.00 |
| 4 | *Terminalia arjuna* leaves (#40) powder | 150.00 | 15.00 |
| 5 | Purified water | Granulation fluid | Quantity sufficient |

Example-9

Formula of Lubrication of granules (Table-4)

| S. No. | Name of the material | Weight per Tablet in mg | Weight per Batch in Kg |
|---|---|---|---|
| 1 | *Terminalia arjuna* Extract Granules (# 16 mesh) | 600.00 | 60.00 |
| 2 | *Terminalia arjuna* spent powder (#40) powder | 100.00 | 10.00 |
| | Total | 700.00 | 70.00 |

Example-10

DISPENSING OF RAW MATERIAL

1. Dispense the raw materials as per Batch Formula

DRY HEAT STERILIZATION

1. Transfer 30.00 kg of spent powder of *Terminalia arjuna* bark (left material after water extraction ) and 15 kg of leaves of *Terminalia arjuna* into trays and sterilize the material @ 160 °C for 60 mins.
2. Unload the sterilized materials in to double lined polybags separately and keep in airtight containers.
3. Send the sample for LOD, BD and Microbial Analysis. Table-5.

Table-5

|   | Parameter | Standard Value |
|---|-----------|----------------|
| 1 | LOD | |
|   | spent powder of *Terminalia arjuna* bark | 2.0-3.0%w/w |
|   | leaves powder of *Terminalia arjuna* | 2.0-2.30% w/w |
| 2 | BD | |
|   | spent powder of *Terminalia arjuna* bark | 0.3-0.5 g/ml |
|   | Leaves powder of *Terminalia arjuna* | 0.2-0.5 g/ml |
| 3 | TVAC | NMT 5000 cfu/gm |
| 4 | Fungal count | NMT 10 cfu/gm |

## SIFTING

1. Sift 24.00 kg of water extract of bark of *Terminalia arjuna* through # 40.
2. Sift 20.00 kg of spent powder of *Terminalia arjuna* bark and 15.00 kg of Leaves powder of *Terminalia arjuna* through #60.
3. Sift 6.30 kg of spent powder of *Terminalia arjuna* bark lubricant through # 40 separately.
4. Collect the above-sifted materials in separate duly labeled double lined polybags.
5. Record Quantity Sifted and the sieve integrity before and after sifting.

## PREPARATION OF GRANULATION FLUID

1. Transfer about 5.0 kg of purified water into a clean Stainless Steel Vessel.
2. Record the observations. Record deviations if any.

## GRANULATION

1. Charge 15.00 Kg of leaves powder of *Terminala arjuna* and then add 1.00 kg of super critical ($CO_2$) extract of *Terminalia arjuna* for about 5 min.
2. Charge 20.00 kg of spent powder of *Terminalia arjuna* bark into RMG and blend for about 5 min.
3. Charge 24.00 kg of water extract of bark of *Terminalia arjuna* into RMG and blend for about 5 min.
4. Slowly add the granulation fluid to the RMG over a period of about 3 min, with medium speed.
5. Stop the mixer and scrape off the mass from the sides and bottom.
6. Continue mixing by operating the impeller at high speed with Chopper ON for about 3 minute.
7. Add additional quantity of purified water, if required.
8. Discharge the mass from the RMG.
9. Record the observations. Record deviations if any.

## WET MILLING

1. Mill the Wet Mass in Multi mill fitted with 8 mm screen.

TRAY DRYING

1. Dry the Wet mass in tray Drier at about 70°C to 80°C for about 60 minutes.
2. Check the Moisture once every 30 minutes. (LOD 4.0 to 6.0% w/w) and record the details.

SIZING

1. Sift the dried granules using a Sifter fitted with sieve #16.
2. Collect the sifted granules in a clean double poly lined HDPE container.
3. Mill the retains (oversize granules) through a Multi Mill fitted with 1.5mm screen with 'Knives Forward' direction.
4. Pass the milled granules through a Sifter fitted with #16 sieve.
5. Collect the sized granules and add them to the sifted granules.
6. Weigh the granules. Record deviations if any.
7. Analyse samples as per below table-6.
8. If required autoclave the granules at 120°C for 20 min.

Table-6

|   | Parameter | Standard values |
|---|-----------|-----------------|
| 1 | LOD | 4.0 - 6.0 % w/w |
| 2 | BD | 0.40 - 0.60 g/ml |
| 3 | Actives | As per finished product spec |
| 4 | TVAC | NMT 5000 cfu/gm |
| 5 | Fungal count | NMT 10 cfu/gm |

BLENDING

Maintain the Temperature and Relative Humidity of the area with in the specified limit (Limit Temperature NMT 25 °C and relative humidity NMT 40%)

1. Transfer the sized granules to the blending area.
2. Transfer the sifted spent powder of *Terminalia arjuna* lubricant to the blending area.
3. Load 10.00 kg of spent powder of *Terminalia arjuna* lubricant and the sized granules into the Double Cone blender.
4. Blend the ingredients for 3 minutes at 20-25 RPM.
5. Record the details.
6. Unload the blend in a clean Double poly-lined HDPE drums and affix duly filled status labels.
7. Weigh the blend and enter the details.

COMPRESSION

Maintain the Temperature and Relative Humidity of the area with in the specified limit (Limit Temperature NMT 25 °C and relative humidity NMT 40%)

1. Check the Temperature and Relative Humidity of the area and record.
2. Bring the drums containing the blend into the compression area.
3. Adjust the machine.
4. Carry out the initial checks before starting the operation as specified in Table -7.

Table-7

| | Description |
|---|---|
| Punch Size | 17x 8 mm caplet |
| Upper Punch | Plain |
| Lower Punch | Plain |

5. Check for Appearance, Average Weight, Individual weight, Thickness, Hardness, Friability & Disintegration of 6 tablets.
6. Check appearance, thickness & hardness of tablets every 30 min.
7. Check Friability and DT every 1-hour.
8. Check Average weight of 20 tablets every 30 mins graphically.
9. Collect the tablets in a double poly-lined, tightly closed, container. Weigh each container and enter the details.

STANDARD PARAMETERS Of TABLETS

Standard parameters are mentioned in Table-8

Table-8

| S1.No. | Parameters | Standard value |
|---|---|---|
| 1 | Theoretical average weight | 700 mg |
| 2 | Weight uniformity | 700 mg $\pm$ 5% (665 mg to 735 mg) |
| 3 | Weight of 20 tablets | 14.00 g $\pm$ 3% |
| 4 | Tablet thickness | 4.5 to 5.5 mm |
| 5 | Tablet hardness | 3 to 5 Kg/cm$^2$ |
| 6 | Friability | NMT 1.0% W/W |
| 7 | Disintegration time | NMT 30 min. |
| 8 | Arjunolic acid<br>Tannins | 0.25 mg<br>120 mg |

1. Collect the Compressed tablets in Double poly lined HDPE drums and record their weights.
2. Affix duly filled status labels to the containers.

PACKING

1. Pack 60 tablets in a HDPE 120 CC container and weigh them for appropriateness of number of tablets.

Example-11

Estimation of arjunolic acid in Arjuna dry extract, granules

Standard arjunolic acid solution (0.1 mg/ml): Weigh accurately 10 mg of standard arjunolic acid in a 10 ml volumetric flask. Add 7 - 8 ml of methanol and dissolve. Make the volume up to the mark with methanol. Take 1 ml of this solution in another 10 ml volumetric flask, dilute and make the volume up to the mark with methanol.

Sample preparation (10 mg/ml): Weigh accurately 0.5 g of sample in a 250 ml flat bottomed flask. Add 50 ml of methanol and reflux it on a water bath at 80 °C for 30 minutes. Filter and transfer it into a 50 ml volumetric flask. Make the volume up to the mark with methanol.

HPLC Conditions were as follows

Column: $C_{18}$ ODS Hypersil (250 x 4.6) particle size: 5 $\mu$ (Make: Thermo)
Mobile phase: Methanol: 0.1% Phosphoric acid (70: 30)
Flow rate: 1 ml/minute
Wavelength: 210 nm

Chromatographic procedure: Stabilize the instrument with the above mentioned mobile phase. Inject 20 $\mu$l of standard solution and record the chromatogram. Similarly, inject 20 $\mu$l of sample solution and record the chromatogram. Calculate the area of standard peak and the corresponding peak in the sample.

Calculation: % w/w Arjunolic acid content can be calculated using the formula.

$$\% \text{ w/w Arjunolic acid} = \frac{\text{Area of sample peak}}{\text{Area of standard peak}} \times \frac{\text{Concentration of standard (mg/ml)}}{\text{Concentration of sample (mg/ml)}} \times \% \text{ Purity of standard}$$

Example-12

LCMSMS STUDIES

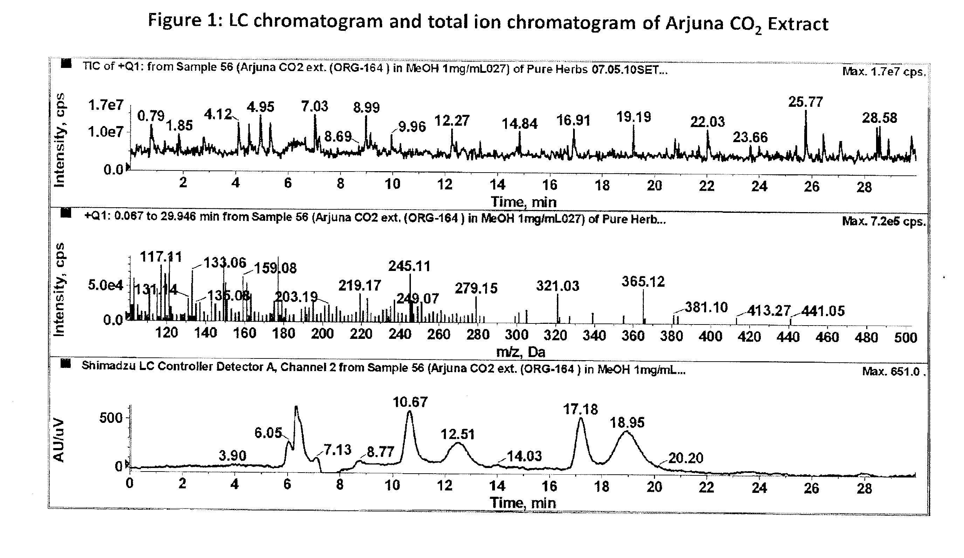

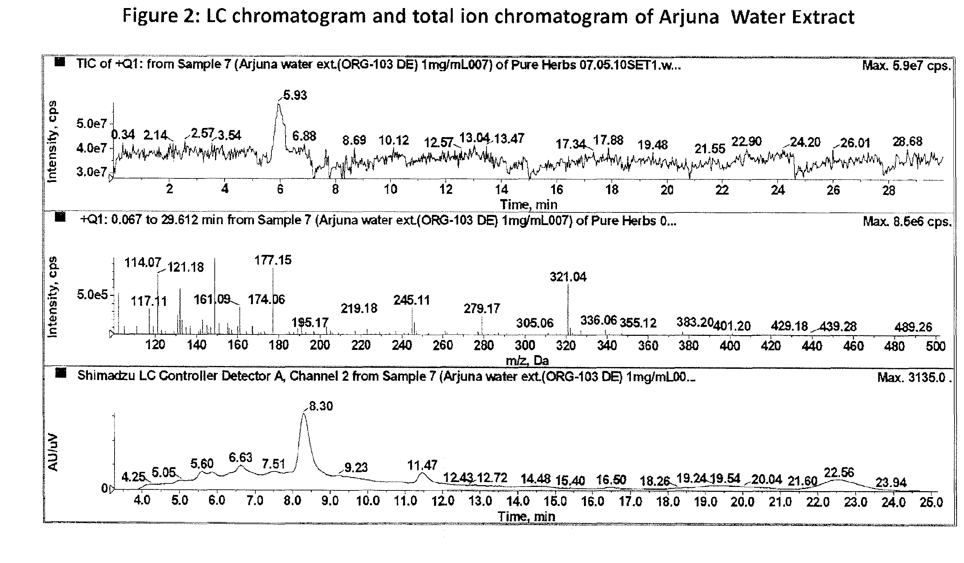

Liquid Chromatography-Mass Spectrometer Analysis of *Terminalia arjuna* bark supercritical ($CO_2$) extract and water extract (Figure 1 and 2):

LCMSMS analysis were carried out by using an applied biosystem-Sciex API 2000 triple quadrupole mass spectrometer equipped with an ion source turbo spray and ESI interface. The liquid chromatography was a LC-20 AD series binary system equipped with an autosampler. The column used was C18 phenomenex (250 X 4.6 mm, 5$\mu$m), flow rate 0.5 ml/min of mobile phase methanol: water (0.1 % acetic acid), (10:90), wave length 275 nm and run time 25 min. The analytes were ionized by ESI in positive-ion mode (PI mode). Final ionization conditions were heated ionization temperature 435°C. Curtain gas Nitrogen 30 psi, particulate -free and $CO_2$- free air was used as ion source gas 1 at a flow rate 50 psi and ion source gas 2 at a flow rate 60 psi.

Sample preparation for *Terminalia arjuna* bark $CO_2$ extract:

Weigh about 50 mg of sample in a 50 ml volumetric flask, and dissolve by sonication in methanol (HPLC grade). Make the volume up to the mark with methanol filter through 0.2 um syringe filter.

Sample preparation for *Terminalia arjuna* bark water extract:

Weigh about 500 mg of finely powdered sample in a 50 ml volumetric flask, and dissolve by sonication in water (HPLC grade). Make the volume up to the mark with water filter through 0.2 um syringe filter.

Example-13

Preparation of *Azadirachta indica* (Neem) water extract

Approximately 100 Kg of shade dried plant material leaves of *Azadirachta indica* was subjected to extraction with 400 Liters of purified water by percolation method at Room Temperature. The water extractions after 24 hours were filtered through muslin cloth and concentrated to thick paste. After achieving the desired total solid content, the soft extract was spray dried to a free flowing dry extract powder.

Example-14

Preparation of *Azadirachta indica* (Neem) SCFE (CO2) Extract

Approximately 25 Kg of leaves of *Azadirachta indica* was pulverized to fine powder and loaded in to extractor. Super Critical Carbon dioxide liquid was pumped in to the extractor at a pressure of 300 bar and 39°C temperature for 2-3 hours. Extract was separated into the container at pressure of 40 bar and 20°C temperature. The $CO_2$ super critical liquid was recycled from the extraction vessel.

Example-15

Formula of Granulation (Table-9)

| S. No | Name of the material | Weight per Tablet in mg | Weight per Batch in Kg |
|---|---|---|---|
| 1 | *Azadirachta indica* leaves powder (Water extract) (# 40) | 200.00 | 20.00 |
| 2 | *Azadirachta indica* leaves (Super critical fluid (CO2) extract) | 80.00 | 8.00 |
| 3 | *Azadirachta indica* stem (#100) powder | 300.00 | 30.00 |
| 4 | Purified water | Granulation fluid | Quantity sufficient |

Example-16

Formula of Lubrication of granules (Table-10)

| S. No. | Name of the material | Weight per Tablet in mg | Weight per Batch in Kg |
|---|---|---|---|
| 1 | *Azadirachta indica* Extract Granules (# 16 mesh) | 580.00 | 58.00 |
| 2 | *Azadirachta indica* stem powder (#100) powder | 20.00 | 02.00 |
| | Total | 600.00 | 60.00 |

Example-17

Manufacturing Details Of Tablets

Dispensing of Raw Material

1. Dispense the raw materials as per Batch record.

Dry Heat Sterilization

1. Transfer 30 kg and 2 kg of stem powder of *Azadirachta indica* into trays and sterilize the material at 160 °C for 60 mins.
2. Unload the materials in to double lined polybags separately and keep in airtight containers.
3. Analyse the sample for LOD, BD and Microbiological Analysis as per Table-11

Table-11

|   | Parameter | Standard values |
|---|---|---|
| 1 | LOD | 2.0 - 4.0 % w/w |
| 2 | BD | 0.20-0.30 g/ml |
| 3 | TVAC | NMT 5000 cfu/gm |
| 4 | Fungal count | NMT 10 cfu/gm |

Sifting

1. Sift 20.00 kg of water extract of leaves of *Azadirachta indica* through # 40
2. Sift 08.00 kg of $Co_2$ extract of leaves *Azadirachta indica* through # 40
3. Sift 30.00 kg of stem powder of *Azadirachta indica* through # 100
4. Sift 2.00 kg of stem powder of *Azadirachta indica* through # 100 separately
5. Collect the above-sifted materials in separate duly labeled double lined polybags.
6. Record the Quantity Sifted and the sieve integrity before and after sifting

Preparation of granulation fluid

1. Transfer about 20.0 kg of Purified water into a clean Stainless Steel Vessel

Granulation

1. Charge 20.00 Kg of water extract of leaves of *Azadirachta indica*, 8.0 kg of $CO_2$ extract of leaves *Azadirachta indica* and 30.00 kg of stem powder of *Azadirachta indica* into the RMG, mix for about 5 minute.
2. Slowly add the granulation fluid to the RMG containing the Sifted materials over a period of about 3 minute, with medium speed.
3. Stop the mixer and scrape off the mass from the sides and bottom.
4. Continue mixing by operating the impeller at high speed with Chopper ON for about 3 minute.
5. Add additional quantity of Purified water, if required.
6. Discharge the mass from the RMG.
7. Record the observations. Record deviations if any.

Wet Milling

1. Mill the Wet Mass in Multi mill fitted with 8mm screen.

Tray Drying

1. Dry the Wet mass in tray drier at about 70 °C to 80 °C for about 60 minutes.
2. Check the Moisture once every 30 minutes. (LOD Limit: 3 to 4% w/w) and record the details

Sizing

1. Sift the dried granules using a Sifter fitted with sieve #16.
2. Collect the sifted granules in a clean double polylined HDPE container.
3. Mill the retains (oversize granules) through a Multi Mill fitted with 1.5 mm screen with 'Knives Forward' direction
4. Pass the milled granules through a Sifter fitted with #16 sieve.
5. Collect the sized granules and add them to the sifted granules
6. Weigh the granules.
7. Analyse the sample as per Table-12

Table-12

|  | Parameter | Standard values |
|---|---|---|
| 1 | LOD | 2.5 - 3.5 % w/w |
| 2 | BD | 0.30 - 0.40 g/ml |
| 3 | Granules to fine ration | 70:30 |
| 4 | TVAC | NMT 5000 cfu/gm |
| 5 | Fungal count | NMT 10 cfu/gm |

Blending

1. Transfer the sized granules into the blending area.
2. Transfer the sifted stem powder of *Azadirachta indica* lubricant into the blending area.
3. Load 5 kg of stem powder of *Azadirachta indica* and the sized granules into the Double Cone blender.
4. Blend the ingredients for about 3 minutes at 20-25 RPM.
5. Record the details
6. Unload the blend in a clean Double poly-lined HDPE drums and affix duly filled status labels.
7. Weigh the blend and enter the details.

Compression

Maintain the Temperature and Relative Humidity of the area with in the specified limit (Limit Temperature NMT 25°C and relative humidity NMT 40%)
1. Check the Temperature and Relative Humidity of the area and record.
2. Bring the drums containing the blend into the compression area.
3. Adjust the machine as per the parameters.
4. Carry out the initial checks before starting the operation as specified in below Table-13

Table-13

|  | Description |
|---|---|
| Punch Size | 17x 8 mm caplet |
| Upper Punch | Plain |
| Lower Punch | Plain |

5. Check for Appearance, Average weight, Individual weight, Thickness, Hardness, Friability & DT of 6 tablets.
6. Check appearance, thickness & hardness of tablets every 30 min.
7. Check Friability and DT every 1-hour.
8. Check Average weight of 20 tablets every 30 mins graphically.
9. Collect the tablets in a double poly-lined, tightly closed, container. Weigh each container and enter the details.

STANDARD PARAMETERS OF TABLETS

Standard parameters are mentioned in Table-14

Table-14

| S.No. | Parameters | Standard value |
|---|---|---|
| 1 | Theoretical average weight | 600 mg |
| 2 | Weight uniformity | 600 mg $\pm$ 5% (570mg to 630mg) |
| 3 | Weight of 20 tablets | 12.0 g $\pm$ 3% (11.64gm to 12.36gm) |
| 4 | Tablet thickness | 4.0 to 5.0 mm |
| 5 | Tablet hardness | 4 to 6 Kg/cm$^2$ |
| 6 | Friability | NMT 1.0% W/W |
| 7 | Disintegration time | NMT 30 min. |
| 8. | Bitters containing Nimbidin | 18 mg per caplet |

10. Collect the Compressed tablets in Double polylined HDPE drums and record their weights.
11. Affix duly filled status labels to the containers.

Packing

1. Pack 60 tablets in a HDPE 120 CC container and weigh them for appropriateness of number of tablets.

Example-18

LCMSMS STUDIES

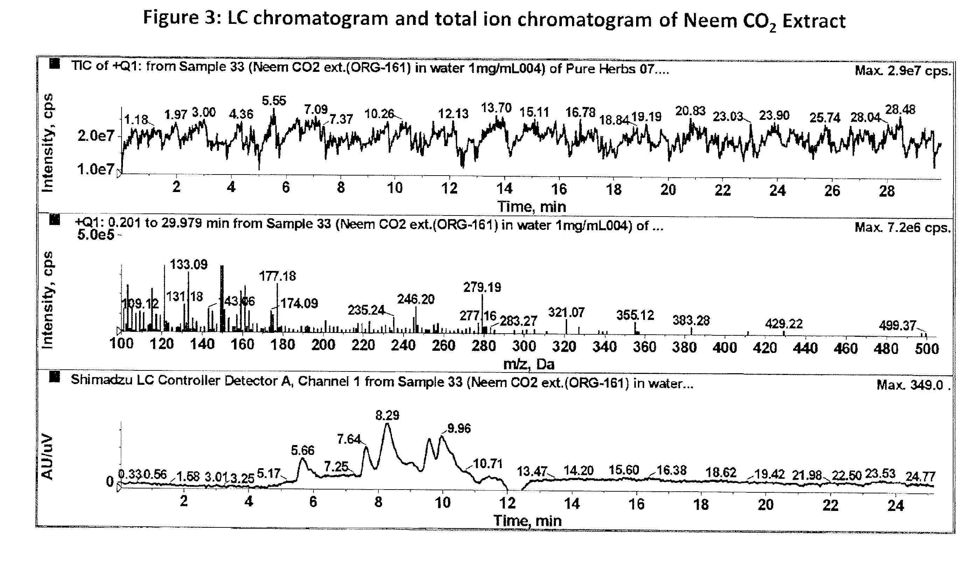

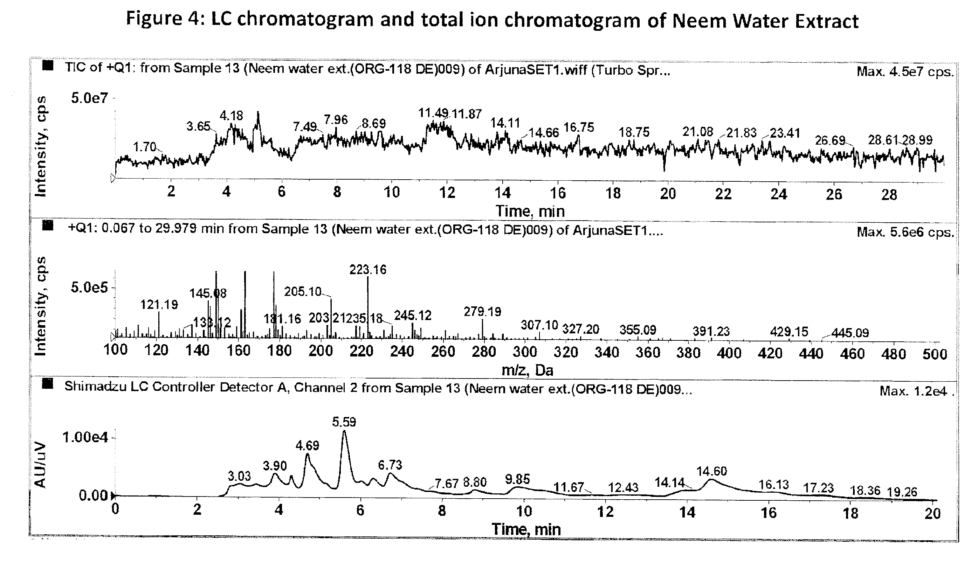

Liquid Chromatography -Mass Spectrometer Analysis of *Azadirachta indica* leaves, supercritical ($CO_2$) extract and water extract (Figure 3 and 4):
LCMSMS analysis were carried out by using an applied biosystem-Sciex API 2000 triple quadrupole mass spectrometer equipped with an ion source turbo spray and ESI interface. The liquid chromatography was a LC-20 AD series binary system equipped with an autosampler. The column used was C18 phenomenex (250 X 4.6mm, 5$\mu$m), flow rate 0.5ml/min of mobile phase methanol: water (0.1% acetic acid), (10:90), wave length 254 nm and run time 25 min. The analytes were ionized by ESI in positive-ion mode (PI mode). Final ionization conditions were heated ionization temperature 435°C. Curtain gas Nitrogen 30 psi, particulate -free and $CO_2$- free air was used as ion source gas 1 at a flow rate 50 psi and ion source gas 2 at a flow rate 60 psi.

Sample preparation for *Azadirachta indica* leaves $CO_2$ extract:

Weigh about 50mg of sample in a 5 0ml volumetric flask, and dissolve by sonication in methanol (HPLC grade). Make the volume up to the mark with methanol filter through 0.2 um syringe filter.

Sample preparation for *Azadirachta indica* leaves water extract:

Weigh about 500 mg of finely powdered sample in a 50 ml volumetric flask, and dissolve by sonication in water (HPLC grade). Make the volume up to the mark with water filter through 0.2 um syringe filter

Example-19

Preparation of Trikatu (*Zingiber officinale* rhizome, *Piper longum* fruits, *Piper nigrum* fruits, (1:1:1)) water extract

Approximately 100 Kg of shade dried plant material was subjected to extraction with 450 Liters of purified water by percolation method at Room Temperature. The water extractions after 24 - 48 hours were filtered through muslin cloth and concentrated to thick paste. After achieving the desired total solid content, the soft extract was spray dried to a free flowing dry extract powder. The water extract was also prepared by hot soxhlation method.

Example-20

Preparation of Trikatu (*Zingiber officinale* rhizome, *Piper longum* fruits, *Piper nigrum* fruits, (1:1:1)) SCFE ($CO_2$) Extract

Approximately 25 Kg of Trikatu material was pulverized to fine powder and loaded in to extractor. Super Critical Carbon dioxide liquid was pumped in to the extractor at a pressure of 325 bar and 32 °C temperature for 2-4 hours. Extract was separated into the container at pressure of 45 bar and 21 °C temperature. The $CO_2$ super critical liquid was recycled from the extraction vessel.

Example-21

Batch Formula for Preparation of Organic Trikatu Granules (Formula-1), Table-15

Table-15

| S. No. | Raw Material | Weight per Tablet in mg | Weight per Batch In kg |
|---|---|---|---|
| 1 | Water extract of Trikatu (60# mesh) | 280.00 | 28.00 |
| 2 | Super critical fluid extract (SCFE, $CO_2$ extract) of Trikatu | 20.00 | 2.00 |
| 4 | Trikatu plant powder (60 # mesh) | 340.00 | 34.00 |
| 5 | Purified Water | Qs* | Qs* |

Formula of Lubrication of Trikatu granules, Table-16

Table-16

| 1 | Trikatu granules (# 16mesh) | Granules | 640.00 | 64.00 |
|---|---|---|---|---|
| 2 | Trikatu plant powder (60# mesh) | Lubricant | 50.00 | 5.00 |
| | | Total | 690.00 | 69.00 |

Example-22

Manufacturing details of Trikatu tablets

DISPENSING OF RAW MATERIAL

1. Dispense the raw materials as per Batch record.

DRY HEAT STERILIZATION

1. Transfer Trikatu plant powder into trays and sterilize the material at 160 °C for 2 hr.
2. Unload the sterilized material in to double lined poly bags separately and keep in airtight containers.
3. Analyse the sample for LOD, BD and Microbiological Analysis. Table-17

Table-17

|   | Parameter | Standard values |
|---|---|---|
| 1 | LOD | 1.0 - 4.0 % w/w |
| 2 | BD | 0.25 - 0.60 g/ml |
| 3 | TVAC | NMT 5000 cfu/gm |
| 4 | Fungal count | NMT 10 cfu/gm |

SIFTING

1. Sift 5.0 kg of water extract powder of Trikatu through # 60.
2. Sift 34.0 and 5.0 kg of Trikatu plant powder through # 60.
3. Collect the above-sifted materials in separate duly labeled double lined poly bags.

PREPARATION OF GRANULATION FLUID

1. Transfer about 10 kg of Purified water into a clean Stainless Steel Vessel.

GRANULATION

1. Charge water extract powder of Trikatu, and Trikatu plant powder into the RMG, mix for about 5 minutes.
2. Slowly add super critical fluid extract ($CO_2$) of Trikatu, and granulation fluid to the RMG containing the Sifted extract powder of Trikatu, and Trikatu plant powder over a period of about 3 minute, with medium speed.
3. Stop the mixer and scrape off the mass from the sides and bottom.
4. Continue mixing by operating the impeller at high speed with Chopper ON for about 3 minute.
5. Add additional quantity of granulation fluid, if required.
6. Discharge the mass from the RMG.

WET MILLING

1. Mill the Wet Mass in Multi mill fitted with 8mm screen.

TRAY DRYING

1. Dry the Wet mass obtained in tray Drier at about 60 °C to 70 °C for about 60 minutes.

SIZING

1. Sift the dried granules from Sifter fitted with sieve #16.
2. Collect the sifted granules in a clean double poly lined HDPE container.
3. Mill the retains (oversize granules) obtained through a Multi Mill fitted with mm screen with 'Knives Forward' direction
4. Pass the milled granules through a Sifter fitted with #16 sieve.
5. Weigh the granules analyse as per Table-18

Table-18

|   | Parameter | Standard values |
|---|-----------|-----------------|
| 1 | LOD | 2.0- 5.0 % w/w |
| 2 | BD | 0.30 - 0.60g/ml |
| 3 | TVAC | NMT 5000 cfu/gm |
| 4 | Fungal count | NMT 10 cfu/gm |

BLENDING

1. Transfer the sized granules into the blending area.
2. Transfer the sifted trikatu herb powder lubricant into the blending area.
3. Load trikatu herb powder and the sized granules into the Double Cone blender.
4. Blend the ingredients for 6 minutes at 10-11 RPM.
5. Record the details
6. Unload the blend in a clean Double poly-lined HDPE drums and affix duly filled status labels.
7. Weigh the blend and enter the details.

COMPRESSION

1. Check the Temperature and Relative Humidity of the area and record.
2. Bring the drums containing the blend into the compression area.
3. Adjust the machine.
4. Carry out the initial checks before starting the operation as specified in below.

Table-19

|   | Description |
|---|-------------|
| Punch Size | 17x8 mm caplet |
| Upper Punch | Plain |
| Lower Punch | Plain |

5. Check for Appearance, Average Weight, Individual weight, Thickness, Hardness, Friability & DT of 6 tablets.
6. Check appearance, thickness & hardness of tablets every 30 min.
7. Check Friability and DT every 1-hour.
8. Check Average weight of 20 tablets every 30 mins graphically.
9. Collect the tablets in a double poly-lined, tightly closed, container. Weigh each container and enter the details.

Example-23

Finished product specification of Trikatu per caplet. Table-20

Table-20

| Parameters | Standard Range |
|---|---|
| Theoretical Average weight | 690 mg |
| Weight uniformity | 690 mg $\pm$ 5% (655 mg to 725 mg) |
| Weight of 20 tablets | 13.8 g $\pm$ 3% (13.39 gm to 14.21 gm) |
| Tablet thickness | 3.5 to 6.5 mm |
| Tablet hardness | 2 to 8 Kg/cm$^2$ |
| Friability | NMT 1.0% W/W |
| Disintegration time | NMT 30 min |
| Gingerols | 3 mg per caplet |
| Piperine | 1.09 mg per caplet |

1. Tablets should be packed immediately after compression.
2. The granules can also be filled in capsules.

PACKING

1. Pack 60 tablets in a HDPE 120 CC container and weigh them for appropriateness of number of tablets.

Example-24

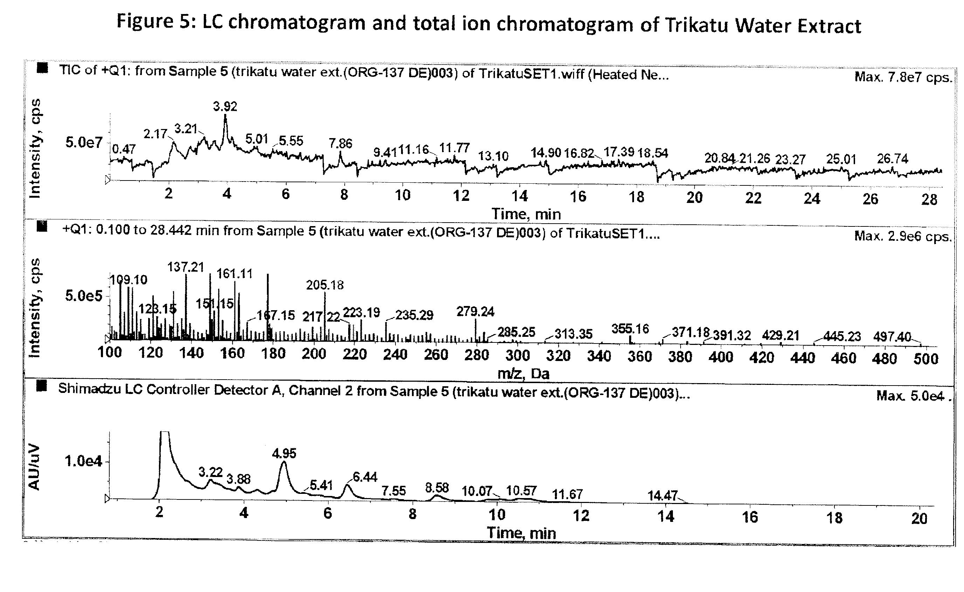

Liquid Chromatography -Mass Spectrometer Analysis of Trikatu water extract: (Figure 5)

LCMSMS analysis was carried out by using an applied biosystem-Sciex API 2000 triple quadrupole mass spectrometer equipped with an atmospheric pressure chemical ionization source and heated nebulizer APCI interface. The liquid chromatography was a LC-20 AD series binary system equipped with an autosampler. The column used was C18 phenomenex (250 x 4.6 mm, 5 $\mu$m), flow rate 1 ml/min of mobile phase water (0.1% acetic acid) (100%), wave length 275 nm and run time 20 min. The analytes were ionized by APCI in positive - ion mode (PI mode). Final ionization conditions were heated nebulizer temperature 450 °C, curtain gas Nitrogen 25 psi, particulate - free and $CO_2$ - free air was used as nebulising gas at a flow rate of 70 psi.

Sample preparation for Trikatu water extract:

Weigh about 500mg of sample in 50ml volumetric flask, and dissolve by sonication in water. Make the volume up to the mark with water and filter through 0.2$\mu$m syringe filter. LCMSMS chromatograms are presented in Figure-5

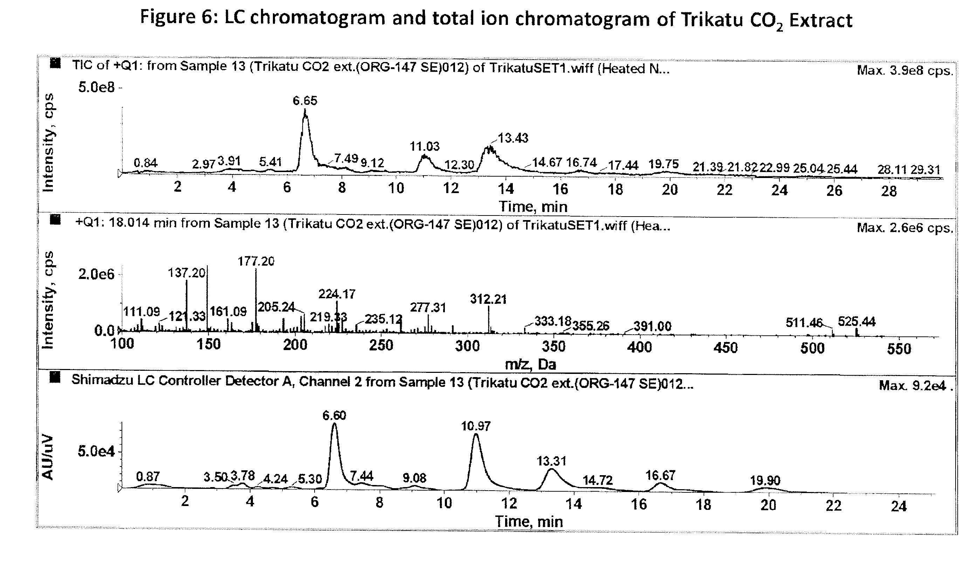

Liquid Chromatography -Mass Spectrometer Analysis of Trikatu Super Critical Extract $CO_2$) extract: (Figure 6)

LCMSMS analysis were carried out by using an applied biosystem-Sciex API 2000 triple quadrupole mass spectrometer equipped with an atmospheric pressure chemical ionization source and heated nebulizer APCI interface. The liquid chromatography was a LC-20 AD series binary system equipped with an autosampler. The column used was C18 phenomenex (250 X 4.6mm, 5$\mu$m), flow rate 1ml/min of mobile phase methanol: water (0.1% acetic acid) ; (65 : 35), wave length 275nm and run time 20 min. The analytes were ionized by APCI in positive -ion mode (PI mode). Final ionization conditions were heated nebulizer temperature 450°C, curtain gas Nitrogen 25 psi, particulate - free and $CO_2$ - free air was used as nebulising gas at a flow rate of 70 psi.

Sample preparation for Trikatu super critical extract ($CO_2$) extract:

Weigh about 50 mg of sample in 50 ml volumetric flask, and dissolved by sonication in methanol. Make the volume up to the mark with methanol and filter through 0.2$\mu$m syringe filter.
LCMSMS chromatogram are presented in Figure-6

Example-25

Preparation of *Tinospora cordifolia* (Guduchi) water extract

Approximately 100 Kg of shade dried plant material stem was subjected to extraction with 400 Liters of purified water by percolation method at Room Temperature. The water extractions after 24 hours were filtered through muslin cloth and concentrated to thick paste. After achieving the desired total solid content, the soft extract was spray dried to a free flowing dry extract powder.

Example-26

Preparation of *Tinospora cordifolia* (Guduchi) SCFE ($CO_2$) Extract

Approximately 25 Kg of the stem of *Tinospora cordifolia* was pulverized to fine powder and loaded in to extractor. Super Critical Carbon dioxide liquid was pumped into the extractor at a pressure of 300 bar and 39 °C temperature for 2-3 hours. Extract was separated into the container at pressure of 40 bar and 20 °C temperature. The $CO_2$ super critical liquid was recycled from the extraction vessel.

Example-27

Formula of Granulation (Table-21)

| S. No. | Name of the material | Weight per Tablet in mg | Weight per Batch in Kg |
|---|---|---|---|
| 1 | *Tinospora cordifolia* stem (Water extract) (# 40 mesh) | 200.00 | 20.00 |
| 2 | *Tinospora cordifolia* stem (Super critical fluid ($CO_2$) extract) | 50.00 | 5.00 |
| 3 | *Tinospora cordifolia* stem (#40 mesh) powder | 400.00 | 40.00 |
| 4 | Purified water | Granulation fluid | Quantity sufficient |

Example-28

Formula of Lubrication of granules (Table-22)

| S. No. | Name of the material | Weight per Tablet in mg | Weight per Batch in Kg |
|---|---|---|---|
| 1 | *Tinospora cordifolia* Extract Granules (# 16 mesh) | 650.00 | 65.00 |
| 2 | *Tinospora cordifolia* stm powder (#80) powder | 50.00 | 5.00 |
| | Total | 700.00 | 70.00 |

Example-29

DISPENSING OF RAW MATERIAL

1. Dispense the raw materials as per Batch Formula.

DRY HEAT STERILIZATION

1. Transfer 45.0 kg of stem powder of *Tinospora cordifolia* in trays and sterilize the materials at 160 °C for 2 hour.

2. Unload the sterilized materials in to double- lined poly bags separately and keep in air tight containers.
3. Analyse the sample for LOD, BD and microbial analysis. Table-23

Table-23

|   | Parameter | Standard values |
|---|---|---|
| 1 | LOD | 1.0 - 4.0% |
| 2 | BD | 0.25 - 0.60 g/ml |
| 3 | TVAC | NMT 5000 cfu/gm |
| 4 | Fungal Count | NMT 10 cfu/gm |

SIFTING

1. Sift 20.0 kg of water extract of stem of *Tinospora cordifolia* through # 40.
2. Sift 5.0 kg of water extract of stem of *Tinospora cordifolia* through # 40.
3. Sift 40.0 kg of stem plant powder through # 80.
4. Sift 5.00 kg of stem plant powder lubricant through # 80 separately.
5. Collect the above-sifted materials in separate duly labeled double lined polybags.
6. Record Quantity Sifted and the sieve integrity before and after sifting.

PREPARATION OF GRANULATION FLUID

1. Transfer about 20.0 kg of Purified water into a clean Stainless Steel Vessel

GRANULATION

1. Charge 20.00 Kg of water extract of stem of *Tinospora cordifolia*, 5.0 kg of super critical extract ($CO_2$) of stem of *Tinospora cordifolia*, 40.0 kg of stem plant powder into the RMG, mix for 5 minute.
2. Slowly add the granulation fluid to the RMG containing the Sifted water extract of stem of *Tinospora cordifolia*, stem plant powder over a period of about 3 minute, with medium speed.
3. Stop the mixer and scrape off the mass from the sides and bottom.
4. Continue mixing by operating the impeller at high speed with Chopper ON for about 3 minute.
5. Add additional quantity of purified water, if required.
6. Discharge the mass from the RMG.

WET MILLING

2 Mill the Wet Mass obtained in Multi mill fitted with 8 mm screen.

TRAY DRYING

1. Dry the Wet mass obtained in tray Drier at about 70°C to 80°C for about 60 minutes.
2. Check the Moisture once every 30 minutes.( LOD Limit: 2.0 to 5.0%w/w) and record the details

SIZING

1. Sift the dried granules using a Sifter fitted with sieve #16.
2. Collect the sifted granules in a clean double poly-lined HDPE container.
3. Mill the retains (oversize granules) obtained through a Multi Mill fitted with 1.5 mm screen with 'Knives Forward' direction.
4. Pass the milled granules obtained through a Sifter fitted with #16 sieve.
5. Collect the sized granules obtained.
6. Weigh the granules.
7. Analyse samples as per below Table-24

Table-24

|   | Parameter | Standard value |
|---|---|---|
| 1 | LOD | 2.0 - 5.0 % |
| 2 | BD | 0.30 - 0.60 g/ml |
| 3 | Granules: Fine ratio | 20:80 - 80:20 |
| 4 | TVAC | NMT 5000 cfu/gm |
| 5 | Fungal count | NMT 10 cfu/gm |

BLENDING

1. Transfer the sized granules to the blending area.
2. Transfer the sifted stem plant powder of *Tinospora cordifolia* lubricant to the blending area.
3. Load 5.0 kg of stem plant powder of *Tinospora cordifolia* and the sized granules into the double cone blender.
4. Blend the ingredients for about 6 minutes at 10-11 RPM.
5. Unload the blend in a clean Double poly-lined HDPE drums and affix duly filled status labels.
6. Weigh the blend and enter the details.

COMPRESSION

Maintain the temperature and relative humidity of the area with in the specified limit (Limit temperature NMT 25 °C and relative humidity NMT 40%)
1. Check the Temperature and Relative humidity of the area and record.
2. Bring the drums containing the blend into the compression area.
3. Adjust the machine.
4. Carry the initial checks before starting the operation as specified in below table-25

Table-25

|   | Description |
|---|---|
| Punch size | 17x8 mm caplet |
| Upper punch | Plain |
| Lower punch | Plain |

5. Check for Appearance, Average Weight., Individual Weight., Thickness, Hardness, and Friability & DT of 6 tablets.
6. Check appearance, thickness & hardness of tablets for every 30 min.
7. Check friability and DT every 1-hour
8. Check for the Average weight of 20 tablets every 30 mins graphically
9. Collect the tablets in a double poly-lined, tightly closed container. Weigh each container and enter the details.

STANDARD PARAMETERS FOR TABLETS:

Table-26

| Sl.No. | Parameters | Standard value |
|---|---|---|
| 1 | Theoretical average weight | 700 mg |
| 2 | Weight uniformity | 700 mg $\pm$ 5% (665mg to 735 mg) |
| 3 | Weight of 20 tablets | 14.00 g $\pm$ 3% (13.58 gm to 14.42gm) |
| 4 | Tablet thickness | 3.5 to 6.5 mm |
| 5 | Tablet hardness | 2 to 8 Kg/cm$^2$ |
| 6 | Friability | NMT 1.0% W/W |
| 7 | Disintegration time | NMT 30 min. |
| 8 | Total Bitters containing Tinosporaside | 24.6 mg per caplet |

Tablets to be packed immediately after compression.

Packing

2. Pack 60 tablets in a HDPE 120 CC container and weigh them for appropriateness of number of tablets.

Example-30

LCMSMS STUDIES

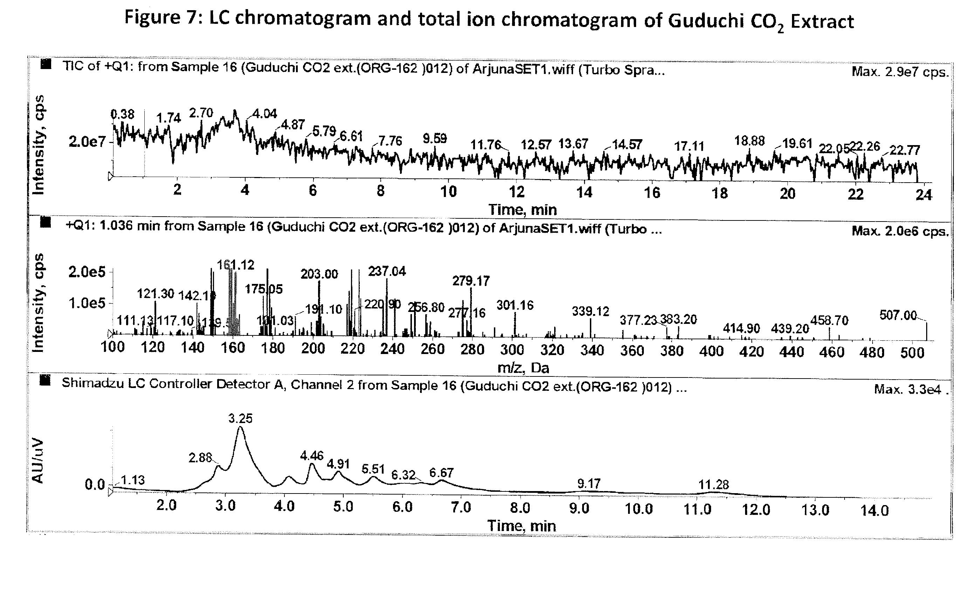

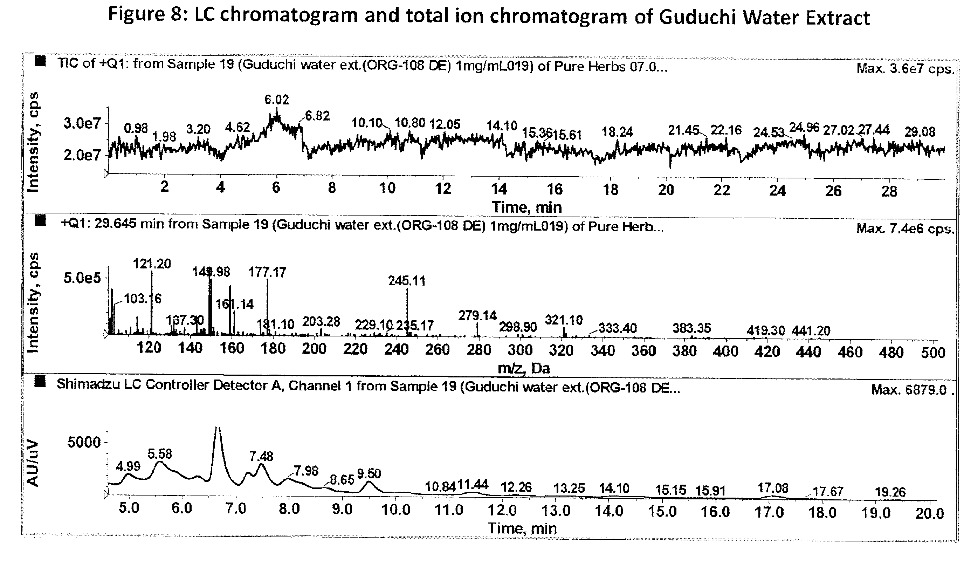

Liquid Chromatography - Mass Spectrometer Analysis of *Tinospora cordifolia* stem supercritical ($CO_2$) extract and water extract (Figure 7 & 8):

LCMSMS analysis were carried out by using an applied biosystem-Sciex API 2000 triple quadrupole mass spectrometer equipped with an ion source turbo spray and ESI interface. The liquid chromatography was a LC-20 AD series binary system equipped with an autosampler. The column used was C18 phenomenex (250 X 4.6mm, 5$\mu$m), flow rate 0.5 ml/min of mobile phase methanol: water (0.1 % acetic acid), (10:90), wave length 254 nm and run time 25 min. The analytes were ionized by ESI in positive-ion mode (PI mode). Final ionization conditions were heated ionization temperature 435 °C. Curtain gas Nitrogen 30 psi, particulate -free and $CO_2$- free air was used as ion source gas 1 at a flow rate 50 psi and ion source gas 2 at a flow rate 60 psi.

Sample preparation for *Tinospora cordifolia* stem $CO_2$ extract:

Weigh about 50 mg of sample in a 50 ml volumetric flask, and dissolve by sonication in methanol (HPLC grade). Make the volume up to the mark with methanol filter through 0.2um syringe filter.

Sample preparation for *Tinospora cordifolia* stem water extract:

Weigh about 500 mg of finely powdered sample in a 50 ml volumetric flask, and dissolve by sonication in water (HPLC grade). Make the volume up to the mark with water filter through 0.2 um syringe filter.

Example-31

Preparation of *Ocimum sanctum* water extract

Approximately 100 Kg of shade dried leaves of *(Ocimum sanctum)* was subjected to extraction with 400 Liters of purified water by percolation method at Room Temperature. The water extractions after 24 hours were filtered through muslin cloth and concentrated to thick paste. After achieving the desired total solid content, the soft extract was spray dried to a free flowing dry extract powder.

Example-32

Preparation of *Ocimum sanctum* SCFE ($CO_2$) extract

Approximately 25 Kg of leaves of *Ocimum sanctum* was pulverized to fine powder and loaded in to extractor. Super Critical Carbon dioxide liquid was pumped in to the extractor at a pressure of 350 bar and 40°C temperature for 2-4 hours. Extract was separated into the container at pressure of 40 bar and 20°C temperature. The $CO_2$ super critical liquid was recycled from the extraction vessel.

Example-33

Formula of Granulation for Tulsi capsules (Table-27)

| S. No | Name of the material | Weight per Tablet in mg | Weight per Batch in Kg |
|---|---|---|---|
| 1 | *Ocimum sanctum* leaves powder (Water extract) (# 40) | 160.00 | 16.00 |
| 2 | *Ocimum sanctum* leaves (Super critical fluid ($CO_2$) extract) | 60.00 | 6.00 |
| 3 | *Ocimum sanctum* leaves (#80 mesh) powder | 500.00 | 50.00 |
| 4 | Purified water | Granulation fluid | Quantity sufficient |

Example-34

Formula of Lubrication of granules for Tulsi capsules (Table-28)

| S. No. | Name of the material | Weight per Tablet in mg | Weight per Batch in Kg |
|---|---|---|---|
| 1 | *Ocimum sactum* Extract Granules (# 16 mesh) | 720.00 | 72.00 |
| 2 | E VEG CAPSULES CT/CT "00" WI/OUT LOGO, empty veg capsule | | 100000 |
| | Fill weight | 720.00 | 72.00 |

Example-35

Formula of Granulation for Tulsi tablets (Table-29)

| S. No | Name of the Material | Weight per Tablet in mg | Weight per Batch In kg |
|---|---|---|---|
| 1 | *Ocimum sanctum* leaves powder (Water extract) (# 40) Dry extract | 220.00 | 22.00 |
| 2 | *Ocimum sanctum* leaves (Super critical fluid (CO2) extract) | 30.00 | 3.00 |
| 3 | *Ocimum sanctum* leaves (#80 mesh) powder | 400.00 | 40.00 |
| 4 | Granulation fluid | Quantity sufficient | |

Example-36

Formula of Lubrication of granules for Tulsi tablets (Table-30)

| 1 | *Ocimum sanctum* leaves granules (# 16 mesh) | Granules | 650.00 | 65.00 |
|---|---|---|---|---|
| 2 | *Ocimum sanctum* leaves ( # 80 mesh ) powder | Lubricant | 50.00 | 5.00 |
| | | Total | 700.00 | 70.00 |

Example-37

Manufacturing details of Tulsi Capsules

DISPENSING OF RAW MATERIAL

1. Dispense the raw materials as per Batch Formula.

DRY HEAT STERILIZATION

1. Transfer 50.0 kg of leaves powder of *Ocimum sanctum* in to trays and sterilize the material at 160 °C for 60 mins
2. Unload the sterilized materials in to double lined polybags separately and keep in airtight containers.
3. Analyze the sample for LOD, BD and Microbiological Analysis Table-31

Table-31

| S.No | Parameter | Standard values |
|---|---|---|
| 1 | LOD | 2.5 - 3.5% |
| 2 | BD | 0.30 - 0.40g/ml |
| 3 | Microbes | NMT 10000 cfu/gm |
| 4 | Fungal count | NMT 100 cfu/gm |

SIFTING

1. Sift water extract of leaves powder of *Ocimum sanctum* through # 40 and leaves powder of *Ocimum sanctum* through #80, weigh as per the required quantity and kept separately in duly-labeled double lined poly bag.
2. Record Quantity Sifted and the sieve integrity before and after sifting.

PREPARATION OF GRANULATION FLUID

1. Transfer about 20.0 kg of Purified water into a clean Stainless Steel Vessel
2. Record the observations. Record deviations if any.

GRANULATION

1. Charge 50.00 Kg of leaves powder of *Ocimum sanctum,* add slowly 6.0 kg of super critical extract ($CO_2$) of leaves

of *Ocimum sanctum* into the RMG mix for 5 minutes.

2. Add 16.00 kg of water extract of leaves of *Ocimum sanctum* to the above blend. Mix it for 5 minutes.

3. Add granulation fluid to RMG and granulate. If required add additional quantity of purified water, mix over a period of about 3 minutes, with medium speed.

4. Stop the mixer and scrape off the mass from the sides and bottom.

5. Continue mixing by operating the impeller at high speed with Chopper ON for 1 minute.

6. Add additional quantity of purified water, if required.

7. Discharge the mass from the RMG.

8. Record the observations. Record deviations if any.

WET MILLING

1. Mill the Wet Mass in Multi mill fitted with 8 mm screen.

DRYING

1. Dry the Wet in FBD/Tray drier at about 55-60 °C for about 60-90 minutes.

2. Check the Moisture once every 30 minutes. (LOD Limit: 2 - 4%) and record the details.

SIZING

1. Sift the dried granules using a Sifter fitted with sieve #18.

2. Collect the sifted granules in a clean double poly - lined HDPE container.

3. Mill the retains (oversize granules) through a Multi Mill fitted with 1.5 mm screen with 'Knives Forward' direction.

4. Pass the milled granules through a Sifter fitted with #16 sieve.

5. Analyze the sample.

6. In-process parameters are in Table-32

Table-32

|   | Parameter | Standard values |
|---|---|---|
| 1 | LOD | 1 - 2% |
| 2 | BD | 0.60 - 0.70 g/ml |
| 3 | Granules to fine ratio | 60:40 to 70:30 |
| 4 | Actives | As per Finished Product spec |
| 5 | Microbes | NMT 10000 cfu/gm |
| 6 | Fungal count | NMT 100 cfu/gm |

CAPSULE FILLING

Note: Maintain the Temperature and Relative Humidity of the area within the specified limit (Temperature 25 °C $\pm$ 2 °C and Relative humidity 40% $\pm$ 2 %)

1. Check the Temperature and Relative Humidity of the area and record.

2. Bring the drums containing the granules into the capsule filling area.

3. Adjust the machine.

4. Check for Appearance, Average Weight, Individual weight, average locking length of 10 capsules& DT of 6 capsules.

5. Check DT every 1-hour.

6. Check Average weight of 20 capsules every 30 min graphically.

7. Collect the capsules in a double poly-lined, tightly closed, container.

8. Weigh each container and enter the details.

9. In process specification for capsules Table-33

Table-33

| Parameter | Standard limit |
|---|---|
| Description | Clear Transparent size 00 capsules filled with brown colored granules |
| Weight of empty capsules | 115-120 mg |
| Fill weight | 720 mg |
| Average weight | 840 ± 5% |
| Weight 20 capsules | 16.8 gm ± 3% |
| Average locking length of 10 capsules | 23.3 ± 0.4 mm |
| Disintegration | NMT 15 min |

8. Collect the filled capsules in double poly-lined HDPE drums and record their weights.

9. Affix duly filled status labels to the containers.

PACKING

1. Pack 60 capsules in a HDPE 120 CC container and weigh them for appropriateness of number of capsules

Example-38

Manufacturing Details of Tulsi Tablets

Dry Heat Sterilization

1. Transfer the leaves plant powder of *Ocimum sanctum* into trays and sterilize the material at 160 °C for 60 minutes.
2. Unload the autoclaved materials in to double lined polybags separately and keep in airtight containers.
3. Analyse sample for LOD, BD and Microbiological Analysis as per Table-34

Table-34

| | Parameter | Standard values |
|---|---|---|
| 1 | LOD | 2.0 - 3.0% |
| 2 | BD | 0.35 - 0.55 g/ml |
| 3 | TVAC | NMT 5000 cfu |
| 4 | Fungal count | NMT 10 cfu |

SIFTING

1. Sift water extract of leaves of *Ocimum sanctum* through # 40 mesh.
2. Sift leaves plant powder of *Ocimum sanctum* through # 80 mesh.
3. Collect the above-sifted materials in separate duly labeled double lined polybags.

PREPARATION OF GRANULATION FLUID

1. Transfer purified water into a clean Stainless Steel Vessel

GRANULATION

1. Charge of water extract of leaves of *Ocimum sanctum* and of leaves powder of *Ocimum sanctum* into the RMG, mix for 5 minutes.
2. Slowly add super critical extract ($CO_2$ extract) of leaves of *Ocimum sanctum,* and mix for about 3 minutes.
3. Add granulation solution to the above blend and mix for about 3 minutes, with medium speed.
4. Stop the mixer and scrape off the mass from the sides and bottom.
5. Continue mixing by operating the impeller at high speed with Chopper ON, if required.
6. Add additional quantity of Purified water, if required.
7. Discharge the mass from the RMG.

WET MILLING

1. Mill the Wet Mass obtained in Multi mill fitted with 8mm screen.

TRAY DRYING

1. Dry the Wet mass in tray Drier at about 70°C to 80°C for about 60 minutes.

SIZING

1. Sift the dried granules using a Sifter fitted with sieve #16.
2. Collect the sifted granules in a clean double poly-lined HDPE container.
3. Mill the retains (oversize granules) through a Multi Mill fitted with 1.5 mm screen with 'Knives Forward' direction.
4. Pass the milled granules obtained through a Sifter fitted with #16 sieve.
5. Collect the sized granules obtained and add them to the sifted granules.

BLENDING

Granules were analysed as per table-35

Table-35

| | Parameter | Standard values |
|---|---|---|
| 1 | LOD | 3 .0 - 4.0 % w/w |
| 2 | BD | 0.35 - 0.55 g/ml |
| 3 | TVAC | NMT 5000 cfu |
| 4 | Fungal count | NMT 100 cfu |

Maintain the Temperature and Relative Humidity of the area within the specified limit (Limit Temperature NMT 25 °C and relative humidity NMT 40%)

1. Transfer the sized granules into the blending area.
2. Transfer the sifted leaves powder of *Ocimum sanctum* lubricant into the blending area.

3. Load 5.0 kg of leaves powder of *Ocimum sanctum* and the sized granules into the Double Cone blender.
4. Blend the ingredients for 5 minutes at 10-15 RPM.
5. Unload the blend in a clean Double poly-lined HDPE drums and affix duly filled status labels.
6. Weigh the blend and enter the details.

COMPRESSION

Maintain the Temperature and Relative Humidity of the area with in the specified limit (Limit Temperature NMT 25 °C and relative humidity NMT 40%)
5. Check the Temperature and Relative Humidity of the area and record.
6. Bring the drums containing the blend into the compression area.
7. Adjust the machine as per the parameters.
8. Carry out the initial checks before starting the operation as specified in below Table-36

Table-36

|  | Description |
|---|---|
| Punch Size | 17x 8 mm caplet |
| Upper Punch | Plain |
| Lower Punch | Plain |

10. Check for Appearance, Average weight, Individual weight, Thickness, Hardness, Friability & DT of 6 tablets.
11. Check appearance, thickness & hardness of tablets every 30 min.
12. Check Friability and DT every 1-hour.
13. Check Average weight of 20 tablets every 30 mins graphically.
14. Collect the tablets in a double poly-lined, tightly closed, container. Weigh each container and enter the details.

STANDARD PARAMETERS OF TABLETS

Standard parameters are mentioned in Table-37

Table-37

| S.No. | Parameters | Standard value |
|---|---|---|
| 1 | Theoretical average weight | 700 mg |
| 2 | Weight uniformity | 700 mg $\pm$ 5% (705mg to 695mg) |
| 3 | Tablet thickness | 4.0 to 5.0 mm |
| 4 | Tablet hardness | 4 to 6 Kg/cm$^2$ |
| 5 | Friability | NMT 1.0% W/W |
| 6 | Disintegration time | NMT 30 min. |
| 7 | Total Oleonolic acids | 3.5 mg per caplet |

12. Collect the Compressed tablets in Double poly-lined HDPE drums and record their weights.
13. Affix duly filled status labels to the containers.

PACKING

14. Pack 60 tablets in a HDPE 120 CC container and weigh them for appropriateness of number of tablets.

Example-39

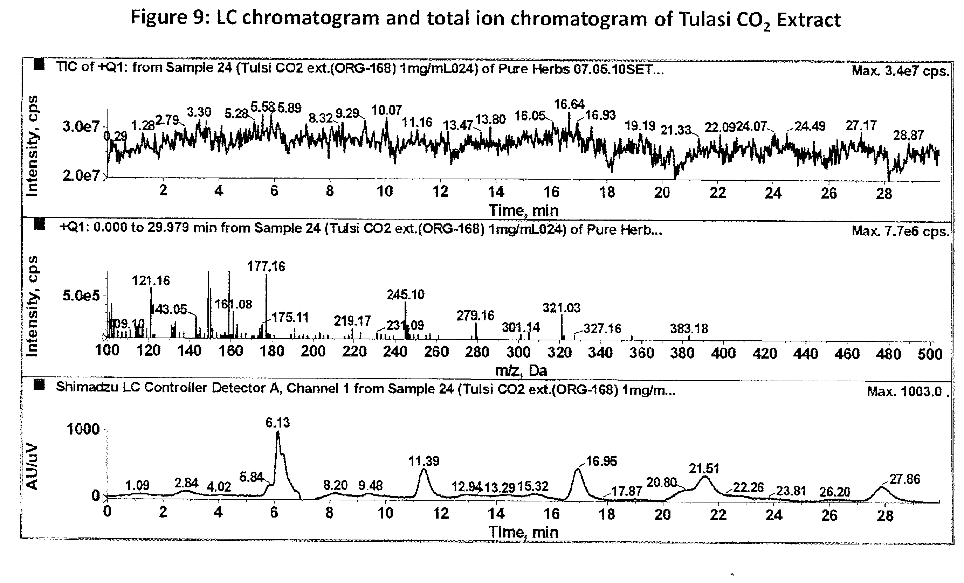

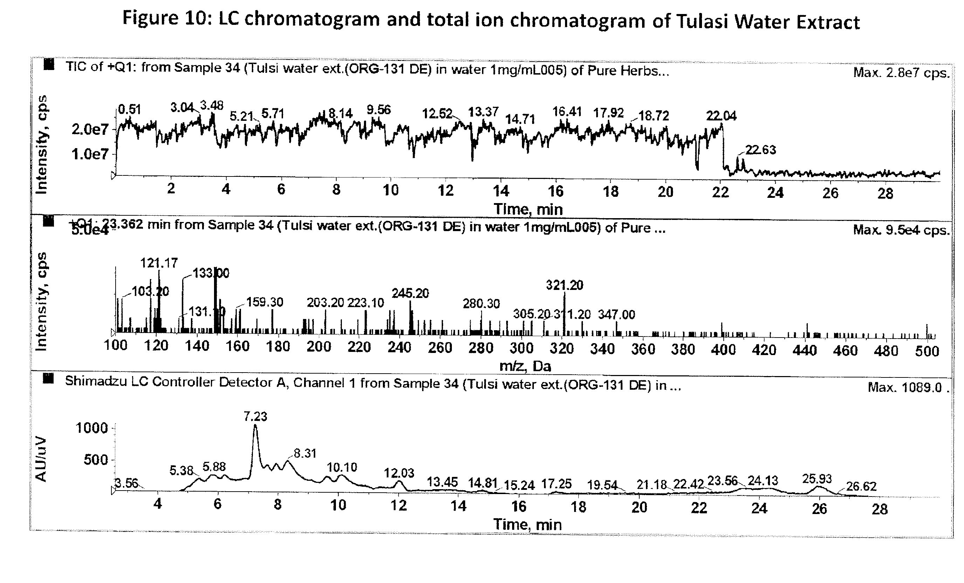

Liquid Chromatography -Mass Spectrometer Analysis of *Ocimum sanctum* leaves, supercritical ($CO_2$) extract and water extract (Figure 9 and 10):

LCMSMS analysis were carried out by using an applied biosystem-Sciex API 2000 triple quadrupole mass spectrometer equipped with an ion source turbo spray and ESI interface. The liquid chromatography was a LC-20 AD series binary system equipped with an autosampler. The column used was C18 phenomenex (250 X 4.6mm, 5$\mu$m), flow rate 0. 5 ml/min of mobile phase methanol: water (0.1% acetic acid), (10:90), wave length 254 nm and run time 25 min. The analytes were ionized by ESI in positive-ion mode (PI mode). Final ionization conditions were heated ionization temperature 435 °C. Curtain gas Nitrogen 30 psi, particulate -free and $CO_2$- free air was used as ion source gas 1 at a flow rate 50 psi and ion source gas 2 at a flow rate 60 psi.

Sample preparation for *Ocimum sanctum* leaves $CO_2$ extract:

Weigh about 50 mg of sample in a 50 ml volumetric flask, and dissolve by sonication in methanol (HPLC grade). Make the volume up to the mark with methanol filter through 0.2 um syringe filter.

Sample preparation for *Ocimum sanctum* leaves water extract:

Weigh about 500 mg of finely powdered sample in a 50 ml volumetric flask, and dissolve by sonication in water (HPLC grade). Make the volume up to the mark with water filter through 0.2 um syringe filter.

Example-40

Preparation of *Withania somnifera* water extract

Approximately 100 Kg of shade dried plant material was subjected to extraction with 400 litres of purified water by percolation method at room temperature. The water extractions after 24-48 hours were filtered through muslin cloth and concentrated to thick paste. After achieving the desired total solid content, the soft extract was spray dried to a free flowing dry extract powder. The water extract was also prepared by hot soxhalation method.

Example-41

Preparation of *Withania somnifera* SCFE ($CO_2$) Extract

Approximately 25 Kg of the roots of *Withania somnifera* was pulverized to fine powder and loaded in to extractor. Super Critical Carbon dioxide liquid was pumped into the extractor at a pressure of 300 bar and 39°C temperature for 2-3 hours. Extract was separated into the container at pressure of 40 bar and 20°C temperature. The $CO_2$ super critical liquid was recycled from the extraction vessel. The resultant extract was analyzed for active markers of *Withania somnifera.*

Example-42

Preparation of Organic Withania Granules (Formula-1)

Table-38

| S. No. | Name of the Material | Weight per Tablet in mg | Weight per Batch in kg |
|---|---|---|---|
| 1 | *Withania somnifera* (water extract) (#40 mesh) | 280.00 | 28.00 |
| 2 | *Withania somnifera* (supercritical fluid extract, $CO_2$) | 10.00 | 1.00 |
| 3 | *Withania somnifera* roots parts ( # 80mesh) | 380.00 | 38.00 |

Example-43

Formula of Lubrication of granules

Table-39

| S. No. | Name of the material | Weight per Tablet in mg | Weight per Batch in Kg |
|--------|----------------------|-------------------------|------------------------|
| 1 | *Withania somnifera* Extract Granules (# 16 mesh) | 670 | 67.00 |
| 2 | *Withania somnifera* (#40 mesh) powder | 0 | 0 |
| | Total | 670 | 67.00 |

Example- 44

Manufacturing procedure of granulation and compression

Dispensing and sifting of raw materials and extracts:

1. Dispensed the raw materials as per Batch Formula.
2. Transferred 38.00 Kg roots plant powder into trays and sterilized the material @ 160°C for 2 hours.
3. Unloaded the autoclaved materials into double lined polybags separately and kept in airtight containers.
4. Sent the sample for LOD, BD and Microbiological Analysis.
5. If required Autoclaved herbal material at 121°C for 40 min.
6. Sifted 38.00 kg of herbal root powder through # 80 sieve.
7. Sifted 28.00 kg of water extract through # 40 sieve.
8. Collected the above-sifted materials in separate duly labeled double lined polybags.
9. Recorded Quantity sifted and the sieve integrity before and after sifting.

Granulation

1. Charged 38.00 Kg of root powder and 1.0 Kg of roots $CO_2$ extract into the RMG mix for about 5 minute.
2. Added 28.00 Kg of water extract and mixed for another 5min.
3. Added Purified water to the RMG containing the root powder, roots $CO_2$ extract and water extract and mixed over a period of about 3 minute, with a medium speed.
4. Stopped the mixer and scraped off the mass from the sides and bottom.
5. Continued mixing by operating the impeller at high speed with Chopper ON for about 2 minute.
6. Added additional quantity of Purified water, if required.
7. Discharged the mass from the RMG.

Wet milling

1. Milled the Wet Mass obtained in Multi mill fitted with 8mm screen.

Drying

1. Dried the Wet mass obtained in Tray Drier/FBD at about 55°C to 65°C for about 60 minutes.
2. Checked the Moisture once every 30 minutes (LOD Limit: 3.0 to 4.0%w/w) and recorded the details.

Sizing

1. Sifted the dried granules using a Sifter fitted with #16 sieve.

2. Collected the sifted granules in a clean double poly-lined HDPE container.

3. Milled the retains (oversize granules) obtained through a Multi Mill fitted with 1.5mm, screen with 'Knives Forward' direction.

4. Passed the milled granules obtained through a Sifter fitted with #16 sieve.

5. Collected the sized granules obtained and added them to the sifted granules obtained in stage 10.3.

6. Blended the above sifted granules for about 3 minutes at 20-25 RPM.

7. Unloaded the blend in a clean double poly-lined HDPE drums and affixed duly filled status labels.

8. Weighed the blend and entered the details.

Table-40

| Parameter | Standard value |
|---|---|
| Loss on drying | 3 .0 - 4.0 % w/w |
| Bulk density | 0.45 - 0.65 g/ml |
| Granules to fine ratio | 60:40 - 90:10 |
| Actives | As per Finished Product spec |
| TVAC | NMT 10000 cfu/gm |
| Fungal count | NMT 100 cfu/gm |

Compression

Adjust the machine as below mentioned tooling in the table

Table-41

| | Description |
|---|---|
| Punch Size | 15x7mm Caplet |
| Upper Punch | Plain |
| Lower Punch | Plain |

1. Carried out the initial checks before starting the operation as specified in standard parameters.

2. Checked for Appearance, Average Weight, Individual weight, Thickness, Hardness, Friability & DT of 6 tablets, checked Appearance, Thickness & Hardness of tablets every 30 min, Checked Friability and DT every 1-hour.

3. Checked for the Average weight of 20 tablets every 30 mins graphically.

4. Collected the tablets in a double poly-lined, tightly closed, container. Weighed each container and entered the details.

5. Collected the Compressed tablets in Double poly-lined HDPE drums and recorded their weights.

Example-45

Finished product specification of *Withania somnifera* per caplet

Table-42

| STANDARD PARAMETERS | |
|---|---|
| 1 .Theoretical average | 670 mg |
| 2. Weight uniformity | 670 mg $\pm$ 5% (636.5 to 703.5 mg) |
| 3. Weight of 20 tablets | 13.40 g $\pm$ 5% (12.73 to 14.07 gm) |

(continued)

| STANDARD PARAMETERS | |
|---|---|
| 1 .Theoretical average | 670 mg |
| 4. Tablet thickness | 5.8 to 6.8 mm |
| 5. Tablet hardness | 2 to 6 Kg/cm$^2$ |
| 6. Friability | NMT 1.0% W/W |
| 7. Disintegration time | NMT 30 min. |
| 8. Total withanolide content | 2.96 mg - 3.9 1 mg |

Example-46

Estimation of amino acids of water extracts of *Withania somnifera* roots by amino acid analyzer .

The samples were analyzed by test method "J. AOAC (Journal of Association of Official Agricultural Chemists), 70, 241-247, 1987". The results are provided in below table;

Table-43

| S. No | Amino Acids Composition | % |
|---|---|---|
| 1 | Aspargine | 0.39 |
| 2 | Glutamine | 1.49 |
| 3 | Hydroxy proline | 0.29 |
| 4 | Serine | 0.21 |
| 5 | Glycine | 0.45 |
| 6 | Histidine | 0.09 |
| 7 | Arginine | 0.02 |
| 8 | Threonine | 0.51 |
| 9 | Alanine | 0.23 |
| 10 | Proline | 0.59 |
| 11 | Tyrosine | 0.03 |
| 12 | Valine | 0.22 |
| 13 | Methionine | 0.63 |
| 14 | Isoleucine | 0.13 |
| 15 | Leucine | 0.17 |
| 16 | Phenyl alanine | 0.11 |
| 17 | Lysine | 0.03 |

Example-47

Estimation of total withanolides in *Withania somnifera.*

Reference method for analysis: Standardisation of botanicals (Testing and extraction methods of medicinal herbs) - By Dr.V.Rajpal, Volume 1, page- 256. Eastern publishers, New Delhi, year 2002.
Withanolides can be extracted by methanol and the ether soluble portion of methanolic extract is taken. The ether soluble portion contains total withanolides, which is dried and weighed.
Estimation procedure for dry extracts and granules:

1. Weigh about 5 g (W) of finely powdered *Withania somnifera* dry extract/granules in 100 ml or 250 ml beaker.

2. Measure 25 ml of methanol and 25 ml of water in a beaker and add to it.

3. Stir for 15 minutes using a magnetic stirrer at room temperature. Filter through ordinary filter paper without loosing the residue. Extract the residue in the similar manner with 3 x 50 ml of methanol: water (1:1) mixture and filter the aqueous methanol extract.

4. Combine the aqueous methanol extracts and transfer to a 500 ml separating funnel.

5. De-fat the extract by gentle shaking with 100 ml of hexane. Discard the hexane layer and repeat the de-fatting two more times with 100 ml each of hexane and discard the hexane layers.

6. Fractionate the remaining aqueous methanolic extract by gentle shaking for 2 minutes with 100 ml of Diethyl ether.

7. Repeat the fractionation with 2 x 100 ml of diethyl ether using the separating funnel.

8. Combine the ether extracts, wash with 2 x 50 ml of water using separating funnel. Separate the water layer and discard.

9. Dry this ether fraction over a pre-weighed ($W_1$) china dish on an evaporation water bath maintained at about 60°C. Keep the dried china dish in oven for 30 minutes at 105°C, Cool and weigh the residue in china dish ($W_2$).

10. The residue represents the totalwithanolides, which can be expressed as %w/w as the original material by calculating total withanolides using formula.

Calculation for Withanolides:

$$\% \text{ w/w of withanolides} \quad = \quad \frac{\text{Weight of the residue } (W_2 - W_1) \text{ in g}}{\text{Weight of the sample taken } (W) \text{ in g}} \quad \text{x} \quad 100$$

Example -48

HPLC analysis method and fingerprint of supercritical fluid ($CO_2$) extract of *Withania somnifera* (Figure 1)

LC-MS/MS (Applied Biosystems , API-2000) method of analysis for *Withania* $CO_2$ extract.

Conditions;

Column: RP C18 (250 * 4.6mm, 5um)
Flow rate: 0.5ml/min
Run time: 45min
Wave length: 223nm, 254nm.
Mobile phase: Methanol: Water (60:40)
Volume of injection: 20ul
Sample preparation: (1mg/ml concentration)
Weighed accurately about 25mg of *Withania somnifera* $CO_2$ extract in a 25ml of clean volumetric flask. Added 20ml of methanol (HPLC grade) and dissolved by sonication for 10min. Make up to volume with methanol (HPLC grade). Filtered the final solution through 0.2$\mu$m syringe filtered before injecting 20 $\mu$l to the instrument.

Example-49

Preparation of *Zingiber officinale* water extract

Approximately 100 Kg of shade dried plant material was subjected to extraction with 400 Liters of purified water by percolation method at Room Temperature. The water extractions after 24 -48 hours were filtered through muslin cloth and concentrated to thick paste. After achieving the desired total solid content, the soft extract was spray dried to a free flowing dry extract powder. The water extract was also prepared by hot soxhlation method.

Example-50

Preparation of *Zingiber officinale* SCFE (CO$_2$) Extract

Approximately 25 Kg of the rhizomes of *Zingiber officinale* was pulverized to fine powder and loaded in to extractor. Super Critical Carbon dioxide liquid was pumped in to the extractor at a pressure of 300 bar and 39°C temperature for 2-3 hours. Extract was separated into the container at pressure of 40 bar and 20°C temperature. The CO$_2$ super critical liquid was recycled from the extraction vessel. The resultant extract was analyzed for active markers of *Zingiber officinale.*

Example-51

Preparation of Organic *Zingiber officinale* Granules (Formula-1)

Table-44

| S. No | Name of the Material | Weight per Tablet in mg | Weight per Batch In kg |
|---|---|---|---|
| 1 | *Zingiber officinale* (water extract) (#40 mesh) | 233.00 | 23.30 |
| 2 | *Zingiber officinale* (supercritical fluid extract, CO2) | 30.00 | 3.00 |
| 3 | *Zingiber officinale* aerial parts (# 60 mesh) | 145.00 | 14.50 |
| 4 | *Zingiber officinale* rhizome (# 60 mesh) | 335.00 | 33.50 |

Example-52

Formula of Lubrication of granules

Table-45

| S. No. | Name of the material | Weight per Tablet in mg | Weight per Batch in Kg |
|---|---|---|---|
| 1 | *Zingiber officinale* Extract Granules (#16 mesh) | 743 | 74.30 |
| 2 | *Zingiber officinale* aerial parts (#60) powder | 39.00 | 3.90 |
| 3 | *Zingiber officinale* rhizome (#60) powder | 38.00 | 3.80 |
| | Total | 820.00 | 82.00 |

Example-53

DISPENSING OF RAW MATERIAL

    1. Dispense the raw materials as per Batch record

DRY HEAT STERILIZATION

1. Transfer 14.5 kg and 3.9 kg of *Zingiber officinale* aerial parts and 33.5 and 3.80 kg of *Zingiber officinale* rhizome powder into trays and sterilize the material @ 160 °C for 60 mins.
2. Unload the sterilized material in to double lined polybags separately and keep in airtight containers.
3. Check the sample for LOD, BD and Microbiological Analysis as per Table-46

Table-46

| S.No | Parameter | Standard values |
|---|---|---|
| 1 | LOD, *Zingiber officinale* aerial parts powder | 2.0 - 4.0 % w/w |
| | LOD, *Zingiber officinale* rhizome parts powder | 2.0 - 4.0 %w/w |
| 2 | BD, *Zingiber officinale* aerial parts powder | 0.2 - 0.3 g/ml |
| | BD, *Zingiber officinale* rhizome parts powder | 0.3 - 0.4 g/ml |
| 3 | TVAC | NMT 5000 cfu/gm/ml |
| 4 | Fungal count | NMT 10 cfu/ gm/ml |

SIFTING

1. Sift 23.30 kg of water extract of *Zingiber officinale* rhizome through # 40
2. Sift 14.5 and 33.5 kg of *Zingiber officinale* aerial parts powder and *Zingiber officinale* rhizome powder through # 60
3. Sift 3.9 and 3.8 kg of *Zingiber officinale* aerial parts powder and *Zingiber officinale* rhizome powder through # 60 separately
4. Collect the above-sifted materials in separate duly labeled double lined polybags.
5. Record Quantity Sifted and the sieve integrity before and after sifting

PREPARATION OF GRANULATION FLUID

1. Transfer about 10 kg of Purified water into a clean Stainless Steel Vessel.
Record the observations. Record deviations if any.

GRANULATION

1. Charge 23.30kg of water extract of *Zingiber officinale* rhizome and 14.5 kg and 33.5 kg of *Zingiber officinale* aerial parts powder and *Zingiber officinale* rhizome powder into the RMG, mix for about 5 minute.
2. Slowly add 3.0kg of supercritical extract of *Zingiber officinale* rhizome and granulation fluid to the RMG containing the sifted water extract of *Zingiber officinale* rhizome and powders of aerial parts of *Zingiber officinale* and powders of rhizome over a period of about 3 minute, with medium speed.
3. Stop the mixer and scrape off the mass from the sides and bottom.
4. Continue mixing by operating the impeller at high speed with Chopper ON for about 3 minute.
5. Add additional quantity of granulation fluid, if required.
6. Discharge the mass from the RMG.
7. Mill the Wet Mass obtained in Multi mill fitted with 8mm screen.

TRAY DRYING

1. Dry the Wet mass obtained in tray Drier at about 60°C to 70°C for about 60 minutes.
2. Check the Moisture once every 30 minutes.( LOD Limit: 2 to 4%w/w) and record the details

SIZING

1. Sift the dried granules using a Sifter fitted with sieve #16.
2. Collect the sifted granules in a clean double polylined HDPE container.
3. Mill the retains (oversize granules) obtained through a Multi Mill fitted with 1.5 mm screen with 'Knives Forward' direction

4. Pass the milled granules obtained through a Sifter fitted with #16 sieve. Collect the sized granules
5. Weigh the granules. And analysed as per standard value. Table-47

Table-47

|   | Parameter | Standard values |
|---|---|---|
| 1 | LOD | 2.0 - 4.0 % w/w |
| 2 | BD | 0.40 - 0.60g/ml |
| 4 | Actives | As per Finished Product spec |
| 5 | TVAC | NMT 5000 cfu/gm |
| 6 | Fungal count | NMT 10 cfu/gm |

BLENDING

1. Transfer the sized granules into the blending area.
2. Transfer the sifted herb powder lubricant into the blending area.
3. Load 3.9kg and 3.8 kg of *Zingiber officinale* aerial powder and *Zingiber officinale* rhizome powder and the sized granules into the Double Cone blender.
4. Blend the ingredients for 6 minutes at 10-11 RPM.
5. Unload the blend in a clean Double poly-lined HDPE drums and affix duly filled status labels.

COMPRESSION

1. Check the Temperature and Relative Humidity of the area and record.
2. Bring the drums containing the blend into the compression area.
3. Carry out the initial checks before starting the operation as specified in below Table-48

Table-48

|   | Description |
|---|---|
| Punch Size | 18x 9 mm caplet |
| Upper Punch | Plain |
| Lower Punch | Plain |

4. Check for Appearance, Av. Wt., Individual wt., Thickness, Hardness, Friability & DT of 6 tablets
5. Check appearance, thickness & hardness of tablets every 30 min
6. Check Friability and DT every 1-hour
7. Check Average weight of 20 tablets every 30 mins graphically
8. Collect the tablets in a double poly-lined, tightly closed, container. Weigh each container and enter the details.
9. Collect the Compressed tablets in Double polylined HDPE drums and record their weights.

Example-54

Finished product specification of *Zingiber officinale* per caplet. Table-49

Table-49

| Parameters | Standard Range |
|---|---|
| Theoretical Average weight | 820 mg |
| Weight uniformity | 820 mg $\pm$ 5% (779mg to 861mg) |

(continued)

| Parameters | Standard Range |
| --- | --- |
| Weight of 20 tablets | 16.4g $\pm$ 3% (15.90 gm to 16.89 gm) |
| Tablet thickness | 4.0 to 6.0 mm |
| Tablet hardness | 2 to 6 Kg/cm$^2$ |
| Friability | NMT 1.0% W/W |
| Disintegration time | NMT 30 min |
| Total gingerols content | 6.0 mg |

Pack tablets immediately after compression.

PACKING

1. Pack 60 tablets in a HDPE 120 CC container and weigh them for appropriateness of number of tablets.

Example-55

Estimation of Total Gingerols by Hplc Method

HPLC CONDITIONS:

Column Details: C8 (Make: Thermo, Part No.: 30305-254630)
Wave Length: 282nm
Flow rate: 1ml/min
Volumn of Injection: 20 $\mu$l
Mobile Phase: Methanol: water (65: 35)
Run Time: About 45 minutes

Preparation of Standards:

Preparation of standard Gingerols (2 mg/ml): Weigh about 100 mg of reference standard (containing 6-Gingerol, 8-Gingerol, 6-Shagoal and 10-Gingerol) in a 50 ml volumetric flask, and dissolve by sonication in methanol. Make up the volume with methanol.

Preparation of sample:

For powdered raw material/Granules (10 mg/ml): Weigh 500 mg of the finely powdered sample in a 50 ml Volumetric flask, Dissolve with methanol and sonicate it for 5 minutes. Make the volume up to mark with methanol. 1.
For standardized extracts (2 mg/ml): Weigh about 100 mg of the sample in a 50 ml volumetric flask, and dissolve by sonication in methanol. Make the volume up to the mark with methanol.

Chromatographic procedure:

After the stabilization of the instrument with the mobile phase, inject 20 $\mu$l of working standard solution into the column of the HPLC instrument. Record the chromatogram for about 45 minutes. Then inject 20 $\mu$l of the sample solution in the similar manner and record the chromatogram.
Calculation: Calculate the % total Gingerols content in sample by using the following formula in Table-50

Table-50

| Total AUC of the peaks in the sample chromatogram corresponding to peaks in standard | | Concentration of standard (mg/ml) | | Purity of standard |
|---|---|---|---|---|
| -------------------------------------------------------- | X | --------------------------------------------- | X | |
| AUC of the peaks in the standard chromatogram | | Concentration of sample (mg/ml) | | |

Example-56

Liquid Chromatography -Mass Spectrometer Analysis of *Zingiber officinale* supercritical ( CO2 extract and water extract:

LCMSMS analysis were carried out by using an applied biosystem-Sciex API 2000 triple quadrupole mass spectrometer equipped with an atmospheric pressure chemical ionization source and heated nebulizer APCI interface. The liquid chromatography was a LC-20 AD Series binary system equipped with an autosampler. The column used was C18 phenomenex (250 X 4.6mm, 5$\mu$m), flow rate 1ml/min of mobile phase methanol:water (65:35), wave length 282 nm and run time 40 min. The analytes were ionized by APCI in positive-ion mode (PI mode). Final ionization conditions were heated nebulizer temperature 450 °C, curtain gas Nitrogen 30 psi, particulate-free and C02-free air was used as nebulising gas at a flow rate of 70 psi.

Sample preparation for Ginger CO2 extract:

Weigh about 50mg of sample in a 50ml volumetric flask, and dissolve by sonication in methanol (HPLC grade). Make the volume up to the mark with methanol filter through 0.2um syringe filter.

Sample preparation for Ginger water extract:

Weigh about 500mg of finely powdered sample in a 50ml volumetric flask, and dissolve by sonication in methanol (HPLC grade). Make the volume up to the mark with methanol filter through 0.2um syringe filter
LCMSMS chromatogram is represented in Figure-12 and 13.

Example-57

Preparation of *Commiphora mukul* water extract

Approximately 100 Kg of dried herbal resin was subjected to extraction with 400 Liters of purified water by percolation method at Room Temperature. The water extractions after 24 - 48 hours were filtered through muslin cloth and concentrated to thick paste. After achieving the desired total solid content, the soft extract was spray dried to a free flowing dry extract powder. The water extract was also prepared by hot soxhlation method.

Example-58

Preparation of *Commiphora mukul* SCFE, super critical fluid extract ($CO_2$) extract

Approximately 25 Kg of resin of *Commiphora mukul* was pulverized to fine powder and loaded in to extractor. Super Critical Carbon dioxide liquid was pumped in to the extractor at a pressure of 300 bar and 39 °C temperature for 2-3 hours. Extract was separated into the container at pressure of 40 bar and 20 °C temperature. The $CO_2$ super critical liquid was recycled from the extraction vessel. The resultant extract was analyzed for active markers of *Commiphora mukul.*

Example-59

Formula of granulation

Table-51

| S. No | Raw Materials | Weight per capsule in mg | Weight per batch in kgs |
|---|---|---|---|
| 1 | *Commiphora mukul* water extract (40 #) | 195.00 | 19.50 |
| 2 | *Commiphora mukul* supercritical fluid extract (SCFE) $CO_2$ Extract | 60.00 | 6.00 |
| 3 | *Commiphora mukul* stem powder (80 #) | 75.00 | 7.50 |
| 4 | Purified water | Quantity sufficient | |

Formula for lubrication

Table-52

| S.No. | Raw materials | Name of material | Weight per capsule in g/caps | Weight per batch in kg |
|---|---|---|---|---|
| 1 | *Commiphora mukul* granules (16 # ) | *Commiphora mukul* granules | 330.00 | 33.00 |
| 2 | *Commiphora mukul* stem powder (80 #) | *Commiphora mukul* stem powder (80 #) | 30.00 | 3.00 |
| 3 | E veg capsules CT/CT "0" | Empty veg capsules | - | 100000 |
| | Fill weight | | 360.00 | 36.00 |

Example-60

Manufacturing process of granules and capsules

DISPENSING OF RAW MATERIAL

    1. Dispense the raw materials as per Batch Formula

DRY HEAT STERILIZATION

1. Transfer 10.5 kg of *Commiphora mukul* stem powder in to trays and sterilize the material @ 160 °C for 60 mins.
2. Unload the sterilized materials in to double lined polybags separately and keep in airtight containers.
3. Send the sample for Loss on drying, Bulk density and Microbiological Analysis. The parameters are mentioned in below table-53

Table-53

| | Parameter | Standard values |
|---|---|---|
| 1 | LOD | 2.5 - 3.5% |
| 2 | BD | 0.15 - 0.25 g/ml |
| 3 | Microbes | NMT 5000 cfu/gm |
| 4 | Fungal count | NMT 10 cfu/gm |

SIFTING

1. Sift *Commiphora mukul* water extract through # 40 and *Commiphora mukul* stem powder through #80, weigh as per the required quantity and kept separately in duly-labeled double lined poly bag.
2. Record Quantity Sifted and the sieve integrity before and after sifting.

PREPARATION OF GRANULATION FLUID

1. Transfer about 15.0 kg of Purified water into a clean Stainless Steel Vessel
2. Take 6.00 liters of purified water in a SS vessel, add 3.5 kg of *Commiphora mukul* stem powder then mix continuously to get a uniform solution.

GRANULATION

1. Charge 16.00 Kg of *Commiphora mukul* water extract, 7.50 kg of *Commiphora mukul* stem powder into the RMG, mix for 5 minute.
2. Add slowly 6 kg of *Commiphora mukul* scfe extract to the above blend. Mix it for 5 minutes.
3. Add step 7.2 to RMG and granulate. If required add additional quantity of purified water, mix over a period of 3 minute, with medium speed.
4. Stop the mixer and scrape off the mass from the sides and bottom.
5. Continue mixing by operating the impeller at high speed with Chopper ON for 3 minute.
6. Add additional quantity of purified water, if required.
7. Discharge the mass from the RMG.
8. Record the observations. Record deviations if any.

WET MILLING

1. Mill the Wet Mass obtained in Multi mill fitted with 8 mm screen.
2. Record the observations.
3. Record deviations if any.

TRAY DRYING

1. Dry the Wet mass obtained in tray Drier at about 60°C for about 60 minutes.
2. Check the Moisture once every 30 minutes. (LOD Limit: 2 - 4%) and record the details

SIZING

1. Sift the dried granules using a Sifter fitted with sieve #16.
2. Collect the sifted granules in a clean double poly - lined HDPE container.
3. Mill the retains (oversize granules) obtained through a Multi Mill fitted with 1.5 mm screen with 'Knives Forward' direction
4. Pass the milled granules obtained through a Sifter fitted with #16 sieve.
5. Sizing of dried granules must be carried out within 2 hours. In case the dried granules are not sized immediately, collect them in double poly-lined HDPE container, duly labeled.
6. Collect the sized granules obtained and add them to the sifted granules

BLENDING

The granules were evaluated for following parameters (Table-54).

Table-54

|   | Parameter | Standard values |
|---|-----------|-----------------|
| 1 | LOD | 2-4% |
| 2 | BD | 0.45 - 0.55 g/ml |
| 3 | Granules to fine ratio | 60:40 to 70:30 |
| 4 | Actives | As per Finished Product spec |
| 5 | Microbes | NMT 5000 cfu/gm |
| 6 | Fungal count | NMT 10 cfu/gm |

1. Maintain the Temperature and Relative Humidity of the area with in the specified limit (Limit Temperature NMT 25 °C and relative humidity NMT 40%)
2. Transfer the sized granules into the blending area.
3. Transfer the sifted *Commiphora mukul* stem powder lubricant into the blending area.
4. Load required quantity of *Commiphora mukul* stem powder and the sized granules into the Double Cone blender.
5. Blend the ingredients for 3 minutes at 20-25 RPM.
6. Record the details
7. Unload the blend in a clean Double poly-lined HDPE drums and affix duly filled status labels.
8. Weigh the blend and enter the details.
9. Record deviations if any.

CAPSULE FILLING

1. Maintain the Temperature and Relative Humidity of the area with in the specified limit.
2. (Limit Temperature NMT 25 °C and relative humidity NMT 40%).
3. Check the Temperature and Relative Humidity of the area and record.
4. Bring the drums containing the blend into the capsule filling area.
5. Adjust the machine as per the parameters mentioned in stage 13.11.
6. Carry out the initial checks before starting the operation.
7. Check for Appearance, Av. Wt., Individual wt., length & DT of 6 capsules.
8. Check for the appearance, length of capsules every 30 min.
9. Check DT every 1-hour.
10. Check Av. weight of 20 capsules every 30 mins graphically.
11. Collect the capsules in a double poly-lined, tightly closed, container. Weigh each container and enter the details.
12. In process specification for capsules (Table-55)

Table-55

| Parameter | Standard limit |
|-----------|----------------|
| Description | Clear Transparent size 0 capsules filled with brown colored granules |
| Weight of empty capsules | 95-100mg |
| Fill weight | 360 mg |
| Average weight | 460± 5% |
| Weight 20 capsules | 9.2 gm ± 3% |
| Average length of 10 capsules | 21.4 ± 0.4 mm |
| DT | NMT 15 min |
| Guggul sterones | NLT 3.75mg/ capsule |

1. Collect the filled capsules in double poly-lined HDPE drums and record their weights.
2. Affix duly filled status labels to the containers.
3. Perform the reconciliation of the filled capsules.

PACKING

1. Pack 60 capsules in a HDPE 120 CC container and weigh them for appropriateness of number of capsules.
2. Put about 15 grams of cotton with minimum pressing and make sure that rattling is minimum.

Example-61

Formula of granulation for *Commiphora mukul* and *Piper longum*

Table-56 Granulation and lubrication formula

| S. No | Raw Materials | Weight per capsule in mg | Weight per batch in kgs |
|-------|---------------|--------------------------|-------------------------|
| 1 | *Commiphora mukul* water extract (40 #) | 180.00 | 0.900 |
| 2 | *Commiphora mukul* supercritical fluid extract (SCFE) $CO_2$ Extract | 60.00 | 0.300 |
| 3 | *Commiphora mukul* stem powder (80 #) | 250.00 | 1.250 |
| 4 | *Piper longum* seed powder (80 #) | 260.00 | 1.300 |
| 5 | Purified water | Quantity sufficient | 2.400 litres |

Example-62

Processing Details

SIFTING

1. Sift *Commiphora mukul* water extract through #40 mesh, *Commiphora mukul* herb powder 80 # and *Piper longum* seed powder #80.

GRANULATION

1. Transfer about 3.0 Ltr of Purified water and heat to 80-90 °C for 30 min into a clean Stainless
2. Steel Vessel. Use this boiled cooled water for the granulation.
3. Charge *Commiphora mukul* (Guggul) water extract, *Commiphora mukul* (Guggul) herb powder, *Piper longum* seed powder into the RMG and dry mix for about 2 minute, and then add *Commiphora mukul* $CO_2$ extract into the RMG, mix for about 3 minute.
4. Slowly add the granulation fluid to the above blend and granulate for about 3 minute. Stop the mixer and scrape off the mass from the sides and bottom.
5. Continue mixing by operating the impeller at high speed with Chopper ON for about 1 minute. Add additional quantity of granulation fluid, if required.
6. Discharge the mass from the RMG.

WET MILLING

1. Mill the wet mass in multi mill fitted with 8 mm screen.

DRYING

1. Dry the Wet mass obtained in fluid bed drier at about 50 °C to 60 °C for about 60 minutes.
2. Check the Moisture. (LOD Limit: 2.0 to 3.0%) and record the details.

SIZING

1. Sift the dried granules using a Vibratory sifter fitted with sieve #16.
2. Collect the sifted granules in a clean double polylined HDPE container.
3. Mill retains the oversized granules through a Multi Mill fitted with 1.5 mm screen.
4. 'Knives Forward' direction Pass the milled granules through a Sifter fitted with #16 sieve.

BLENDING

Blend the granules for about 5 minute at 10-15 RPM in octagonal blender.

COMPRESSION

1. Compress the granules into 17x8 mm plain caplet shaped punch.
2. Carry out the compression and check for the standard parameters (Table-57).

Table-57

| STANDARD PARAMETERS | |
|---|---|
| 1. Average weight | 750 mg |
| 2. Weight uniformity | 750 mg $\pm$5% (712.5 mg to 787.5 mg) |
| 3. Weight of 20 caplets | 15.00 g $\pm$ 3% (14.55 gm to 15.45 gm) |
| 4. Tablet hardness | NLT 4.0 Kg/cm$^2$ |
| 5. Friability | NMT 1.0% W/W |
| 6. Disintegration time | NMT 30 min. |

Example-63

FORMULA - *Commiphora mukul* (Guggul) Tablets (Round) Table-58

Table-58

| S.NO | NAME OF THE INGREDIENT | MG PER DOSAGE | QTY/ BATCH IN KG 10000 caplets |
|---|---|---|---|
| Granulation formula | | | |
| 1 | *Commiphora mukul* water extract #40 | 250.00 | 2.50 |
| 2 | *Commiphora mukul* stem powder #40 | 250.00 | 2.50 |
| 3 | Purified water | Quantity sufficient | 1.0 Ltr |
| 4 | *Commiphora mukul* granules #16 | 500.00 | 5.000 |

Blending Formula Table-59

Table-59

| S.No. | Raw materials | Name of material | Weight per capsule in mg/caps | Weight per batch in kg |
|---|---|---|---|---|
| 1 | *Commiphora mukul* granules (16 #) | *Commiphora mukul* granules | 500.00 | 50.00 |
| 2 | *Commiphora mukul* stem powder (40 #) | *Commiphora mukul* stem powder (40 #) | 100.00 | 10.00 |
| | Fill weight | | 600.00 | 60.00 |

PROCESSING DETAILS:

SIFTING

1. Sift *Commiphora mukul* water extract and *Commiphora mukul* stem powder through #40 mesh.

PREPARATION OF GRANULATION FLUID

1. Transfer about 1.0 Ltr of Purified water and heat to 80-90 °C for 30 min into a clean Stainless
2. Steel Vessel. And use boiled cooled water for the granulation.

GRANULATION

1. Charge Commiphora mukul water extract, Commiphora mukul stem powder in to the Rapid mixture granulator and dry mix for about 2 minutes, Slowly add the granulation fluid to the above blend and granulate for about 5 minutes.
2. Stop the mixer and scrape off the mass from the sides and bottom. Continue mixing by operating the impeller at high speed with Chopper ON for about 1 minute.
3. Add additional quantity of granulation fluid, if required. Discharge the mass from the rapid mixture granulator.

WET MILLING

1. Mill the wet mass in multi mill fitted with 8 mm screen.

DRYING

1. Dry the Wet mass obtained in fluidized bed dryer FBD at about 50°C to 60°C for about 60 minutes.
2. Check the Moisture. (LOD Limit: 2.0 to 3.0%) and enter the details.

SIZING

1. Sift the dried granules using a Vibratory sifter fitted with sieve #16.
2. Collect the sifted granules in a clean double polylined HDPE container.
3. Mill the retains the oversized granules through a Multi Mill fitted with 1.5 mm screen in 'Knives Forward' direction Pass the milled granules through a Sifter fitted with #16 sieve.

BLENDING

1. Blend granules with *Commiphora mukul* stem powder for about 5 minute at 10-15 RPM in octagonal blender OGB.

COMPRESSION

1. Compress the granules in to 12.6 mm 40 through round shaped punch.
2. Carry out the compression and check for the standard parameters, (Table -60)

Table- 60

| STANDARD PARAMETERS | |
|---|---|
| 1.Average weight | 600mg |
| 2.Weight uniformity | 600 mg $\pm$5% (570.0 mg to 630.0mg) |
| 3.Weight of 20 caplets | 12.00 g $\pm$ 3% (11.64 gm to 12.36 gm) |
| 4.Tablet hardness | NLT 4.0 Kg/cm$^2$ |
| 5.Friability | NMT 1.0% W/W |
| 6.Disintegration time | NMT 30 min. |

Example-64

1. Estimation of guggul sterones by HPLC Standard preparation (1 mg/ml): Weigh accurately abot 50 mg of standard Guggul sterone in a 50 ml volumetric flask. Add 40 ml of acetonitrile and dissolve by sonication. Make the volume up to the mark with acetonitrile.

2. Sample preparation: Weigh accurately about 50 mg of sample in a 50 ml volumetric flask. Add 40 ml of acetonitrile and dissolve by sonication. Make the volume up to the mark with acetonitrile.

HPLC Conditions are as follows.

Column: $C_{18}$ hypersil ODS column with dimension 250 x 4.6 mm, particle size - 5$\mu$
Solvent system: Acetonitrile: Water (60: 40)
Flow rate: 1 ml/min
Detection: 241 nm
Injection volume: 20 $\mu$l
Run time: Approximately 30 minutes
Approximate retention time: Guggul sterone E-10 minutes, Guggul sterone Z - 13 minutes.
Chromatographic procedure: Inject 20 $\mu$l of standard to the HPLC injector and record the chromatogram. Two major peaks obtained in the chromatogram correspond to Guggul sterone E and Z isomers. In the similar manner inject 20 $\mu$l of sample and record the chromatogram. Add the AUC of both the major peaks (Isomers E and Z) for the purpose of calculation.
Calculation: Table-61

Table-61

$$\% \text{ Guggul sterone} = \frac{\text{AUC of sample}}{\text{AUC of standard}} \times \frac{\text{Concentration of standard}}{\text{Concentration of sample}} \times \% \text{ Purity of standard}$$

Example-65

Details on amino acid composition in water extract of *Commiphora mukul.* Analysis method: Journal of Association of Official Agricultural Chemists, 70, 241-247, 1987. Results are summarized in Table-62.

Table-62

| S.No. | Amino Acid | % in water extract |
|---|---|---|
| 1 | Asparagine | 1.69 |
| 2 | Glutamine | 1.69 |
| 3 | Serine | 1.56 |
| 4 | Glycine | 1.28 |
| 5 | Histidine | 0.33 |
| 6 | Arginine | 0.52 |
| 7 | Threonine | 1.16 |
| 8 | Alanine | 1.45 |
| 9 | Proline | 1.24 |
| 10 | Tyrosine | 1.16 |
| 11 | Valine | 1.16 |
| 12 | Methionine (estimated as methionine sulfone) | 0.24 |
| 13 | Cystine (estimated as cysteic acid) | 0.52 |
| 14 | Isoleucine | 1.39 |
| 15 | Leucine | 2.25 |
| 16 | Phenylalanine | 1.07 |
| 17 | Lysine | 0.93 |

Example-66

LCMSMS Analysis of *Commiphora mukul* extract (Figure 1)
LCMSMS method of analysis for *Commiphora mukul,* super critical fluid extract, $CO_2$ extract.
Instruments details: LC-MS/MS (Applied Biosystems, API-2000)
Conditions:
Column: RP C18 (250 * 4.6 mm, 5 um)
Flow rate: 0.5 ml/min
Run time: 60 min
Wave length: 241 nm, 254 nm.
Mobile phase: Acetonitrile: Water (60:40)
Volume of injection: 20 ul

Sample preparation: (1mg/ml)

Weigh accurately about 25mg of *Commiphora mukul* $CO_2$ extract in a 25 ml of clean volumetric flask. Add 20 ml of Acetonitrile (HPLC grade) and dissolve by sonication for 10 min. Make up to volume with Acetonitrile (HPLC grade). Filter the final solution through 0.2 $\mu$m syringe filter before injecting 20 $\mu$l to the instrument.

Example-67

Preparation of *Allium sativum* (Garlic) Water Extract

Approximately 100 Kg of shade dried Organic garlic bulbs were coarsely powdered and subjected to extraction with 400 Liters of purified water by percolation method at room temperature for 24 hours. The water extractions thus obtained were filtered through muslin cloth and concentrated to thick paste. After achieving the desired total solid content, the soft extract was spray dried to a free flowing dry extract powder. The dried extract was passed through #40 mesh stainless steel sieves

Example-68

Preparation of *Allium sativum* (Garlic) powder by Freeze Drying

The fresh bulbs of Organic Garlic were depoded with its outer skin and subjected to cleaning with fresh water. The cleaned material was chopped randomly in mechanized cutter. The material was transferred into stainless steel trays in freeze dryer. The material is frozen to - 25°C for 4-5 hrs. The frozen material was loaded in to the chamber for drying under vacuum at 0.65 to 0.80 mBar and temperature from -25°C to + 30°C. The dried material was unloaded and checked for moisture content. The dried material was subjected to hand crushing and passed through #16 mesh size stainless steel sieves.

Example-69

Preparation of *Allium sativum* (Garlic) Aerial parts powder by pulverization

The fresh aerial parts material of Organic Allium sativum (Garlic) was cleaned with purified water and shade dried. The shade dried materials was chopped into small pieces and then pulverized to coarse powder. The coarse powder was again pulverized and passed through #80 mesh stainless steel sieves to obtain fine garlic aerial parts powder.

Example-70

Preparation of Organic Garlic Granules (Formula-1)

Table-63

| S. No. | Name of the Material | Weight per Tablet in mg | Weight per Batch In kg |
|--------|----------------------|-------------------------|------------------------|
| 1 | *Allium sativum* (water extract) (#40 mesh) | 170.00 | 17.00 |
| 2 | *Allium sativum* aerial parts (# 80mesh) | 150.00 | 15.00 |

Example-71 Preparation of Organic Garlic granules lubrication (Formula-2)

Table-64

| S.No. | Name of material | Weight per Tablet in mg | Weight per Batch in Kg |
|-------|------------------|-------------------------|------------------------|
| 1 | *Allium sativum* freeze dried material (#16 mesh) | 320.00 | 32.00 |
| 2 | *Allium sativum* granules (#20 mesh) | 325.00 | 32.50 |
| 3 | *Allium sativum* aerial parts (# 80 mesh) | 55.00 | 5.50 |
| | Total | 700.00 | 70.00 |

Example-72

Procedure for sterilization of Organic Garlic Aerial parts

1. Transfer weighed quantity of *Allium sativum* aerial parts in trays and sterilize the materials @ 160°C for 2 hour
2. Unload the sterilized materials in to double- lined poly bags separately and keep in air tight containers.
3. Samples were analysed for LOD, BD and microbial analysis as per Table-3

Table-65

|   | Parameter | Standard values |
|---|-----------|-----------------|
| 1 | LOD | 1.0 - 4.0 % |
| 2 | BD | 0.25 - 0.5 g/ml |
| 3 | TVAC | NMT 5000 cfu/gm |
| 4 | Fungal count | NMT 10 cfu/gm |

Example-73

Procedure for preparation of Organic Garlic granules

1. Transfer about 15.0 kg of Purified water into a clean Stainless Steel Vessel for preparation of granulation fluid
2. Charged 17.0 Kg of *Allium sativum* water extract, 15.0 kg of *Allium sativum* aerial parts into the RMG, mix for 5 minute for granulation
3. Slowly add the granulation fluid to the RMG containing the Sifted *Allium sativum* water extract, *Allium sativum* aerial parts.
4. Top the mixer and scrape off the mass from the sides and bottom.
5. Continue mixing by operating the impeller at high speed with Chopper ON for about 3 minutes.
6. Add additional quantity of purified water, if required.
7. Discharge the mass from the RMG.
8. Mill the Wet Mass obtained in Multi mill fitted with 8mm screen.
9. Dry the Wet mass obtained in tray Drier at about 70°C to 80°C for about 60 minutes.
10. Sift the dried granules using a Sifter fitted with sieve #16.
11. Analysis of granules as per the specification mentioned in Table-66

Table-66

|   | Parameter | Standard value |
|---|-----------|----------------|
| 1 | LOD | 2.0 - 6.0 % |
| 2 | BD | 0.35 - 0.70 g/ml |
| 4 | TVAC | NMT 5000 cfu/gm |
| 5 | Fungal count | NMT 10 cfu/gm |

Eample-74

Procedure for lubrication and compression of Organic Garlic granules

1. Transfer the sifted Allium sativum granules into the blending area
2. Transfer the sifted Allium sativum aerial parts lubricant
3. Transfer the sifted Allium sativum freeze dried material (#16-20 mesh)

4. Load 5.5 kg of Allium sativum aerial parts lubricant, 32.50 Kg of the # 20 mesh sized garlic granules and 32 Kg of Organic Freeze dried garlic material into the double cone blender

5. Blend the ingredients for about 6 minutes at 10-11 RPM.

6. Check the Temperature and Relative humidity of the area and record for compression of tablets

7. Transfer the granulated blend material into the compression area. Punch size- 17x8 mm caplet, upper and lower punch plain.

8. The punched caplets would comply the following finished product specifications.

.Example-75

Finished product specifications of Organic Garlic Caplets

| 1. Theoretical average weight | : | 700 mg |
|---|---|---|
| 2. Weight uniformity | : | 700 mg $\pm$ 5% (665 mg to 735 mg) |
| 3. Weight of 20 tablets | : | 14.00 g $\pm$ 3% (13.58 gm to 14.42 gm) |
| 4. Tablet thickness | : | 3.5 to 6.5 mm |
| 5. Tablet hardness | : | 2.0 to 8.0 Kg/cm$^2$ |
| 6. Friability | : | NMT 1.0% W/W |
| 7. Disintegration time | : | NMT 30 min |
| 8. Allicin (By HPLC) | : | NLT 1600 $\mu$g/Caplet |

Example-76

Analytical procedure for estimation of Allicin in Garlic cloves/Freeze dried materials/caplets

**Introduction:** The method is carried out as per BP using butyl parahydroxybenzoate R as the internal standard. Carry out the assay as per timelines mentioned in the procedure.

**Internal standard solution (0.02g):** Weigh accurately 20 mg (0.02 g) of butyl parahydroxybenzoate R in 100 ml volumetric flask, dissolve with methanol: water (1: 1) mixture by sonication and make the volume up to the mark with methanol: water (1: 1) mixture.

**Sample preparation:** Weigh accurately 0.8 g of the powdered garlic powder into 100 ml beaker and add 20 ml of water and homogenize the mixture in an ultrasonic bath at 4°C (using ice cubes) for 5 minutes (use timer). Allow to stand to room temperature for 30 minutes (use timer). Transfer homogenized mixture in 20 ml centrifuge tube and centrifuge for 30 minutes (use timer) at 2500 rpm.

**Preparation of dilution mixture:** 1% v/v solution of anhydrous formic acid: methanol (40: 60).

**Stock solution:** Dilute 10 ml of the supernatant to 25 ml with a dilution mixture.

Keep the sample in refrigerator. Remove it before 10 minutes of injection. Transfer the stock solution in 20 ml centrifuge tube and centrifuge for 5 minutes (use timer) at 2500 rpm. Place 0.5 ml of the internal standard solution in a 10 ml volumetric flask and make up the volume up to the mark with supernatant (centrifuged sample).

**Chromatographic conditions:**

| Column | : | C$_{18}$ stainless steel column with 0.25 m long and 4 mm in internal diameter packed with silanised octadecylsilyl silica gel (5 $\mu$m) for chromatography |
|---|---|---|
| Wavelength | : | 254 nm |
| Flow rate | : | 0.8 ml/min |
| Volume of injection | : | 20 $\mu$l |
| Mobile phase | : | 1%v/v solution of Anhydrous formic acid: Methanol (40: 60). |
| Run time | : | 30 minutes |

**Procedure:** Inject 20 $\mu$l of the internal standard solution and record the chromatogram to identify internal standard peak. Generally the internal standard peak is obtained between 14 and 18 minutes.

Inject 20 $\mu$l of the sample solution and record the chromatogram. The peak corresponding to relative retention time of 0.33 to 0.38 with respect to internal standard in same chromatogram is considered as an allicin peak (the peak is observed approximately about 5 - 7 minutes as shown in chromatogram). Calculate the content of allicin as per the following formula.

**Calculation:**

$$\% \text{ of allicin} = \frac{S_1 \text{ x } m_2 \text{ x } 22.75}{S_2 \text{ x } m_1}$$

$S_1$ = Area of the peak corresponding to allicin (RRT 0.33 to 0.38).
$S_2$ = Area of the peak corresponding to butyl parahydroxybenzoate peak obtained with sample solution in same chromatogram.
$m_1$ = Mass of the sample taken in grams.
$m_2$ = Mass of butyl parahydroxybenzoate in grams in 100 ml of the internal standard solution.

Example-77

Estimation of Amino acids of Garlic Water Extract by Amino acid analyzer

Table-67

| S.No. | Name of the Amino acid | Qty of A.acid (%) |
|---|---|---|
| 1 | Asparagine | 1.64 |
| 2 | Glutamine | 4.18 |
| 3 | Serine | 0.54 |
| 4 | Hydroxyproline | 0.073 |
| 5 | Glycine | 0.6255 |
| 6 | Histidine | 0.199 |
| 7 | Argenine | 1.067 |
| 8 | Threonine | 0.43 |
| 9 | Alanine | 1.065 |
| 10 | Proline | 0.426 |
| 11 | Tyrosine | 0.280 |
| 12 | Valine | 0.634 |
| 13 | Methionine | 0.324 |
| 14 | Cysteic acid | 1.027 |
| 15 | Isoleucine | 0.33 |
| 16 | Leucine | 0.76 |
| 17 | Phenylalanine | 0.47 |
| 18 | Lysine | 0.3075 |

Conclusion

It is evident from the above examples that the preparation of herbal solid formulation having the required content of Allicin and other Pharmaceutical properties of tablets is obtained by specialized process comprising the granulation of garlic water extract and autoclaved aerial parts powder and then the granulated mixture was lubricated with freeze dried garlic and autoclaved plant powder to compress into tablets. By this unique process, we can avoid the degradation of Allicin during granulation and compression procedure and also could be able to achieve the required parameters of disintegration, friability and hardness etc. The final tablets obtained by this procedure contain the active content Allicin at not less than 1600 micro gram per tablet along with blend of 16 aminoacids.

Example-78

Clinical trial of Andrographis paniculata in human beings

A Double Blind Placebo Controlled Clinical Trial of *Andrographis* on common cold

Objectives of the study: To evaluate the efficacy and safety of Andrographis in common cold. The clinical study was carried out at Department of ENT, SCB Medical College, Cuttack
Study design: The present study was a double blind placebo controlled clinical trial. Primary endpoints: The predefined primary endpoints were the symptomatic relief from common cold.
Secondary endpoints: The predefined secondary endpoints were incidence of adverse effects and overall compliance to the study drug under investigation.
Inclusion criteria:

- Patients suffering from common cold.
- Patients of both sex aged between 18 to 60 years
- Willing to sign informed consent form

Exclusion criteria:

- Pregnant patients
- Patients who are in immunocompromised state
- Patients having the symptoms of common cold from long duration and taking any other medication.
- Unwilling to sign informed consent form.

Methodology

Fifty patients suffering from common cold aged between 18 to 60 years were included in the trial. Group A patients received Andrographis Caplets and Group B received placebo at a dose of one Caplet twice daily for a period of 1 week. The dose remained constant throughout the study. No other medication was allowed throughout the study. Efficacy of the drug was analyzed based on the comparison between the baseline score and the score after the treatment. The patients were followed upto 1 week. After a week patients were analyzed for the symptomatic relief from common cold. The score was given as follows:

Excellent - > 75% reduction in common cold
Good -50-74% reduction in common cold
Moderate - 25-49% reduction in common cold
Mild - < 25% reduction in common cold
Poor - No reduction in common cold

Statistical analysis:

The values are expressed as Mean $\pm$ SD. Statistical analysis was carried out using Fisher's Exact Test using GraphPad Prism, Version 4.03 for windows, Graphpad Software, San Diego, California, USA, for presence or absence of various signs and symptoms. Repeated measures of ANOVA followed by Dunnett's Multiple Comparison Posthoc Test were used for the analysis clinical parameters.

Results:

This study included 50 patients. Patients were aged between 18-60 years. The demographic details of both the groups Andrographis Caplet and placebo namely are listed in table 68.

Table 68

| Demographic data of patients on entry (n=50) | | |
|---|---|---|
| Parameter | Andrographis Caplet (n=25) | Placebo(n=25) |
| Age in years (mean ± SD) | 27.38 ± 05.06 | 27.72 ± 07.56 |
| Weight in Kg (mean ± SD) | 64.10 ± 12.35 | 56.60 ± 18.70 |
| Sex ratio/ M:F | 12:13 | 10:15 |
| H/o smoking (No. of cases) | 6 | 4 |
| H/o alcohol consumption (No. of cases) | 2 | 2 |
| Diet (veg/mixed) | 12/13 | 15/10 |

The result of subjects treated with Andrographis Caplet is as follows. Most patients started responding to the therapy at the end of 2 days of treatment. At 4th day follow up, 8 patients showed moderate response (25-49% reduction), 5 patients showed good response (50-74% reduction). At the end of 6 days treatment, 4 patients showed excellent response, 8 patients showed a good response, 7 patients showed moderate response, 4 patients showed mild response and 2 patients showed poor response.

In patients treated with placebo there were no significant improvement in symptoms of common cold

Table 69

| Common cold score observed at the interval of two days (n=50) | | | | | | |
|---|---|---|---|---|---|---|
| | Number of cases (n) | | | | | |
| Common Cold Scores | Andrographis Caplet (n=25) | | | Placebo(n=25) | | |
| | Day 2 | Day 4 | Day 6 | Day 2 | Day 4 | Day 6 |
| Poor response (No reduction in common cold score) | 9 | 6 | 2 | 15 | 10 | 9 |
| Mild response (< 25% reduction in common cold score) | 10 | 6 | 4 | 5 | 9 | 8 |
| Moderate response (25-49% reduction in common cold score) | 6 | 8 | 7 | 5 | 6 | 5 |
| Good response (50-74% reduction in common cold score) | - | 5 | 8 | - | - | 3 |
| Excellent response (> 75% reduction in common cold score) | - | - | 4 | - | - | - |

<u>Discussion and conclusion:</u> Ayurveda documents several plants, including *Andrographis,* which are, categorized as rasayana. The properties ascribed to rasayanas in Ayurveda are remarkably similar to those of adaptogens. In this study, *Andrographis* caplets showed overall improvement in common cold. The results of the study indicate that administration of *Andrographis* significantly decreases the occurrence of common cold. Furthermore, *Andrographis* shortens the mean duration of colds and reduces the severity of cold symptoms without any side effects. In this study it was found that the herbal formulation reduced common cold and showed significant improvement with Andrographis treatment. There were no serious adverse effects either observed or reported. Therefore, it may be concluded that Andrographis is effective and safe in the treatment of common cold.

Example-79

Clinical trial of *Terminalia arjuna* Caplets in human being

A randomized, double blind, placebo controlled study on the effects of Arjuna caplets in mild hypertension

To evaluate safety and efficacy of Arjuna tablets in humans, a clinical study was carried out in IPGE&R (Ayu) & SVSP Hospital, Kolkata during March 2010 - December 2011.

Objective of the Study: The present study was conducted to evaluate the effect of Arjuna caplets in hypertension
Study design: Randomized, double blind, placebo controlled clinical study
Primary end points: The predefined primary endpoints were reduction in systolic and diastolic blood pressure
Secondary end points: The predefined secondary endpoints were incidence of adverse effects and overall compliance to the drug under investigation
Inclusion criteria:

- Subjects with newly diagnosed mild hypertension 140-160 mm/Hg (Systolic blood pressure) and 90-100 mm/Hg (Diastolic blood pressure)
- Age group ranging from 35-60 years
- Willing to sign informed consent

Exclusion criteria:

- Patients with cardiac decompensation, recent cerebrovascular accident and severely symptomatic ones
- Not willing to sign informed consent form

Methodology:

A total of 50 subjects diagnosed with hypertension 140-160 mm/Hg (Systolic blood pressure) and 90-100 mm/Hg (Diastolic blood pressure) were included in the trial. All subjects were in the age group between 35-60 years. Patients with cardiac decompensation, recent cerebrovascular accident and severely symptomatic ones were excluded from the study.

Randomization was performed by random number generation and the group assignments were made into respective treatment and placebo groups. The primary endpoint of this study was effects of 6 weeks of Arjuna administration on hypertension.

Subjects were randomly assigned to either Arjuna (Group 1) or the placebo (Group 2). Subjects were administered 1 Arjuna caplet daily for 12 weeks. Systolic and diastolic pressure was recorded at each visit. Subjects were asked to comment on acceptability of the regimen and side effects during their visits. Compliance to the medication was assessed at each visit throughout the study.

Statistical analysis:

The results were analysed statistically using Repeated Measures of ANOVA using Friedman Test followed by Dunnet's multiple comparison test. The values were expressed as Mean $\pm$ SD or incidence of occurrence. The differences were considered significant at $p < 0.05$.

Results:

The results indicate that Arjuna brought significant reduction in the blood pressure levels. In Arjuna group, there was gradual reduction in systolic pressure from week 4 compared to the placebo group (Table 71). Also there was reduction in the diastolic pressure noticed in Arjuna compared to the placebo group with a significance of $p < 0.05$.(Table 72). There were no adverse effects either reported or observed during the study period and overall compliance to the study treatment was good.

Table 70

| Showing age and sex distribution in our 50 patients | | |
|---|---|---|
| Age in years | Male | Female |
| 35 - 40 | 10 | 7 |
| 41 - 45 | 8 | 4 |
| 46-50 | 5 | 3 |
| 51 - 55 | 4 | 3 |
| 56-60 | 6 | 0 |
| Total | 33 | 17 |

Table 71

| Showing range of systolic pressure before and after Arjuna caplets | | | | | | | | |
|---|---|---|---|---|---|---|---|---|
| Systolic Pressure in mm Hg | No. of cases before treatment | | 2 weeks after treatment | | 6 weeks after treatment | | 12 weeks after Arjuna | |
| | n=25 | n=25 | n=25 | n=25 | n=25 | n=25 | n=25 | n=25 |
| | Arjuna | Placebo | Arjuna | Placebo | Arjuna | Placebo | Arjuna | Placebo |
| 120 - 130 | 0 | 0 | 8 | 0 | 11* | 1 | 18* | 15 |
| 131 - 140 | 15 | 14 | 10 | 13 | 10* | 12 | 7* | 9 |
| 141 - 150 | 6 | 7 | 4 | 9 | 4 | 9 | 0 | 1 |
| 151 - 160 | 4 | 4 | 3 | 3 | 0 | 3 | 0 | 0 |
| Mean value | 153 $\pm$1.13 | 152 $\pm$11.20 | 142 $\pm$1.77 | 148 $\pm$11.43 | 130 $\pm$ 11.9* | 140.8 $\pm$11.24 | 124.2 $\pm$9.3* | 134.6 $\pm$9.8 |
| p<0.05 * with respect to baseline values | | | | | | | | |

Table 72

| Showing range of diastolic pressure before and after Arjuna caplets | | | | | | | | |
|---|---|---|---|---|---|---|---|---|
| | | | | | | | | |
| Diastolic Pressure in mm Hg | No. of cases before treatment | | 2 weeks after treatment | | 6 weeks after treatment | | 12 weeks after Arjuna | |
| | n=25 | n=25 | n=25 | n=25 | n=25 | n=25 | n=25 | n=25 |
| | Arjuna | Placebo | Arjuna | Placebo | Arjuna | Placebo | Arjuna | Placebo |
| 80-90 | 4 | 0 | 8 | 4 | 15* | 9 | 23* | 15 |
| 91 - 100 | 21 | 25 | 17 | 21 | 10* | 16 | 2* | 10 |
| Mean value | 104 $\pm$8.8 | 100 $\pm$6.4 | 96 $\pm$9.7 | 100 $\pm$2.4 | 90 $\pm$ 6.09* | 96.3 $\pm$2.8 | 84 $\pm$6.41* | 90.4 $\pm$2.2 |
| p<0.05 * with respect to baseline values | | | | | | | | |

Adverse effects:

There were no significant differences in resting heart rates or any other clinical parameters during Arjuna caplets therapy compared to baseline values. No untoward effects were noticed, or reported by study subjects during Arjuna caplets or placebo therapy periods.

Conclusion :

The present study showed that significant reduction in the systolic and diastolic blood pressure was observed in the patients treated with Arjuna caplets. There were no adverse effects, either reported or observed in the clinical study during the study period and the compliance to the study drug was good. There were no drop outs or withdrawal from the study and all the 50 patients completed the study. So, it can be concluded that Arjuna is effective and safe in the management of patients suffering from mild and newly diagnosed hypertension.

Example-80

Clinical trials of Azadirachta indica in Acne treatment

A clinical study to evaluate the efficacy and safety of Neem caplets in Acne Vulgaris: A double blind placebo controlled study.

To evaluate safety and efficacy of neem tablets in humans, a clinical study was carried out in Department of Dermatology, Mandya Institute of Medical Sciences, Mandya during June 2010 - December 2011.

Objectives of the study: To evaluate the safety and efficacy of a pure herb Neem (Azadirachta indica) in patients suffering from acne vulgaris

Study design: Double blind placebo controlled study.

Primary end points: The primary end point was clinical recovery from the signs and symptoms of Acne vulgaris

Secondary end points: Incidence of adverse effects and overall compliance to the drug.

Inclusion criteria:

Adult patients of both sexes suffering from mild to moderate acne vulgaris.

Both male and female patients aged more than 18 years of age.

Patients willing to give written informed consent and comply with the study procedures.

Exclusion criteria:

Patients with below eighteen years of age

patients presenting with severe acne-vulgaris,

pre-existing systemic disease necessitating long-term medications, genetic and endocrinal disorders,

subjects with known history or present condition of allergic response to any cosmetic/herbal products, toiletries or its components or ingredients in the test products Pregnant and lactating women.

Patients not willing to give written informed consent.

Methodology:

Ninety six subjects aged between 18 to 34 years suffering from mild to moderate acne vulgaris and who were willing to give informed written consent were included in the study. After obtaining informed consent baseline history was obtained which include personal data, a description of symptoms and details of past medical history, family history of acne, history of possible exacerbating factor/s, etc. Thereafter all patients underwent a clinical examination and thorough skin examination. The grading of acne vulgaris was as follows:

    Grade I : Mild acne with only papules;
    Grade II : Moderate acne with papules and comedones;
    Grade III : Severe acne with papules and pustules;
    Grade IV : Very severe acne with papules, pustules and cysts.

Patients were randomly treated with either Neem caplet or a similar looking placebo at a dose of 1 caplet once daily for a period of 6 weeks. Patients were instructed not to use any other medication for acne. The response to therapy was evaluated at intervals of two weeks upto the six weeks by calculating the Acne Lesion Scores (ALS)and the efficacy was determined by the percentage reduction in the ALS at the end of six weeks of treatment. Improvement in the form of reduction in the ALS was graded. Grading of ALS was as follows

| Grade | % Reduction in ALS |
| --- | --- |
| 0 | No reduction in ALS. |
| 1 | < 25% reduction in ALS. |
| 2 | 25-49% reduction in ALS. |
| 3 | 50-74% reduction in ALS |
| 4 | > 75% reduction in ALS. |

During each follow-up visit, local skin examination was done and observations were recorded in the case report form. In addition, patients were also evaluated for control of local inflammation (erythema and telangiectesia), new comedone (black and white) formation, relief of pruritus and pain in inflamed acne lesions, soothing and moisturizing effects and healing without hyperpigmentation or scarring were also considered for the overall response to the treatment.

Statistical analysis:

The values are expressed as Mean $\pm$ SD. Statistical analysis was carried out using Fisher's Exact Test using GraphPad Prism, Version 4.03 for windows, Graphpad Software, San Diego, California, USA. for presence or absence of various signs and symptoms. Repeated measures of ANOVA followed by Dunnett's Multiple Comparison Posthoc Test were used for the analysis clinical parameters.

Results:

The demographic details of both the groups neem caplet and placebo namely are listed in table 73. All subjects completed the study as planned.

Table 73

| Demographic data of patients on entry (n=96) | | |
|---|---|---|
| | | |
| Parameter | Neem caplet (n=48) | Placebo(n=48) |
| Age in years (mean $\pm$ SD) | 27.38$\pm$ 08.06 | 24.72$\pm$ 07.56 |
| Weight in Kg (mean $\pm$ SD) | 64.10 $\pm$ 12.35 | 56.60 $\pm$ 18.70 |
| Sex ratio/ M:F | 22:26 | 20: 28 |
| H/o smoking (No. of cases) | 6 | 4 |
| H/o alcohol consumption (No. of cases) | 2 | 2 |
| Diet (veg/mixed) | 24/24 | 28/20 |
| H/o acne-vulgaris (years) | 1.2 $\pm$ 0.9 | 1.44$\pm$0.89 |
| **Skin Type** | Normal to dry skin (No. of patients) 12 | 15 |
| | Dry skin (No. of patients) 4 | 2 |
| | Normal to oily skin (No. of patients) 13 | 14 |
| | Oily skin (No. of patients) 19 | 17 |

The results of subjects treated with Neem caplet is as follows. Most patients started responding to the therapy at the end of 2 weeks of treatment. At 4th week follow up, 16 patients showed moderate response (25-49% reduction in acne lesions), 10 patients showed good response (50-74% reduction). At the end of 6 weeks treatment, 8 patients showed excellent response, 15 patients showed a good response, 14 patients showed moderate response and 04 patients showed poor response.

There was significant reduction in black and white comedones after treatment Significant reduction in black comedones was observed from 10.11 $\pm$ 8.01 at entry to 7.30 $\pm$ 4.85, 4.20 $\pm$ 4.06 and 2.08 $\pm$ 0.16 at the end of 2, 4 and 6 weeks of treatment, respectively with significance of p<0.001. There was also a significant reduction in white comedones from 7.71 $\pm$ 4.04 at entry to 5.21 $\pm$ 3.02, 3.37 $\pm$ 1.01 and 1.27 $\pm$ 0.30 at the end of 2, 4 and 6 weeks of treatment, respectively with significance of p<0.001 (Table 75). Significant reduction in papules was observeved from 16.08 $\pm$ 7.40 at entry to 12.60 $\pm$ 7.34, 9.80 $\pm$ 6.35 and 5.25 $\pm$ 2.54 at the end of 2, 4 and 6 weeks of treatment, respectively with significance of p<0.001.

Table 74

| Acne lesion score observed at the interval of two weeks (n=96) | | | | | | |
|---|---|---|---|---|---|---|
| | Number of cases | | | | | |
| Acne Lesion scores | Neem caplet(n=48) | | | Placebo(n=48) | | |
| | Week 2 | Week 4 | Week 6 | Week 2 | Week 4 | Week 6 |
| 0 (No reduction in acne lesion score) | 18 | 10 | 04 | 28 | 19 | 15 |
| 1 (< 25% reduction in acne lesion score) | 20 | 12 | 07 | 10 | 18 | 16 |
| 2 (25-49% reduction in acne lesion score) | 10 | 16 | 14 | 10 | 11 | 12 |
| 3 (50-74% reduction in acne lesion score) | - | 10 | 15 | - | - | 05 |
| 4 (> 75% reduction in acne lesion score) | - | - | 08 | - | - | - |

In addition, The mean baseline score for erythema significantly reduced from 1.08 $\pm$ 1.00 to 0.98 $\pm$ 0.64, 0.38 $\pm$ 0.18 and 0.24 $\pm$ 0.14 at the end of 2, 4 and 6 weeks, respectively. The mean baseline score for telangiectesia significantly reduced from 0.34 $\pm$ 0.12 to 0.23 $\pm$ 0.11, 0.14 $\pm$ 0.04 and 0.12 $\pm$ 0.05 at the end of 2, 4 and 6 weeks, respectively. Good response was also noted in reduction in inflammation and pruritis. Majority of the patients experienced a soothing effect with neem caplet. There was no hypersensitivity or flaring of lesions seen during the treatment. None of the patients reported any adverse effects during the entire period of the trial. No worsening of acne infection was observed in any patient during the trial. There were no significant clinical improvement in patients treated with placebo.

Table 75

| Overall response at the interval of two weeks (n=96) | | | | | | | | |
|---|---|---|---|---|---|---|---|---|
| | Neem caplet (n=48) | | | | Placebo (n=48) | | | |
| Overall Response (score) | At entry | Week 2 | Week 4 | Week 6 | At entry | Week 2 | Week 4 | Week 6 |
| Black comedones | 10.11 $\pm$ 8.01 | 7.30 $\pm$ 4.85* | 4.20 $\pm$ 4.06** | 2.08$\pm$ 0.16** | 10.21 $\pm$ 8.14 | 09.30 $\pm$ 3.87 | 8.20 $\pm$ 2.16 | 7.88 $\pm$ 0.16 |
| White comedones | 7.71 $\pm$ 4.04 | 5.21 $\pm$ 3.02* | 3.37$\pm$ 1.01** | 1.27 $\pm$ 0.30** | 7.79 $\pm$ 3.84 | 7.21 $\pm$ 3.11 | 6.87 $\pm$ 1.22 | 7.02 $\pm$ 1.33 |
| Papules | 16.08 $\pm$ 7.40 | 12.60 $\pm$ 7.34* | 9.80 $\pm$ 6.35** | 5.25 $\pm$ 2.54** | 15.67 $\pm$ 7.30 | 15.60 $\pm$ 5.34 | 15.11 $\pm$ 7.35 | 14.25 $\pm$ 2.88 |
| Erythema | 1.08 $\pm$ 1.00 | 0.98 $\pm$ 0.64 | 0.38 $\pm$ 0.18 | 0.24 $\pm$ 0.14 | 1.58 $\pm$ 0.89 | 1.49 $\pm$ 0.54 | 1.38 $\pm$ 0.22 | 1.40 $\pm$ 0.14 |
| Telangiectesia | 0.34 $\pm$ 0.12 | 0.23 $\pm$ 0.11 | 0.14 $\pm$ 0.04 | 0.12 $\pm$ 0.05 | 0.36 $\pm$ 0.32 | 0.33 $\pm$ 0.11 | 0.24 $\pm$ 0.14 | 0.24 $\pm$ 0.05 |
| Pruritis | 0.76 $\pm$ 0.54 | 0.36 $\pm$ 0.25 | 0.20 $\pm$ 0.14 | 0.04 $\pm$ 0.02 | 0.76 $\pm$ 0.56 | 0.75 $\pm$ 0.55 | 0.58 $\pm$ 0.44 | 0.54 $\pm$ 0.32 |
| Hyper-pigmentation | 1.68 $\pm$ 0.90 | 1.02 $\pm$ 0.47 | 0.88 $\pm$ 0.58 | 0.62 $\pm$ 0.30 | 1.98 $\pm$ 0.90 | 1.62 $\pm$ 0.32 | 1.48 $\pm$ 0.88 | 1.29 $\pm$ 0.80 |
| Values are expressed in mean $\pm$ SD. *$p$<0.01 as compared to "At entry" values, **$p$<0.00 as compared to "At entry" values. | | | | | | | | |

Discussion and conclusion:

Acne is a common disease of the pilosebaceous units of the skin and it is an end result of the interplay of multiple factors. Various treatment modalities are adapted to treat acne. These include locally applicable antibacterial agents like erythromycin and various systemic drugs from tetracycline and spiranolactone to cimetidine have been used with varying success. These medications have their own contraindications and side effects. Results of the study showed that significant reduction in inflammation, reduction in black and white comedones, relief of pruritus and pain in inflamed acne lesions was observed along with significant improvent in ALS score in patients treated with Neem caplet. None of the patients reported any adverse effects during the entire period of the trial. No worsening of acne infection was observed in any patient during the trial. Therefore, it may be concluded that Neem caplet is clinically effective and safe in the management of mild to moderate acne vulgaris.

Example-81

Clinical study of trikatu in functional dyspepsia

Evaluation of the efficacy and safety of Trikatu in Functional dyspepsia: An open label, prospective clinical study
Objectives of the study: To evaluate the efficacy and safety of Trikatu in functional dyspepsia
Study design: An open label , prospective clinical study was carried out in humans to evaluate safety and efficacy of trikatu tablets at Guwahati Ayurvedic Medical College, Guwahati during September 2010 to January 2011.
Primary endpoints: The predefined primary endpoints were rapid symptomatic relief from upper abdominal pain, heart burn, bloating, and fullness after meals
Secondary endpoints: The predefined secondary endpoints were short- term and long-term safety and overall compliance to the drug treatment

Inclusion criteria:

- Patients suffering from pain centered in the upper abdomen with associated bloating, and nausea
- Age 18-70 years
- Willing to sign informed consent form.

Exclusion criteria:

- Patients having severe vomiting and diarrhea

    - Patients suffering with gastric or duodenal ulcer, cancer or varices disease.
    - Unwilling to provide informed consent form.

Methodology:

The present study consists of 50 patients suffering from pain centered in the upper abdomen with associated bloating, belching and nausea. The history was noted by interviewing the patient. Thorough clinical examination and symptomatic evaluation was carried out and the details were noted down. All the patients were administered Trikatu at a dose of 1 caplet daily for 4 weeks. All patients were followed-up every week till the end of treatment and symptomatic evaluation and clinical examination was done, along with recording the occurrence of any adverse events.

Statistical Analysis:

The statistical analysis was done on the basis of "Friedman's test followed by Dunn's Multiple Comparison test analysis". The minimum level of significance was considered to be $p < 0.05$.

Results :

A total of 50 patients suffering from functional dyspepsia were included in the clinical trial and all patients completed the study. The study group had 30 male and 20 female patients. The mean age of the patients was 36.8 years. Out of 50 patients, all of them were suffering from upper abdominal pain and heartburn, 42 patients had abdominal bloating, 38 patients had belching, 37 patients had fullness after meals, and 13 patients had nausea. A significant reduction ($p < 0.05$) in mean symptom score of upper abdominal pain from $1.82 \pm 0.133$ to $0.24 \pm 0.067$, heartburn from $1.74 \pm 0.164$ to $0.38 \pm 0.085$, abdominal bloating from $1.86 \pm 0.149$ to $0.44 \pm 0.095$, belching from $1.76 \pm 0.155$ to $0.32 \pm 0.088$, fullness of stomach after meals from $1.280 \pm 0.137$ to $0.240 \pm 0.073$, and nausea from $1.140 \pm 0.134$

to 0.210 $\pm$ 0.062 were observed after 4 weeks of treatment with Trikatu. The reduction in these symptoms score started appearing from the week 2 of treatment itself. There were no clinically significant adverse events, either reported or observed, during the entire study period.

Table 76

| Reduction in mean symptom score of upper abdominal pain, heartburn, bloating, belching, fullness after meals, and nausea with Trikatu treatment | | | | | |
|---|---|---|---|---|---|
| Parameter | At entry | Week 1 | Week 2 | Week 3 | Week 4 |
| Upper abdominal pain | 1.820 $\pm$ 0.133 | 1.520 $\pm$ 0.127 | 0.920 $\pm$ 0.112* | 0.640 $\pm$ 0.084* | 0.240 $\pm$ 0.067* |
| Heartburn | 1.740 $\pm$ 0.164 | 1.420 $\pm$ 0.147 | 0.800 $\pm$ 0.137* | 0.500 $\pm$ 0.104* | 0.380 $\pm$ 0.085* |
| Bloating | 1.860 $\pm$ 0.149 | 1.540 $\pm$ 0.149 | 0.900 $\pm$ 0.149* | 0.600 $\pm$ 0.121* | 0.440 $\pm$ 0.095* |
| Belching | 1.760 $\pm$ 0.155 | 1.580 $\pm$ 0.146 | 0.840 $\pm$ 0.137* | 0.500 $\pm$ 0.100* | 0.320 $\pm$ 0.088* |
| Fullness after meals | 1.280 $\pm$ 0.137 | 0.980 $\pm$ 0.119 | 0.660 $\pm$ 0.104* | 0.340 $\pm$0.081* | 0.240 $\pm$0.073* |
| Nausea | 1.140 $\pm$ 0.134 | 0.880 $\pm$ 0.108 | 0.650 $\pm$ 0.081 * | 0.320 $\pm$ 0.072* | 0.210 $\pm$ 0.062* |
| *$p$<0.05 as compared to the at entry values | | | | | |

Discussion

Dyspepsia refers to chronic, recurrent pain or discomfort centered in the upper abdomen. Once an evaluation has been performed and organic etiologies for the dyspeptic symptoms have been excluded, an affected patient is said to be suffering from functional dyspepsia (FD; previously termed non-ulcer dyspepsia). The present clinical study observed a significant relief in the symptoms of dyspepsia like upper abdominal pain, heart burn, abdominal bloating, abdominal belching, fullness of stomach after meals, and nausea, and vomiting after 4 weeks of treatment with Trikatu caplets. The reduction in these symptoms started appearing from the week 2 of treatment itself and complete relief by 4th week. There were no clinically significant adverse events, either reported or observed, during the entire study period.

Conclusion

Functional dyspepsia is a commonly encountered syndrome in medical practice. Being a multifactorial syndrome complex, many therapeutic interventions have been studied. However, there is no clinically effective and safe medication that can be recommended in the management of functional dyspepsia. This study was conducted to evaluate the clinical efficacy and safety of Trikatu in functional dyspepsia. This study observed a significant symptomatic relief from upper abdominal pain, heart burn, abdominal bloating, belching and fullness of stomach after meals. Symptomatic relief was evident in 2nd week of study in majority of the patients, while almost all were relieved within 4 weeks of treatment. There were no clinically significant adverse events, either reported or observed, during the entire study period. Therefore, it may be concluded that Trikatu is clinically safe and effective in the management of functional dyspepsia.

Example-82

Clinical study of Tinospora cordifolia in human beings

Evaluation of the efficacy and safety of Guduchi in common cold: A randomized, double blind, placebo controlled clinical study

Objectives of the study: To evaluate the efficacy and safety of Guduchi in common cold. The study was carried out at Ayushman Ayurvedic Center, Ganjam Street, Kakinada Study design: The present study was a randomized, double blind, placebo controlled clinical study

Primary endpoints: The predefined primary endpoints were symptomatic relief from common cold

Secondary endpoints: The predefined secondary endpoints were incidence of adverse effects and overall compliance to the drug under investigation.

Inclusion criteria:

- Patients suffering from common cold
- Patients of either sex
- Patients aged between 18-60 years
- Willing to sign informed consent form

Exclusion criteria:

- Pregnant or lactating women
- Patients who are in immunocompromised state
- Patients having the symptoms of common cold from long duration and taking any other medication
- Patients unwilling to sign informed consent form

Methodology:

Eighty patients suffering from common cold aged between 18-60 years were included in the trial. Guduchi caplets at a dose of 1 caplet twice daily for 1 week. Group B patients received placebo at the same dose for 1 week. Efficacy of the drug was analyzed based on the comparison score between the initial value and the score after the treatment. The score was given as follows: Excellent-4, Good-3, Moderate-2, Mild-1, poor-0. The patients were followed upto 1 week. After a week, patients were analyzed for the symptomatic relief from common cold.

Statistical analysis:

The values were expressed as Mean $\pm$ SD. Statistical analysis was carried out using Repeated measures of ANOVA followed by Dunnett's Multiple Comparison Posthoc Test for the analysis clinical parameters.

Results:

This study included 80 patients. Patients were aged between 18-60 years. The demographic details of both the groups Guduchi caplets and placebo namely are listed in table 77. The results of subjects treated with Guduchi caplet is as follows. Most patients started responding to the therapy at the end of 2 days of treatment. At 4th day follow up, 15 patients showed moderate response (25-49% reduction in acne lesions), 12 patients showed good response (50-74% reduction). At the end of 6 days treatment, 8 patients showed excellent response, 11 patients showed good response, 8 patients showed moderate response, 9 patients showed mild response and 4 patients showed poor response.

Table 77

| Demographic data of patients on entry (n=80) | | |
|---|---|---|
| Parameter | Guduchi caplet (n=40) | Placebo (n=40) |
| Age in years (mean $\pm$ SD) | 26.27$\pm$ 08.08 | 24.63$\pm$ 06.56 |
| Weight in Kg (mean $\pm$ SD) | 64.12 $\pm$ 12.32 | 56.40 $\pm$ 18.60 |
| Sex ratio/ M:F | 21:19 | 16:24 |
| H/o smoking (No. of cases) | 5 | 6 |
| H/o alcohol consumption (No. of cases) | 2 | 2 |
| Diet (veg/mixed) | 20/20 | 22/18 |

Table 78

| Common cold score observed at the interval of two days (n=80) | | | | | | |
|---|---|---|---|---|---|---|
| Common cold scores | Number of cases | | | | | |
| | Guduchi caplet(n=40) | | | Placebo(n=40) | | |
| | Day 2 | Day 4 | Day 6 | Day 2 | Day 4 | Day 6 |
| 0 (No reduction in common cold score) | 11 | 7 | 04 | 20 | 18 | 15 |
| 1 (< 25% reduction in common cold score) | 14 | 10 | 09 | 10 | 11 | 12 |
| 2 (25-49% reduction in common cold score) | 15 | 11 | 08 | 10 | 11 | 08 |
| 3 (50-74% reduction in common cold score) | - | 12 | 11 | - | - | 05 |
| 4 (> 75% reduction in common cold score) | - | - | 08 | - | - | - |

Discussion:

Guduchi has immunomodulatory property and thus, it has been shown exhibit antistress, antiinflammatory antiallergic, antioxidant, antineoplastic, and radioprotective actions. Guduchi indicated in various conditions like respiratory tract, infectious wounds like diabetic foot ulcer and antimalarial and antileprotic activities. The results of the present study indicated that administration of Guduchi significantly decreases the occurrence of common cold. Furthermore, Guduchi shortens the mean duration of cold and reduces the severity of cold symptoms without any side effects.

Conclusion:

In this study it was found that the herbal formulation reduces common cold and showed significant improvement after 6 days of Guduchi treatment. There were no serious adverse effects either observed or reported. Therefore, it may be concluded that Guduchi is effective and safe in the treatment of common cold.

Example-83

Clinical study of Ocimum sanctum in human beings
Evaluation of safety and efficacy of Holy Basil capsules in Anxiety: An Open Clinical Study
Objectives of the study: To evaluate the safety and efficacy of holy basil in anxiety. The clinical study in humans was carried out at Department of Manasaroga, S.T.M. College of Ayurveda, Hassan, India.
Study design: The present study was an open clinical trial.
Primary endpoints: The predefined primary efficacy endpoint was rapid symptomatic relief from anxiety.
Secondary endpoints: The predefined secondary safety endpoints were short and long-term safety, as assessed by the incidence of adverse events and patient compliance to therapy.

Inclusion criteria:

- Patients suffering from anxiety
- Patients from the age group of 18-60 years
- Willing to sign informed consent form

Exclusion criteria:

- Patients with hepatic and/or renal disorders, severe depression, organic lesion and uncontrolled diabetics
- Pregnant/ Lactating women
- Unwilling to sign informed consent form.

Methodology:

The enrolled fifty patients were undergone detailed general examination and psychiatric evaluation. The patients were assessed on the basis of Brief Psychiatric Rating Scale. The improvement in the Anxiety symptoms were assessed using a predefined symptom score scale from 0 to 7 (1. not present, 2.very mild, 3.mild, 4.moderate, 5.moderately severe, 6.severe, 7.extremely severe) Assessment criteria's included anxiety, stress, depressive mood, attention, cognitive difficulty. 50 of the trial participants were given Holy Basil and were instructed to take 1 capsule twice daily for 6 weeks. The dose remained constant throughout the study (i.e., no increase or decrease in dose). Any Concomitant illness and medication during study period were recorded throughout the study. All patients were followed up every 2 weeks till the end of treatment (6weeks), and symptomatic evaluation and clinical examination was done.

Statistical Analysis

Statistical analysis was performed by repeated measures of ANOVA using Friedman test followed by Dunnett's multiple comparison posthoc test. Values were expressed mean $\pm$ SD. The Minimal level of significance was fixed at $p<0.05$. Statistical analysis was carried out using GraphPad Prism Version 4.03.

Results

This study included 50 subjects. Study comprised of 28 (56%) male patients and 22 (44%) female. (Table 79)
There were 15 patients who belonged to the age group of 18- 30 years, 22 patients belonged to the age group of 30-40 years and only 13 patients were above 40 Years. (Table 2). The results indicated that Holy Basil significantly reduced anxiety-stress-disorders (Table 3). The overall clinical features also improved after the treatment. Before the test drug, Holy Basil therapy, the baseline score of anxiety score was 6.25 $\pm$ 1.45, Stress score was 5.25 $\pm$ 2.65 and score for depressed mood was 6.40 $\pm$ 2.15, Adjustment was 4.12 $\pm$ 1.50, Attention was 5.32 $\pm$ 0.50, After 6 weeks of therapy anxiety score reduced to 3.15 $\pm$ 1.50, stress score to 2.15 $\pm$ 1.25, depressed mood was improved to 2.25 $\pm$ 1.23. Adjustment and Attention improved to 2.00 $\pm$ 1.25, 2.50 $\pm$ 1.12 respectively. Cognitive difficulty was also improved significantly from 3.21 $\pm$ 2.20 to 1.5 $\pm$ 2.12. The *p* value was <0.001 at the end of 6 weeks. There were no adverse effects reported during the study and compliance to the use of formulation was good.

Table 79

| Demographic data of subjects on entry (n=50) | |
|---|---|
| Parameter | |
| Weight in Kg (mean $\pm$ SD) | 75.05 $\pm$ 8.40 |
| Sex ratio/ M:F | 28:22 |
| H/o smoking (No. of cases) | 7 |
| H/o alcohol consumption (No. of cases) | 3 |
| Diet (veg/mixed) | 20/30 |

Table 80

| Demographic data showing age wise distribution | |
|---|---|
| Age in years | No. of patients |
| 18-30 years | 15 |
| 30-40 years | 22 |
| above 40 yrs | 13 |

Table 81

| Symptomatic improvement with Holy Basil treatment | | | | |
|---|---|---|---|---|
| Parameters | Baseline (Day 0) | Visit 1 (week 2) | Visit 2 (week 4) | Visit 3 (week 6) |
| Anxiety | 6.25 ± 1.45 | 6.12 ± 1.00 | 5.35±1.10* | 3.15±1.50** |
| Stress | 5.25±2.65 | 5.50±1.06 | 3.50±1.50* | 2.15±1.25** |
| Depressed mood | 6.40±2.15 | 6.25±1.02 | 4.35±1.35* | 2.25±1.23** |
| Adjustment | 4.12±1.50 | 4.03±1.53 | 2.47±1.42* | 2.00±1.25** |
| Attention | 5.32±0.50 | 5.10±0.45 | 3.18±0.52* | 2.50±1.12** |
| Intellectual difficulty | 3.21±2.20 | 3.00±1.51 | 2.25±0.65* | 1.5±2.12** |
| (cognitive) | | | | |
| *P<0.01 (as compared to baseline values) * *P<0.001 (as compared to baseline values) | | | | |

Discussion and conclusion

This study was done in fifty patients suffering from anxiety. This study showed that Holy Basil reduced anxiety and stress, and improved the quality of life in people suffering from stress. There was significant change in the assessment criteria's like stress, depressive mood, adjustment and cognitive difficulty. There were no adverse effects reported which proved the safety of the formulation. In this study it was found that the herbal formulation reduces anxiety significantly, the assessment criteria's like anxiety, stress, depressive mood, cognitive difficulty, Adjustment, attention showed significant improvement after 6 weeks of Holy Basil treatment. There were no serious adverse effects either observed or reported. Therefore, it may be concluded that Holy Basil is effective and safe in the treatment of anxiety.

Example-84

A clinical study of Withania somnifera in human beings

A Prospective Randomized, Double Blind Placebo Controlled Clinical Trial of Ashwagandha caplets on stress and anxiety
Objectives of the study: To evaluate the efficacy and safety of Ashwagandha caplets in stress and anxiety.
Study design: The present study was a randomized double blind placebo controlled clinical trial. The clinical study was carried out at , Dr.A.V.Baliga Hospital, Doddana Gudde, Udupi Primary endpoints: The predefined primary endpoints were reduction in anxiety, difference in anxiety scores and stress parameters.
Secondary endpoints: The predefined secondary endpoints were reduced incidence of adverse effects and overall compliance to the drug under investigation.

Inclusion criteria:

- Patients diagnosed with generalized anxiety disorder, mixed anxiety and depression, panic disorder and adjustment disorder with anxiety.
- Patients of both sex aged between 18 to 69 years
- Willing to sign informed consent form

Exclusion criteria:

- Past or present history of alcohol and smoking
- Other medical or psychiatric illness

- Unwilling to sign informed consent form.

Methodology:

One hundred ten patients suffering from anxiety and stress related problems aged between 18 to 69 years were included in the trial. Past or present history of alcohol and smoking, other medical or psychiatric illness and those who were not willing to sign the informed consent were excluded from the study. The patients were divided into 2 groups of 55 each. Group A patients received Ashwagandha caplets and Group B received placebo. Treatment was given at a dose of one caplet daily for a period of 3 months. The dose remained constant throughout the study. No other medication was allowed throughout the study. Efficacy of the drug was analyzed based on the comparison between the baseline score and the score after the treatment. After the 3rd month, patients and investigator independently rated the overall improvement on the following scale: 1-Nil, 2-Mild, 3-Moderate, 4-Good, and 5-Excellent.

Statistical analysis:

The values are expressed as Mean $\pm$ SD. Statistical analysis was carried out using Fisher's Exact Test using GraphPad Prism, Version 4.03 for windows, Graphpad Software, San Diego, California, USA. for presence or absence of various signs and symptoms. Repeated measures of ANOVA followed by Dunnett's Multiple Comparison Posthoc Test were used for the analysis clinical parameters.

Results:

This study included 110 patients. Patients were aged between 18-69 years. The demographic details of both the groups Ashwagandha and placebo namely are listed in table 82.

Table 82

| Demographic data of patients on entry (n=110) | | |
|---|---|---|
| Parameter | Ashwagandha caplets (n=55) | Placebo(n=55) |
| Age in years (mean $\pm$ SD) | 27.49$\pm$ 08.04 | 24.62$\pm$ 07.54 |
| Weight in Kg (mean $\pm$ SD) | 65.08 $\pm$ 12.46 | 56.40 $\pm$ 18.60 |
| Sex ratio/ M:F | 27:28 | 20:35 |

The results of subjects treated with Ashwagandha caplet is as follows. Most patients started responding to the therapy at the end of 2nd month of treatment. At 3rd month follow up, most of the patients showed significant improvement in the reduction of symptoms scores. Before the treatment, the baseline score of anxiety score was 6.42 $\pm$ 1.36 in the treatment group and 6.38 $\pm$1.42 in placebo group , stress score was 5.34 $\pm$ 2.52 in the treatment group and 5.31$\pm$ 2.30 in placebo group, depressed mood was 6.38 $\pm$ 2.16 in treatment group and 6.29 $\pm$ 2.12 in the placebo group. After 3 months of treatment, anxiety score reduced to 3.20 $\pm$1.30, stress score reduced to 2.15 $\pm$1.25 and depressed mood score reduced to 2.20 $\pm$1.22 in treatment group when compared to the placebo group. The p value was <0.001 at the end of 3 months (Table 83).

Table 83

| Symptomatic improvement with Ashwagandha treatment | | | | | | | | |
|---|---|---|---|---|---|---|---|---|
| Parameters | Ashwagandha caplets (n=55) | | | | Placebo (n=55) | | | |
| | At entry | Month 1 | Month 2 | Month 3 | At entry | Month 1 | Month 2 | Month 3 |
| Anxiety | 6.42 $\pm$1.36 | 6.20 $\pm$1.10 | 5.24 $\pm$1.12* | 3.20 $\pm$1.30* | 6.38 $\pm$1.42 | 6.32 $\pm$1.36 | 6.18 $\pm$1.12 | 5.98 $\pm$1.54 |
| Stress | 5.34 $\pm$2.52 | 5.23 $\pm$1.36 | 3.50 $\pm$1.50* | 2.15 $\pm$1.25* | 5.31 $\pm$2.30 | 5.28 $\pm$1.32 | 4.25 $\pm$1.42 | 3.84 $\pm$1.28 |
| Depressed mood | 6.38 $\pm$2.16 | 6.24 $\pm$1.12 | 4.28 $\pm$1.32* | 2.20 $\pm$1.22* | 6.29 $\pm$2.12 | 6.18 $\pm$1.14 | 5.48 $\pm$1.30 | 4.82 $\pm$1.18 |
| *p<0.001 as compared to the respective baseline values | | | | | | | | |

Discussion and conclusion: In this study, Ashwagandha caplets showed overall improvement in anxiety and stress. The results of the study indicate that administration of Ashwagandha caplets significantly decreases the occurrence of anxiety and stress. Furthermore, Ashwagandha caplets showed improvement in the depressed mood symptom without any adverse effects. Also the overall compliance to the study treatment was good. **The study** showed significant improvement after 3 months of Ashwagandha treatment. There were no serious adverse effects either observed or reported. Therefore, it may be concluded that Ashwagandha is effective and safe in the treatment of anxiety and stress.

Example-85

Clinical study of Commiphora mukul in patients with hyperlipidemia
Evaluation of the safety and efficacy of Guggul in patients with hyperlipidemia: A randomized, double blind, placebo controlled trial
Objective of the Study: To evaluate the safety and efficacy of Guggul in patients with hyperlipidemia. The study was carried out at Dr. B.R.K.R.Govt., Ayurvedic College, Hyderabad.
Study design: Randomized, double blind, placebo controlled trial.
Primary endpoint: The predefined primary endpoints include lowering of serum total cholesterol levels and serum triglyceride levels
Secondary end point: The predefined secondary endpoints include reduction of adverse events and overall compliance to treatment

Inclusion criteria

- Patients with serum cholesterol levels more than 200 mg/dL or serum triglyceride levels more than 200 mg/dL
- Patients of both the sex aged more than 18 years.
- Patients willing to sign informed consent

Exclusion criteria

- Patients with secondary hyperlipidemia, alcoholism, or body weight more than 15% above the ideal for their height were excluded from the study
- Pregnant or lactating women
- Unwilling to sign informed consent

Methodology

This study was carried out in 70 patients with hyperlipidemia in the age group of 29 to 64 years. All the patients had serum cholesterol levels of more than 200 mg/dl or more and serum triglyceride 200 mg/dL or more.
Each patient underwent routine hematological and biochemical laboratory investigations. The study planned was randomized, double blind, placebo controlled study for 8 weeks. The written informed consent was obtained from all the patients. Patients were randomized into treatment and placebo group in accordance with random number table. Patients in the treatment group (n=35) were given 2 capsules of Guggul twice daily for 8 weeks. The same dose of placebo was given to the placebo group (n=35) for 8 weeks. The clinical side effects if any were recorded at each visit and discussed with the patient to know the nature, severity and frequency. Repeated laboratory investigations and electrocardiography were done after completion of the study. All patients were evaluated for serum cholesterol and serum triglycerides every two weeks and the observations were recorded in the case report forms.

Statistical analysis:

The results were analyzed statistically using Repeated Measures of ANOVA using Friedman Test. The values are expressed as mean $\pm$ SD or incidence of occurrence. The differences were considered significant at $p<0.05$.

Results

All the patients completed the study. Results of the patients who took Guggul showed a reduction in the cholesterol from 216.30 $\pm$ 7.32mg/ dl to 194.30 $\pm$ 9.08 mg/dl. Triglycerides levels were also reduced from 216.0 $\pm$ 26.37 mg/dl to 186.7 $\pm$ 21.28 mg/dl respectively. The HDL levels increased from 39.00 $\pm$ 2.84 mg/dl to 40.26 $\pm$ 2.83 mg/dl. Similarly, LDL levels were reduced from 133.10 $\pm$ 7.07 mg/dl to 115.20$\pm$ 8.45 mg/dl with Guggul. The VLDL levels were reduced from 41.47 $\pm$ 4.33 mg/dl to 39.52 $\pm$ 4.16 mg/dl with Guggul. The treatment with Guggul was found to be effective during the trial when compared to the placebo group. The level of significance was found to be $p<0.05$ when compared to the placebo groups. There were no adverse effects, either observed or reported during the study period.

Table 84

| Effect of Guggul on the level of lipids before and after treatment | | | | |
|---|---|---|---|---|
| Parameters | Before treatment | | After treatment | |
| | Guggul (n=35) | Placebo (n=35) | Guggul (n=35) | Placebo (n=35) |
| Total cholesterol (mg/dl) | 216.30 $\pm$ 7.32 | 218.20 $\pm$ 6.22 | 194.30 $\pm$ 9.08* | 209.12 $\pm$ 7.21 |
| Triglycerides (mg/dl) | 216.00 $\pm$ 26.37 | 215.20 $\pm$ 4.32 | 186.70 $\pm$ 21.28* | 200.15 $\pm$ 5.24 |
| HDL (mg/dl) | 39.00 $\pm$ 2.84 | 38.24 $\pm$ 3.21 | 40.26 $\pm$ 2.83 | 39.26 $\pm$ 2.12 |
| LDL (mg/dl) | 133.10 $\pm$ 7.07 | 131.28 $\pm$ 8.21 | 115.20 $\pm$ 8.45* | 120.26 $\pm$ 3.54 |
| VLDL (mg/dl) | 41.47 $\pm$ 4.33 | 40.25 $\pm$ 3.28 | 39.52 $\pm$ 4.16* | 40.02 $\pm$ 2.89 |
| *$p<0.05$ as compared to the before treatment value | | | | |

<u>Discussion and conclusion:</u> This study indicates the efficacy of Guggul in bringing about a significant fall in serum cholesterol and triglycerides. It also increases HDL levels at the end of 8 weeks. The exact mechanism of Guggul in decreasing lipids is now known, but, probably it acts by inhibiting the synthesis of cholesterol in liver. The improvement in HDL levels is a significant advantage of Guggul. This study indicates efficacy of Guggul on hyperlipidemic patients and reducing the serum cholesterol and triglycerides and significantly increased HDL-cholesterol. There were no adverse effects, either reported or observed in the clinical study during the study period and the compliance to the study drug was good. There were no drop outs or withdrawal from the study and all the 70 patients completed the study. So, it can be concluded that Guggul is effective and safe in the management of hyperlipidemia.

While this invention has been described in detail with reference to certain preferred embodiments, it should be appreciated that the present invention is not limited to those precise embodiments. Rather, in view of the present disclosure, which describes the current best mode for practicing the invention, many modifications and variations would present themselves to those skilled in the art without departing from the scope and spirit of this invention.

Abbreviated terms for following.

1) LOD Loss on drying
2) BD Bulk density
3) RMG Rapid Mixer Granulator
4) FBD Fluid Bed Drier
5) TVAC Total Viable Aerobic Count
6) NMT Not More Than
7) DT Disintegration Time

**Claims**

**1.** A herbal solid formulation comprising a blend of Super Critical Fluid (CO2) extract, water extract and powder of herb *Andrographis paniculata, Terminalia arjuna, Azadirachta indica, Trikatu (Zingiber officinalis, Piper longum, Piper nigrum), Tinospora cordifolia* or *Ocimum sanctum,* wherein said blend of extract and said powder of herb is mixed in a ratio of about 1:0.5 to about 1:90.

**2.** The herbal solid formulation of claim 1, wherein said herbal solid formulation is essentially free of additives/excipients.

**3.** The process of claim 1, wherein the extract/powder of the herbs is obtained using bark & leaf of *Terminalia arjuna,* leaf & stem of *Azadirachta indica* rhizome, seed **&** pepper stem of Trikatu *(Zingiber officinalis, Piper longum, Piper nigrum),* leaf of *Andrographis paniculata,* stem of *Tinospora cordifolia or* leaf of *Ocimum sanctum.*

**4.** The herbal solid formulation of claim 1, wherein said solid formulation is preferably granules, tablet, caplet or capsule.

**5.** The herbal solid formulation of claim 1, wherein said solid formulation is a tablet.

**6.** The herbal solid formulation of claim 5, wherein the tablet is having hardness of about 2 to about 8 kg/cm$^2$, a friability of less than about 1% and disintegration time is less than about 60 min.

**7.** The herbal solid formulation of claim 6, wherein the tablet is having disintegration time is less than about 30 min.

**8.** The herbal solid formulation of claim 1, wherein said solid formulation is a caplet.

**9.** The herbal solid formulation of claim 8, wherein the caplet comprising *Andrographis paniculata* containing 9 mg of Andrographolide or *Azadirachta indica* containing 18 mg of bitters including Nimbidin or *Terminalia arjuna* containing 0.25 mg of Arjunolic acid and 125 mg of total tannin or *Ocimum sanctum* containing 3.5 mg of total oleonolic acids or *Trikatu* containing 3 mg of Gingerols and 1.09 mg of Piperine or *Tinospora cord#olia* containing 24.6 mg of bitters including tinosporaside.

**10.** A process for preparing a herbal solid formulation as claimed in claim 1, comprising:

autoclaving powder of a herb;
granulating the autoclaved powder with a water extract of the herb;
lubricating the granulated mixture by adding the autoclaved powder of the herb; and
preparing the solid formulation.

**11.** The process of claim 10, wherein the extract of herb is obtained by employing Super Critical Fluid (CO2) extraction, percolation, hot soxhalation or refluxing.

**12.** The process of claim 11, wherein the percolation, hot soxhalation or refluxing method is performed in the presence of a solvent selected from water, grain alcohol or combinations thereof.

**13.** The process of claim 10, wherein the powder of herb is obtained by pulversing the herb to a powder having mesh size between about 20 to about 100.

**14.** The process of claim 10, wherein the extract of herb is obtained by pulversing the herb to a powder having mesh size between about 20 to 80.

**15.** The process of claim 10, wherein the granules are passed through a mesh having size between about 12 to 24.

**16.** The process of claim 10, wherein the granulation is carried out by employing a solvent system selected from water, grain alcohol or combinations thereof.

**17.** A herbal solid formulation comprising a blend of freeze dried powder of *Allium sativum* and water extract of *Allium sativum* along with autoclaved powder of *Allium sativum,* wherein said blend of extract and said powder of herb is mixed in a ratio of about 1:0.5 to about 1:10.

**18.** The herbal solid formulation of claim 17, wherein said herbal solid formulation is essentially free of additives/excipients.

**19.** The process of claim 17, wherein the extract/powder of the *Allium sativum* is obtained using bulb & aerial.

**20.** The herbal solid formulation of claim 17, wherein said solid formulation is preferably granules, tablet or capsule.

**21.** The herbal solid formulation of claim 17, wherein said solid formulation is a tablet or caplet.

**22.** The herbal solid formulation of claim 21, wherein the tablet is having hardness of about 2 to about 8 kg/cm$^2$, a friability of less than about 1% w/w and disintegration time is less than about 60 min.

**23.** The herbal solid formulation of claim 22, wherein the tablet is having disintegration time is less than about 10 min.

**24.** The herbal solid formulation of claim 17, wherein the tablet is having Allicin content not less than 1600 microgram per tablet

**25.** A herbal solid formulation of claim 17, wherein the tablet is having mixture of amino acids comprising Asparagine, Glutamine, Serine, Hydroxyproline, Glycine, Histidine, Argenine, Threonine, Alanine, Proline, Tyrosine, Valine, Methionine, Cysteic acid, Isoleucine, Leucine, Phenylalanine and Lysine

**26.** A process for preparing a herbal solid formulation as claimed in claim 17, comprising:

granulating a blend of water extract and autoclaved plant powder from aerial parts;
lubricating the granulated mixture by adding the powder of *Allium sativum* and freeze dried garlic powder containing the required amount of Allicin; and
preparing the solid formulation.

**27.** The process of claim 26, wherein the extract of *Allium sativum* is obtained by employing percolation, hot soxhalation or refluxing.

**28.** The process of claim 27, wherein the percolation, hot soxhalation or refluxing method is performed in the presence of a solvent selected from alcohol, water, grain alcohol or combinations thereof and preferably water.

**29.** The process of claim 26, wherein the water extract of *Allium sativum* is passed through a mesh having size between about 20 to 80.

**30.** The process of claim 26, wherein the powder of *Allium sativum* is obtained by pulversing the herb to a powder having mesh size between about 20 to about 100.

**31.** The process of claim 26, wherein the granulation is carried out by employing a solvent system selected from water, grain alcohol or combinations thereof and preferably water.

**32.** The process of claim 26, wherein the granules are passed through a mesh having size between about 12 to 24.

**33.** A herbal solid formulation comprising a blend of Super Critical Fluid ($CO_2$) extract, water extract and powder of herb *Withania somnifera, Zingiber officinale* or *Commiphora mukul,* wherein said blend of extract and said powder of herb is mixed in a ratio of about 1:0.5 to about 1:10.

**34.** The herbal solid formulation of claim 33, wherein said herbal solid formulation is essentially free of additives/excipients.

**35.** The process of claim 33, wherein the extract/powder is obtained using resin & stem of *Commiphora mukul,* resin & stem of *Withania somnifera* and bulb & aerial of *Zingiber officinale.*

**36.** The herbal solid formulation of claim 33, wherein said solid formulation is preferably granules, tablet or capsule.

**37.** The herbal solid formulation of claim 33, wherein said solid formulation is a tablet.

**38.** The herbal solid formulation of claim 37, wherein the tablet is having hardness of about 3 to about 4 kg/cm$^2$, a friability of less than about 1% and disintegration time is less than about 30 min.

**39.** The herbal solid formulation of claim 37, wherein the tablet is having disintegration time is less than about 10 min.

**40.** A process for preparing a herbal solid formulation as claimed in claim 33, comprising:

autoclaving powder of a herb;
granulating the autoclaved powder with a water extract of the herb;
lubricating the granulated mixture by adding the autoclaved powder of the herb; and
preparing the solid formulation.

**41.** The process of claim 40, wherein the extract of herb is obtained by employing Super Critical Fluid ($CO_2$) extraction,

percolation, hot soxhalation or refluxing.

42. The process of claim 41, wherein the percolation, hot soxhalation or refluxing method is performed in the presence of a solvent selected from water, grain alcohol or combinations thereof.

43. The process of claim 40, wherein the powder of herb is obtained by pulversing the herb to a powder having mesh size between about 10 to about 100.

44. The process of claim 43, wherein the powder of herb is obtained by pulversing the herb to a powder having mesh size between about 20 to 80.

45. The process of claim 40, wherein the granulation is carried out by employing a solvent system selected from water, grain alcohol or combinations thereof.

# Figure 1: LC chromatogram and total ion chromatogram of Arjuna CO$_2$ Extract

TIC of +Q1: from Sample 56 (Arjuna CO2 ext. (ORG-164 ) in MeOH 1mg/mL027) of Pure Herbs 07.05.10SET...  Max. 1.7e7 cps.

+Q1: 0.067 to 29.946 min from Sample 56 (Arjuna CO2 ext. (ORG-164 ) in MeOH 1mg/mL027) of Pure Herb...  Max. 7.2e5 cps.

Shimadzu LC Controller Detector A, Channel 2 from Sample 56 (Arjuna CO2 ext. (ORG-164 ) in MeOH 1mg/mL...  Max. 651.0 .

EP 2 524 695 A1

# Figure 2: LC chromatogram and total ion chromatogram of Arjuna Water Extract

EP 2 524 695 A1

# Figure 3: LC chromatogram and total ion chromatogram of Neem $CO_2$ Extract

EP 2 524 695 A1

Figure 4: LC chromatogram and total ion chromatogram of Neem Water Extract

# Figure 5: LC chromatogram and total ion chromatogram of Trikatu Water Extract

TIC of +Q1: from Sample 5 (trikatu water ext.(ORG-137 DE)003) of TrikatuSET1.wiff (Heated Ne...    Max. 7.8e7 cps.

+Q1: 0.100 to 28.442 min from Sample 5 (trikatu water ext.(ORG-137 DE)003) of TrikatuSET1....    Max. 2.9e6 cps.

Shimadzu LC Controller Detector A, Channel 2 from Sample 5 (trikatu water ext.(ORG-137 DE)003)...    Max. 5.0e4.

EP 2 524 695 A1

# Figure 6: LC chromatogram and total ion chromatogram of Trikatu CO$_2$ Extract

TIC of +Q1: from Sample 13 (Trikatu CO2 ext.(ORG-147 SE)012) of TrikatuSET1.wiff (Heated N...    Max. 3.9e8 cps.

+Q1: 18.014 min from Sample 13 (Trikatu CO2 ext.(ORG-147 SE)012) of TrikatuSET1.wiff (Hea...    Max. 2.6e6 cps.

Shimadzu LC Controller Detector A, Channel 2 from Sample 13 (Trikatu CO2 ext.(ORG-147 SE)012...    Max. 9.2e4 .

EP 2 524 695 A1

# Figure 7: LC chromatogram and total ion chromatogram of Guduchi CO$_2$ Extract

■ TIC of +Q1: from Sample 16 (Guduchi CO2 ext.(ORG-162 )012) of ArjunaSET1.wiff (Turbo Spra...    Max. 2.9e7 cps.

■ +Q1: 1.036 min from Sample 16 (Guduchi CO2 ext.(ORG-162 )012) of ArjunaSET1.wiff (Turbo ...    Max. 2.0e6 cps.

■ Shimadzu LC Controller Detector A, Channel 2 from Sample 16 (Guduchi CO2 ext.(ORG-162 )012) ...    Max. 3.3e4 .

EP 2 524 695 A1

# Figure 8: LC chromatogram and total ion chromatogram of Guduchi Water Extract

EP 2 524 695 A1

## Figure 9: LC chromatogram and total ion chromatogram of Tulasi CO$_2$ Extract

## Figure 10: LC chromatogram and total ion chromatogram of Tulasi Water Extract

TIC of +Q1: from Sample 34 (Tulsi water ext.(ORG-131 DE) in water 1mg/mL005) of Pure Herbs...  Max. 2.8e7 cps.

+Q1: 23.362 min from Sample 34 (Tulsi water ext.(ORG-131 DE) in water 1mg/mL005) of Pure ...  Max. 9.5e4 cps.

Shimadzu LC Controller Detector A, Channel 1 from Sample 34 (Tulsi water ext.(ORG-131 DE) in ...  Max. 1089.0 .

EP 2 524 695 A1

# Figure 11: LC chromatogram and total ion chromatogram of Withania $CO_2$ Extract

EP 2 524 695 A1

# Figure 12: LC chromatogram and total ion chromatogram of Ginger $CO_2$ Extract

TIC of +Q1: from Sample 1 (Ginger CO2 ext.(ORG-152 SE) in MeOH 003) of DataSET1.wiff (Hea...    Max. 3.8e8 cps.

+Q1: 0.100 to 39.972 min from Sample 1 (Ginger CO2 ext.(ORG-152 SE) in MeOH 003) of Data...    Max. 1.9e6 cps.

Shimadzu LC Controller Detector A, Channel 1 from Sample 1 (Ginger CO2 ext.(ORG-152 SE) in M...    Max. 1.1e5 .

EP 2 524 695 A1

## Figure 13: LC chromatogram and total ion chromatogram of Ginger Water Extract

## Figure 14: LC chromatogram and total ion chromatogram of Guggul CO$_2$ Extract

TIC of +Q1: from Sample 9 (Guggul CO2 ext.(ORG-166 CO2) B.No.RO90/0573 in ACN 1mg/mL...    Max. 2.7e7 cps.

+Q1: 0.301 to 59.993 min from Sample 9 (Guggul CO2 ext.(ORG-166 CO2) B.No.RO90/0573 in ...    Max. 4.3e4 cps.

+Q1: 21.424 to 24.499 min from Sample 9 (Guggul CO2 ext.(ORG-166 CO2) B.No.RO90/0573 in...    Max. 9.6e4 cps.

Shimadzu LC Controller Detector A, Channel 1 from Sample 9 (Guggul CO2 ext.(ORG-166 CO2) B....    Max. 1.1e5.

EP 2 524 695 A1

Figure-15

Europäisches
Patentamt

European
Patent Office

Office européen
des brevets

# EUROPEAN SEARCH REPORT

**Application Number**

EP 11 16 6389

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE TCM [Online]<br>SIPO;<br>24 November 2004 (2004-11-24),<br>Ma Yuefeng et al.: "A Herba Andrographis product and its process for preparation, and its use in pharmacy",<br>XP002666175,<br>Database accession no. CN-03136192-A<br>* abstract *<br>& CN 1 548 041 A (ZHEJIANG KELISI AN PHARMACEUTI [CN] ZHEJIANG SOUTH OCEAN PHARMACEU [CN)<br>24 November 2004 (2004-11-24) | 1-16,<br>33-45 | INV.<br>A61K36/185<br>A61K36/58<br>A61K36/19<br>A61K36/67<br>A61K36/8962<br>A61K36/9068<br>A61K36/59<br>A61K36/53 |
| Y | DATABASE WPI<br>Week 201058<br>Thomson Scientific, London, GB;<br>AN 2010-K02321<br>XP002666176,<br>& CN 101 766 780 A (TIANJIN ZHONGXIN PHARMACY GROUP CO LTD D)<br>7 July 2010 (2010-07-07)<br>* abstract * | 1-16,<br>33-45 | |
| Y | DATABASE WPI<br>Week 201082<br>Thomson Scientific, London, GB;<br>AN 2010-L68890<br>XP002666177,<br>& CN 101 810 768 A (TENG S)<br>25 August 2010 (2010-08-25)<br>* abstract * | 1-16,<br>33-45 | TECHNICAL FIELDS<br>SEARCHED (IPC)<br><br>A61K<br>A61P |
| Y | US 7 014 874 B1 (MAJEED MUHAMMED [US] ET AL) 21 March 2006 (2006-03-21)<br>* the whole document * | 1-16,<br>33-45 | |

-/--

The present search report has been drawn up for all claims

| Place of search | Date of completion of the search | Examiner |
|---|---|---|
| Munich | 16 May 2012 | Thalmair-De Meyere |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 11 16 6389

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2006/193928 A1 (SOMAN GIRISH S [US] ET AL) 31 August 2006 (2006-08-31) * the whole document * | 1-16, 33-45 | |
| Y | DATABASE EPODOC [Online] EUROPEAN PATENT OFFICE, THE HAGUE, NL; 16 January 2000 (2000-01-16), Kim Yeong Cheol: "Process for preparing garlic pill", XP002674613, Database accession no. KR-19990009700-A * abstract * & KR 2000 0060990 A (KIM Y C) 16 October 2000 (2000-10-16) | 17-32 | |
| Y | RATTI C. ET AL.: "Drying of garlic (Allium sativum) and its effect on allicin retention.", DRYING TECHNOLOGY, vol. 25, no. 1--3, 1 September 2007 (2007-09-01), page 349, XP009158768, DRYING TECHNOLOGY 2007 DEP. DES SOLS ET DE GENIE AGROALIMENTAIRE, INST. OF NUTRACEUTICAL & FUNCTIONAL FOODS (INAF), UNIV. LAVAL, CANADA * the whole document * | 17-32 | TECHNICAL FIELDS SEARCHED (IPC) |
| E | US 2011/318436 A1 (MITRA SHANKAR KUMAR [IN] ET AL) 29 December 2011 (2011-12-29) * the whole document * | 17-32 | |

The present search report has been drawn up for all claims

| Place of search | Date of completion of the search | Examiner |
|---|---|---|
| Munich | 16 May 2012 | Thalmair-De Meyere |

EPO FORM 1503 03.82 (P04C01)

Europäisches
Patentamt

European
Patent Office

Office européen
des brevets

**Application Number**

EP 11 16 6389

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

88

Europäisches
Patentamt

European
Patent Office

Office européen
des brevets

**LACK OF UNITY OF INVENTION**
**SHEET B**

Application Number

EP 11 16 6389

The Search Division considers that the present European patent application does not comply with the requirements of unity of invention and relates to several inventions or groups of inventions, namely:

1. claims: 1-16, 33-45

   Herbal solid formulation comprising a blend of Super Critical Fluid (CO2) extract, water extract and powder of herb Andrographis paniculata, Terminalia arjuna, Azadirachta indica, Trikatu (Zingiber officinalis, Piper longum, Piper nigrum), Tinospora cordifolia or Ocimum sanctum, Whitania somnifera, Zingiber officinale, Commiphora mukul, wherein said blend of extract and said powder of herb is mixed in a certain ratio (1:0.5 to 1:90 or 1:0.5 to 1:10, respectively).

   ---

2. claims: 17-32

   Herbal solid formulation comprising a blend of freeze dried powder of Allium sativum and water extract of Allium sativum along with autoclaved powder of Allium sativum, wherein said blend of extract and said powder of herb is mixed in a ratio of about 1:0.5 to about 1:10.

   ---

## ANNEX TO THE EUROPEAN SEARCH REPORT
## ON EUROPEAN PATENT APPLICATION NO.

EP 11 16 6389

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2012

| Patent document cited in search report | | Publication date | Patent family member(s) | Publication date |
|---|---|---|---|---|
| CN 101766780 | A | 07-07-2010 | NONE | |
| CN 101810768 | A | 25-08-2010 | NONE | |
| US 7014874 | B1 | 21-03-2006 | NONE | |
| US 2006193928 | A1 | 31-08-2006 | NONE | |
| US 2011318436 | A1 | 29-12-2011 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6207189 B, Mercati

- US 6468563 B, Schmidt

**Non-patent literature cited in the description**

- *J. AOAC,* 1987, vol. 70, 241-247
- By Dr.V.Rajpal. Eastern publishers, 2002, vol. 1, 256

- *Journal of Association of Official Agricultural Chemists,* 1987, vol. 70, 241-247